(19) **Europäisches Patentamt**
**European Patent Office**
**Office européen des brevets**

(11) **EP 2 583 647 B1**

(12) **EUROPEAN PATENT SPECIFICATION**

(45) Date of publication and mention
of the grant of the patent:
**17.09.2014 Bulletin 2014/38**

(51) Int Cl.:
*A61F 13/15* (2006.01)     *A41B 9/12* (2006.01)
*A61F 13/472* (2006.01)    *A61F 13/49* (2006.01)
*A61F 13/53* (2006.01)     *B32B 5/08* (2006.01)
*D04H 1/407* (2012.01)

(21) Application number: **11795743.1**

(22) Date of filing: **14.06.2011**

(86) International application number:
**PCT/JP2011/063610**

(87) International publication number:
**WO 2011/158838 (22.12.2011 Gazette 2011/51)**

(54) **ABSORBENT SHEET AND METHOD FOR PRODUCING SAME**

SAUGFÄHIGRE FOLIE UND VERFAHREN ZU IHRER HERSTELLUNG

FEUILLE ABSORBANTE ET SON PROCÉDÉ DE PRODUCTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.06.2010   JP 2010136627
15.06.2010   JP 2010136628
15.06.2010   JP 2010136629**

(43) Date of publication of application:
**24.04.2013   Bulletin 2013/17**

(60) Divisional application:
**14151192.3 / 2 722 026**

(73) Proprietor: **Asahi Kasei Chemicals Corporation**
**Tokyo 101-8101 (JP)**

(72) Inventors:
• **AKIYAMA Tsutomu**
**Tokyo 101-8101 (JP)**
• **OKAMOTO Hiroshige**
**Tokyo 101-8101 (JP)**
• **ISHIKAWA Yoshimasa**
**Kanonji-shi**
**Kagawa 768-0033 (JP)**

(74) Representative: **O'Brien, Simon Warwick**
**D Young & Co LLP**
**120 Holborn**
**London EC1N 2DY (GB)**

(56) References cited:
**EP-A1- 1 178 149     WO-A1-2006/121148
JP-A- 4 352 877      JP-A- 8 246 395
JP-A- 9 156 013      JP-A- 9 156 014
JP-A- 9 187 476      JP-A- 10 329 252
JP-A- 2002 159 533   JP-A- 2007 275 331
JP-A- 2008 086 590   JP-A- 2008 119 860
JP-A- 2008 237 430**

## Description

### Technical Field

The present invention relates to an absorbent sheet and a method for producing the same.

### Background Art

In recent years, absorbent resins that absorb a large amount of water to gel have been developed, and are used especially in the field of sanitary materials such as disposable diapers and sanitary napkins. An absorbent resin is usually in the form of fine powder and thus has a disadvantage in that it is difficult to be handled, and accordingly in the field of sanitary materials such as disposable diapers, the absorbent resin is supported by pulp, for example, and used in a state packed in a bag. This method has a problem that it is difficult to provide a certain level of performance of the absorbent resin because the thickness of the absorbent body becomes large and uneven distribution occurs in the bag. Furthermore, the method has a problem that productivity is low. To solve these problems, the use of an absorbent body that is formed in a sheet by fixing a powdered absorbent resin on a sheet has been studied.

As methods for forming a sheet of such an absorbent body, an example in which a hot-melt adhesive is used to bond an absorbent resin so as to form a sheet by fixing the absorbent resin (e.g., Patent Literature 1), an example in which a mixture of pulverized pulp and a thermoplastic fiber is formed into a sheet by applying heat treatment and the resulting sheet is made to carry a powdered solid of adsorbent resin (e.g., Patent Literature 2), a method in which the adhesive component in adsorbent resin is used and wet pulp and the adsorbent resin are fixed while being dried (e.g., Patent Literature 3), an example in which an adsorbent resin and a fibrous material such as pulp are uniformly mixed to effect hydrogen bonding of pulp fibers to each other with water and the resulting mixture is formed in a sheet (e.g., Patent Literature 4), an example in which absorbent polymer particles being polymerized are adhered to a fibrous base material and polymerization of absorbent resin is performed on the fibrous base material (e.g., Patent Literature 5), and an absorbent sheet in which absorbent resins are directly bonded to a hydrophilic base material to form conduits from the base material to the absorbent resins and that can exhibit high absorption capacity of the absorbent resins themselves (e.g., Patent Literature 6), for example, are known.

### Citation List

#### Patent Literatures

[Patent Literature 1] Japanese Patent No. 3196933
[Patent Literature 2] Japanese Patent Application Laid-Open Publication No. 53-4789
[Patent Literature 3] Japanese Patent Application Laid-Open Publication No. 56-60556
[Patent Literature 4] Published Japanese Translation of PCT Application No. 2003-508647
[Patent Literature 5] Japanese Patent Application Laid-Open Publication No. 2003-11118
[Patent Literature 6] PCT Application Publication No. WO 2006/121148

### Summary of Invention

### Problems to be Solved by the Invention

However, an absorbent sheet having a structure excellent in absorption speed and dry comfort while completely fixing an absorbent resin thereon has not been obtained.

It is an object of the present invention to provide an absorbent sheet excellent in absorption speed and dry comfort after absorption and a method for producing the same.

### Means for Solving the Problems

As a result of exhaustive research to address the above-identified problem, the inventors of the present invention found that when producing a sheet constructed of absorbent resins, hydrophilic fibers, and hydrophobic fibers, the absorbent resins and the hydrophilic fibers are bonded by dehydrating and drying them in a state of being in contact with each other, the resulting resins are sterically arranged in a grid of the hydrophobic fibers and formed into a sheet by heat, and thus an absorbent sheet in which the absorbent resins each having a conduit is sterically arranged in space can be constructed. The absorbent sheet of the present invention can minimize blocking of the resins to each other and repulsion thereof when the sheet swells because the resins are sterically arranged, and can quickly absorb liquid into the absorbent resins because the hydrophilic fibers and the absorbent resins are efficiently bonded, which is also excellent in dry comfort.

In addition, the inventors found that when the absorbent resins and the hydrophilic fibers are in contact with water, presence of ammonium ions can facilitate permeation of liquid from the hydrophilic fibers to the absorbent resins and has an effect of improving dry comfort. Furthermore, the inventors found that when fixing the absorbent resins into the grid formed by the hydrophobic fibers, because of the presence of ammonium ions, repulsion acts between the hydrophobic fibers and the hydrophilic fibers and/or the absorbent resins, thereby securing spaces for the absorbent resins to swell, and liquid can be quickly absorbed.

More specifically, the present invention provides an absorbent sheet and a method for producing the same as follows.

[1] A method for producing an absorbent sheet containing absorbent resins, hydrophilic fibers, and hydrophobic fibers, the method including:

a dehydration step of dehydrating the absorbent resins and the hydrophilic fibers from a state of being in contact with each other and containing water to obtain composite compositions; and

a sheet-forming step of forming a sheet of the composite compositions and the hydrophobic fibers by heating while bringing them into contact with each other.

[2] The method for producing an absorbent sheet according to [1], wherein the absorbent sheet is a sheet in which the composite compositions are dispersed among the hydrophobic fibers.

[3] The method for producing an absorbent sheet according to [1] or [2], wherein the dehydration step and the sheet-forming step are performed in this order or simultaneously.

[4] The method for producing an absorbent sheet according to any one of [1] to [3] further including a first mixing step of mixing the absorbent resins and the hydrophilic fibers to bring them into contact with each other, wherein the first mixing step and the dehydration step are performed in this order or simultaneously.

[5] The method for producing an absorbent sheet according to any one of [1] to [4] further including a second mixing step of mixing the composite compositions and the hydrophobic fibers to bring them into contact with each other, wherein

the second mixing step and the sheet-forming step are performed in this order or simultaneously.

[6] The method for producing an absorbent sheet according to any one of [1] to [5], wherein at the first mixing step, the absorbent resins and the hydrophilic fibers to be mixed contain a total of 0.1 to 100 parts by mass of water per 100 parts by mass of the absorbent resins.

[7] The method for producing an absorbent sheet according to any one of [1] to [6], wherein at the dehydration step, the absorbent resins and the hydrophilic fibers in a state containing water contain a total of 20 to 1000 parts by mass of water per 100 parts by mass of the absorbent resins.

[8] The method for producing an absorbent sheet according to any one of [1] to [7], wherein at the sheet-forming step, the composite compositions and the hydrophobic fibers before being heated contain a total of 1 to 200 parts by mass of water per 100 parts by mass of the absorbent resins.

[9] The method for producing an absorbent sheet according to any one of [1] to [8], wherein at the sheet-forming step, on a support that is continuously fed, the composite compositions and the hydrophobic fibers are heated while being brought into contact with each other.

[10] The method for producing an absorbent sheet according to [9], wherein the support is a fibrous support in which content ratio of hydrophobic fibers is 90 mass% or more.

[11] The method for producing an absorbent sheet according to any one of [1] to [10], wherein ratio of the hydrophilic fibers to the hydrophobic fibers in the absorbent sheet is 9:1 to 2:8 in mass ratio.

[12] The method for producing an absorbent sheet according to any one of [1] to [11], wherein ratio of the absorbent resins to the hydrophilic fibers in the absorbent sheet is 10:1 1 to 1:5 in mass ratio.

[13] The method for producing an absorbent sheet according to any one of [1] to [12], wherein with respect to whole quantity of the absorbent resins, absorbent resins capable of passing through a sieve having sieve openings of 90 micrometers constitute 50 mass% or less, and absorbent resins incapable of passing through a sieve having sieve openings of 425 micrometers constitute 50 mass% or less.

[14] The method for producing an absorbent sheet according to any one of [1] to [13], wherein water-absorption capacity of the absorbent resins under no pressure is 50 g/g or more.

[15] The method for producing an absorbent sheet according to any one of [1] to [14], wherein surface strength of the absorbent resins is 0.1 to 5.5 N.

[16] The method for producing an absorbent sheet according to any one of [1] to [15], wherein the absorbent resins contain a carboxy group and a crosslinking agent capable of reacting with the carboxy group.

[17] The method for producing an absorbent sheet according to any one of [1] to [16], wherein salt concentration on outer surfaces of the absorbent resins before dehydration is 85% or more.

[18] The method for producing an absorbent sheet according to any one of [1] to [17], wherein at the dehydration step, surface salt concentration of the absorbent resins before dehydration and surface salt concentration of the absorbent resins after dehydration are different.

[19] The method for producing an absorbent sheet according to any one of [1] to [18], wherein at the sheet-forming step, surface salt concentration of the absorbent resins before heating and surface salt concentration of the absorbent resins after heating are different.

[20] A method for producing an absorbent product, the method including a step of adhering an absorbent sheet obtained by the method for producing an absorbent sheet according to any one of [1] to [19] to a top sheet that is continuously fed and/or a back sheet that is continuously fed.

[21] A method for producing an absorbent product, the method including:

a step of supplying and fixing an absorbent sheet that is obtained by a production method including steps (1) to (5) described below or an absorbent sheet that is obtained by a production method including steps (6) to (9) described below onto a continuum of material for a back sheet that is conveyed along a circumferential surface of an operating drum; and

a step of cutting the continuum of material for the back sheet to which the absorbent sheet is fixed into lengths each corresponding to a dimension of the absorbent product:

(1) a step of mixing hydrophilic fibers and absorbent resins;
(2) a step of humidifying a mixture of the hydrophilic fibers and the absorbent resins;
(3) a step of performing dehydration in such a state that the hydrophilic fibers and the absorbent resins are in contact with each other;
(4) a step of mixing composite compositions constructed of the hydrophilic fibers and the absorbent resins with hydrophobic fibers;
(5) a step of continuously performing shaping in a sheet form by heating;
(6) a step of mixing the hydrophilic fibers, the absorbent resins, and the hydrophobic fibers;
(7) a step of humidifying a mixture of the hydrophilic fibers, the absorbent resins, and the hydrophobic fibers;
(8) a step of performing dehydration in such a state that the hydrophilic fibers and the absorbent resins are in contact with each other; and
(9) a step of continuously performing shaping in a sheet form by heating.

[22] The method for producing an absorbent product according to [21], the method further including a step of supplying and fixing a continuum of material for a top sheet to the continuum of material for the back sheet to which the absorbent sheet is fixed, wherein the step of fixing is performed before the step of cutting.

[23] The method for producing an absorbent product according to [21] or [22], wherein the absorbent sheet is obtained by changing mixing ratio of the composite compositions to the hydrophobic fibers or mixing ratio of the absorbent resins, the hydrophilic fibers, and the hydrophobic fibers in a width direction and/or a length direction of the absorbent sheet, and content of the absorbent resins differs depending on positions.

[24] The method for producing an absorbent product according to any one of [21] to [23], wherein compounding ratio of the hydrophilic fibers to the hydrophobic fibers is 9:1 to 2:8 in mass ratio.

[25] The method for producing an absorbent product according to any one of [21] to [24], wherein compounding ratio of the absorbent resins to the hydrophilic fibers is 10:1 to 1:5 in mass ratio.

[26] The method for producing an absorbent product according to any one of [21] to [25], wherein surface salt concentration of the absorbent resins is 85% or more.

[27] The method for producing an absorbent product according to any one of [21] to [26], wherein between before and after step (3) or step (8), the surface salt concentration of the absorbent resins changes.

[28] A composite composition for producing an absorbent sheet, the composite composition being obtained by dehydrating absorbent resins and hydrophilic fibers from a state of being in contact with each other and containing water.

[29] The composite composition according to [28], wherein average fiber length of the hydrophilic fibers is 20 to 1000 micrometers.

[30] The composite composition according to [28] or [29], wherein ratio of average fiber length of the hydrophilic fibers to average particle size of the absorbent resins is 0.05:1 to 2:1.

[31] A method for producing the composite composition according to any one of [28] to [30], wherein the absorbent resins are hydrous gels, the method including a step of mixing the absorbent resins containing water with the hydrophilic fibers to bring them into contact with each other and performing dehydration.

[32] An absorbent sheet containing at least absorbent resins, hydrophobic fibers, and hydrophilic fibers, wherein the absorbent resins and the hydrophilic fibers are in contact with each other, and the absorbent resins, the hydrophilic fibers, or composite compositions constructed of the absorbent resins and the hydrophilic fibers are dispersed among the hydrophobic fibers.

[33] The absorbent sheet according to [32], wherein the hydrophobic fibers are substantially fused with each other.

[34] The absorbent sheet according to [32] or [33], wherein the absorbent resins are crosslinked with a condensation crosslinking agent and also are fixed substantially in the absorbent sheet.

[35] The absorbent sheet according to any one of [32] to [34], wherein average fiber length of the hydrophilic fibers constituting the composite compositions is 20 to 1000 micrometers.

[36] The absorbent sheet according to any one of [32] to [35], wherein ratio of average fiber length of the hydrophilic fibers constituting the composite compositions to average particle size of the absorbent resins is 0.05:1 to 2:1.

[37] The absorbent sheet according to any one of [32] to [36] further containing ammonium ions at 0.5 to 18 mass%.

[38] The absorbent sheet according to any one of [32] to [37] further including an absorbent layer containing the absorbent resins and a hydrophobic fiber layer containing the hydrophobic fibers provided on one side or both sides of the absorbent layer, wherein fiber density of the absorbent layer is lower than fiber density of the hydrophobic fiber layer.

[39] The absorbent sheet according to any one of [32] to [38], wherein diameter of the hydrophobic fibers contained in the hydrophobic fiber layer is smaller than diameter of the hydrophobic fibers contained in the absorbent layer.

[40] The absorbent sheet according to any one of [32] to [39], wherein the hydrophobic fibers contained in the hydrophobic fiber layer and the hydrophobic fibers contained in the absorbent layer have layers constructed of organic resin on their surfaces.

[41] The absorbent sheet according to any one of [32] to [39], wherein the absorbent resins contain carboxy groups and, in the absorbent sheet, part of the carboxy groups form a neutralized salt and there is a concentration gradient in which an amount of the neutralized salt present therein decreases from inside toward surface of each of the absorbent resins.

[42] The absorbent sheet according to any one of [32] to [41], wherein absorption speed is 13 seconds or less.

[43] The absorbent sheet according to any one of [32] to [42], wherein rewet amount is 0.2 gram or less.

[44] An absorbent product including the absorbent sheet according to any one of [32] to [43] and a top sheet provided on one side of the absorbent sheet and/or a back sheet provided on the other side of the absorbent sheet.

[45] An absorbent product produced by the method for producing an absorbent product according to any one of [21] to [27].

[46] An absorbent sheet including an absorbent layer that is constructed of at least absorbent resins, hydrophobic fibers, and hydrophilic fibers and in which the hydrophobic fibers are substantially fused with each other, wherein in the absorbent layer, area density of the hydrophobic fibers is 10 to 50 $g/m^2$, ratio of area density of the hydrophilic fibers to the area density of the hydrophobic fibers is 10:90 to 70:30, area density of the absorbent resins is 10 to 50 $g/m^2$, and thickness of the absorbent layer is 2 millimeters or less.

[47] A grill drip pan including the absorbent sheet according to [46].

[48] A coffin lining pad including the absorbent sheet according to [46].

[49] A dew condensation preventing material including the absorbent sheet according to [46].

[50] A sweat-absorbent pad including the absorbent sheet according to [46].

[51] An antifouling pad for clothes including the absorbent sheet according to [46].

[52] A bedding cover including the absorbent sheet according to [46].

[53] A thawing pad including the absorbent sheet according to [46].

[54] A water removing pad including the absorbent sheet according to [46].

[55] A mask including the absorbent sheet according to [46].

[56] A wet proof pad including the absorbent sheet according to [46].

## Effects of the Invention

According to the present invention, an absorbent sheet that is excellent in absorption speed and dry comfort after absorption and has high flexibility and good texture, and a method for producing the same can be provided. Not only can this absorbent sheet be preferably used for sanitary materials, but also can simplify a process for producing sanitary materials, for example. In addition, the absorbent sheet of the present invention can be processed as desired and can be used in any desired shape with almost no detachment occurring, and accordingly can be widely used also for other than sanitary materials.

**Brief Description of Drawings**

[Fig. 1] Fig. 1 is a scanning electron microscope (SEM) picture of a cross-section of an absorbent sheet according to the present embodiment.
[Fig. 2] Fig. 2 is a schematic sectional view illustrating an absorbent sheet according to one embodiment.
[Fig. 3] Fig. 3 is a schematic sectional view illustrating an absorbent product according to one embodiment.
[Fig. 4] Fig. 4 is a schematic sectional view illustrating an absorbent product according to one embodiment.
[Fig. 5] Fig. 5 is a schematic sectional view illustrating an absorbent product according to one embodiment.

**Embodiments for Carrying Out the Invention**

Embodiments of the present invention will now be described in detail. Note that the present invention is not limited to the embodiments below, and can be practiced with various modification within the spirit of the invention.

[1. Absorbent Sheet]

An absorbent sheet of the present invention is constructed of at least absorbent resins, hydrophilic fibers, and hydrophobic fibers. In addition, in the absorbent sheet of the present invention, the absorbent resins and the hydrophilic fibers are in contact with each other, and the absorbent resins, the hydrophilic fibers, or composite compositions constructed of the absorbent resins and the hydrophilic fibers are dispersed among the hydrophobic fibers.

The speed of the hydrophilic fibers temporarily capturing a liquid is fast, but they release the liquid when a force is applied thereto. In contrast, the absorbent resins, although their absorption speed is slow, can completely capture a liquid and retain the liquid even when a force is applied. An absorbent sheet whose absorption speed is fast and that is excellent in dry comfort is considered to be a sheet that can quickly send a liquid that the hydrophilic fibers have captured to the absorbent resins. Until now, absorbers having various structures have been proposed by trial and error, but satisfactory absorption speed or dry comfort cannot be accomplished.

The inventors have found that the causes of this are that blocking effect due to contact between absorbent resins occurs, swelling is blocked because space for resins to swell and increase their volume is not secured, it is difficult for water to permeate from the hydrophilic fibers to the absorbent resins because there is little bonding between the hydrophilic fibers and the absorbent resins. In view of this, as an ideal absorbent structure for solving these three problems at the same time, the inventors thought of an absorbent structure (absorbent sheet) in which the absorbent resins and the hydrophilic fibers are sufficiently bonded, the absorbent resins are not in contact with each other and, to secure space for the absorbent resins to swell, the absorbent resins are sterically arranged in a grid formed by fibers.

A large amount of fibers or absorbent resins detaching from the absorbent sheet is not preferable. An absorbent sheet with such detachment occurring is limited to applications in which fine powders may detach, and there is also a possibility that unevenness is observed in performance. Accordingly, it is preferable that the absorbent resins be substantially fixed in the absorbent sheet.

Until now, a number of methods for fixing absorbent resins have been proposed, but none of them can satisfy both water-absorption capability and adhesiveness. Conventional fixing methods are classified into a method using an adhesive and a method using no adhesive. As an adhesive, a hydrophilic one and a hydrophobic one are available. Examples of hydrophilic adhesives include soluble components that absorbent resins with a high degree of crosslinking contain. However, the inventors have found that these soluble components cause dry comfort to deteriorate. As a hydrophobic adhesive, one that is melted by heat is generally used. However, to obtain adhesive force for completely fixing small absorbent resins, it is necessary to use an excessive amount of adhesive and to excessively melt it. The inventors have found that this results in the hydrophobic adhesive covering surfaces of the resins, thereby causing both the absorption speed and the absorption amount to decrease, it is also difficult to adhere the resins by applying heat uniformly over a whole area, and thus when heat is applied to a part where heat cannot easily reach, a part where heat can easily reach excessively melts, further resulting in the adhesive covering the absorbent resins. Examples of methods using no adhesive include a method of performing polymerization in a base material and a method of making hydrophilic fibers on a surface of a base material penetrate among absorbent resins by using water. However, the inventors have ascertained that when performing polymerization in the base material, because it is difficult for polymerization to proceed, there is a problem that residual monomers are likely to remain, low molecular components increase, and dry comfort is poor, and also ascertained that the method of making hydrophilic fibers on a surface of a base material penetrate among absorbent resins by using water is a method satisfying both fixation and absorbed amount, but because the resin particles are almost two-dimensionally arranged on the surface of the base material and the amount of the hydrophilic fibers effectively used is small, there is a problem that absorption speed is considered insufficient. Particularly, when placing emphasis on quick absorption, to gain the absorption speed of the absorbent resins themselves, resins whose particle size is especially small are used. In this case, the inventors have ascertained that particles become likely to come in contact with each other when the absorbent resin particles are arranged on the surface of the base material.

The inventors thought an alienation between the problem of the conventional technique on fixation and the above-described ideal absorbent structure is due to a fact that small absorbent resins cannot be sterically arranged as a main problem. In other words, the inventors thought an absorbent sheet closer to the ideal structure can be obtained by mixing absorbent resins and bulky hydrophilic fabrics in advance, not only directly bonding them to secure conduits but also increasing the apparent volume of the absorbent resins, and further forming a sheet of the absorbent resins and the hydrophilic fabrics while utilizing entanglement between the fibers to sterically arrange them. This bonding is preferred to be hydrogen bonding between the absorbent resins and the hydrophilic fibers or direct bonding by which the hydrophilic fibers are incorporated into surfaces or insides of the absorbent resins and, from a viewpoint that the absorption speed improves by sending a liquid into the absorbent resins, the hydrophilic fibers are preferred to be incorporated into the surfaces or insides of the absorbent resins. The inventors have found that such bonding by which the hydrophilic fibers are incorporated into surfaces or insides of the absorbent resins can be performed by using water and dehydrating and drying them, for example. However, it is hard to obtain sufficient adhesive force merely by bonding and entanglement between the absorbent resins and the hydrophilic fibers, it is difficult to form them into a sheet and, if no measures are taken, it is difficult to construct a sheet having few detaching components. Accordingly, the inventors thought of using hydrophobic thermoplastic fibers, making a grid with the thermoplastic fibers to build a basic skeleton of the sheet, and arranging bonded substances of the absorbent resins and the hydrophilic fibers in the grid.

Using hydrophobic fibers as the thermoplastic fibers is considered preferable from a viewpoint of dry comfort. Although a difficulty in using thermoplastic hydrophobic fibers to form a sheet is to control the degree of melting, the absorbent resins in the absorbent sheet of the present invention are not absorbent resins alone, but they are absorbent resins that are mixed and bonded with the hydrophilic fibers and thus are bulky, and further entanglement between the hydrophilic fibers and the hydrophobic fibers is considered usable. Accordingly, because the total amount of heat can be small, the surfaces of the absorbent resins will not be covered by hydrophobic materials. In addition, because permeation of water between grid sections made of the hydrophobic fibers is performed by the hydrophilic fibers, the absorbent resins are considered to be effectively usable.

To achieve full performance over the whole area, it is preferable to contrive a way to homogenize the degree of melting by uniformly applying heat. This can be achieved by moisture control in the system, for example. If water is present in a space for forming a sheet by heating, the water is in equilibrium and desorbs and adsorbs repeatedly between the absorbent resins or the fibers and the space. During this time, because the hydrophilic fibers serve as conduits, the amount of moisture in the system is leveled without unevenness, whereby the temperature in the system is homogenized until the moment when the moisture disappears. Accordingly, the temperature in the system can be homogenized up to 100°C at the boiling point of water. When hydrophobic fibers having a glass-transition temperature equal to or lower than 100°C are used, the hydrophobic fibers can be uniformly heated and fused by this homogenization and, also when hydrophobic fibers having a glass-transition temperature equal to or higher than 100°C, differences between positions can be reduced because temperature increase from 100°C to the forming temperature becomes small unlike the case of heating from room temperature. Such advantages of moisture are not limited to the temperature control. Between the hydrophobic fibers and the absorbent resins having particularly strong hydrophilicity, repulsion acts because of the presence of water. This makes it possible to reduce probability that the absorbent resins are coated by the hydrophobic fibers. Furthermore, this repulsion makes the grid size of the hydrophobic fibers larger, thereby forming a sufficient space for the absorbent resins to swell. In general, when the grid size becomes larger, displacement of absorbent resins becomes likely to occur, but if using mixed and bonded substances of the absorbent resins and hydrophilic fibers, this problem can be avoided, and thus a particularly preferable structure can be formed.

According to the present invention, the ideal absorbent structure for quickly and completely absorbing liquid can be built in which the hydrophilic fibers and the absorbent resins are directly bonded without using an adhesive, the hydrophobic fibers serve as an adhesive, but the hydrophobic fibers form a grid while the hydrophobic fibers and the absorbent resins are hardly bonded directly to each other, and the hydrophilic fibers and the absorbent resins that are mixed and bonded are sterically arranged in the grid with entanglement being used.

When strength is required for the absorbent sheet, it is preferable that a layer of the hydrophobic fibers whose weight ratio is 90% or more (hydrophobic fiber layer) and/or a layer of a base material exist in the sheet structure. A layer including the hydrophobic fibers whose weight ratio is high takes a shape closer to one of the base material by strongly performing thermal fusion. When performing strong thermal fusion, there is a concern that thermal fusion of the hydrophobic fibers in the absorbent layer might be excessively promoted because heat is generally applied also to the absorbent layer, but when water is present in the system, it is possible to strongly fuse only the hydrophobic fiber layer containing no water while suppressing temperature increase in the absorbent body in which the hydrophilic fibers and the absorbent resins exist. These layers can be used as layers for providing dry comfort on the surface, and can serve as a top sheet for a disposable diaper or a sanitary product. In addition, by controlling the thickness of the layers, the layers can be used as layers for stopping liquid, and can serve as a back sheet for a disposable diaper or a sanitary product.

Furthermore, the inventors have found that it becomes possible to generate repulsion for securing a space between the hydrophobic fibers to each other and to accelerate permeation of water from the hydrophilic fibers to the absorbent resins by using ammonium ions as a result of exhaustive research on a method therefor. The ammonium ions are preferably contained at 0.5 to 18 mass% with respect to the whole quantity of the absorbent sheet, more preferably at 1.5 to 15 mass%, further preferably at 3 to 13 mass%, still further preferably at 4 to 12 mass%, and most preferably at 5 to 10 mass%. Too small an amount of ammonium ions is not preferable because the absorbent sheet becomes inferior in absorption speed and dry comfort. As the amount of ammonium ions is larger, its effect becomes higher, but if the amount is excessively large, the amount of constituting members such as absorbent resins, hydrophilic fibers, and hydrophobic fibers becomes insufficient. The amount of ammonium ions can be obtained by immersing the sheet in a sufficient volume of saline, letting them stand for 24 hours, and then measuring the concentration of ammonium ions in the saline by ion chromatography.

Ammonium ions are ions particularly excellent in affinity for oxygen atoms of a hydroxy group, a carboxy group, and other groups. The absorbent resins are generally considered to have a good affinity therefor because they contain a hydroxy group or a carboxy group. In addition, because the affinity of the hydrophilic fibers is generally added by using a hydroxy group, the hydrophilic fibers have a good affinity for ammonium ions. For example, cellulose fibers that are representative of the hydrophilic fibers do not dissolve in most solvents, but are known to dissolve in a copper-ammonium solution. Accordingly, because of the presence of ammonium ions, the probability of contact between the absorbent resins and the hydrophilic fibers increases, and further the speed of liquid permeation between the both improves. Conversely, the affinity of the ammonium ions for the hydrophobic fibers is particularly low, and repulsion is generated therebetween. Accordingly, in the sheet, force to expand a space between the absorbent resins and the hydrophobic fibers acts, and a space for the water-absorbent resins to swell when absorbing liquid is secured as a result. In addition, because the repulsion becomes strong because of the presence of water therebetween, an impediment to the swelling becomes unlikely to occur. Note that unlike the hydrophilic fibers, the hydrophobic fibers can maintain a space that the hydrophobic fibers form therebetween even when receiving the repulsion. Furthermore, when the hydrophobic fibers are constructed of carbon and hydrogen, the repulsion becomes stronger, and thus outer surfaces of the hydrophobic fibers are preferred to be constructed of carbon and hydrogen.

With respect to the ammonium ions, any ones of the hydrophilic fibers, the absorbent resins, and other substances having an property of fixing ammonium ions could contain them, but from a viewpoint of enhancing absorbability, it is preferable that either ones of the hydrophilic fibers and the absorbent resins contain them, and it is preferable that the absorbent resins contain them. The hydrophilic fibers are excellent in liquid-capturing capability even without ammonium ions. The absorbent resins exhibit a higher absorption speed because of an osmotic pressure difference as ionic concentration of the insides thereof becomes higher. Accordingly, it is preferable that the absorbent resins contain the ammonium ions and the ammonium ions move between the absorbent resins and the hydrophilic fibers at the start of absorption. Furthermore, it is more preferable that the absorbent resins contain the ammonium ions because repulsion between the absorbent resins and the hydrophobic fibers becomes stronger and a space for swelling is easily secured. From a viewpoint of enhancing initial absorption capability of the absorbent resins and a viewpoint of suppressing absorption of moisture, it is preferable that the absorbent resins have distribution in salt concentration. More specifically, it is preferable that the salt concentration be low at outer surfaces of the resins and the salt concentration be high at insides of the resins.

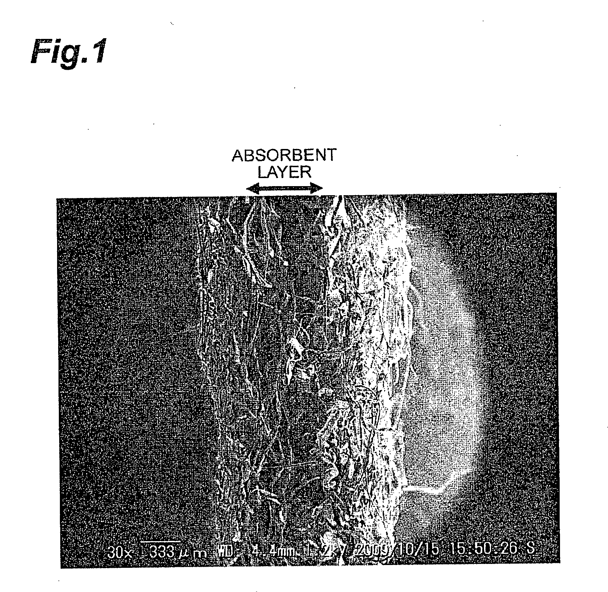

It is preferable that not only repulsion by which a space for the absorbent resins to swell during liquid absorption act but also the space be secured in the absorbent structure. More specifically, it is preferable that the absorbent sheet according to the present embodiment can include an absorbent layer containing the absorbent resins and a hydrophobic fiber layer containing the hydrophobic fibers on one side or both sides of the absorbent layer, and further the fiber density of the absorbent layer be lower than the fiber density of the hydrophobic fiber layer. In addition, it can be considered to be preferable that the porosity of the absorbent layer be higher than the porosity of the hydrophobic fiber layer. The absorbent layer herein means a portion containing the absorbent resins. The absorbent resins detaching from the sheet being not preferable, it is preferable that the outermost surface layer of the sheet do not contain the absorbent resins. The outermost surface layer may be the hydrophobic fiber layer. The thickness of the outermost surface layer herein is considered to be sufficiently preferable if it is about 10 micrometers. In addition, this outermost surface containing no absorbent resins does not have to be provided on both sides of the sheet, and the provision thereof only on one side is sufficient. Low fiber density of the absorbent layer is preferable in securing a space for the absorbent resins to swell, and high fiber density in the other parts is considered preferable in enhancing liquid-capturing capability and in increasing water-permeation speed by utilizing capillarity. Fiber density in the sheet structure including the absorbent layer can be checked by cutting the absorbent sheet and observing a cross-section thereof with an SEM (see Fig. 1). Fig. 1 is a scanning electron microscope (SEM) picture of a cross-section of the absorbent sheet according to the present embodiment. In the central portion of the absorbent sheet, the absorbent layer whose fiber density is lower than that of other portion can be observed.

To lower the fiber density of the absorbent layer, it is preferable that the absorbent layer be constructed of hydrophobic fibers whose diameter is relatively large in the absorbent layer. To enhance liquid-capturing capability and increase water-permeation speed by capillarity, layers (the hydrophobic fiber layer, for example) other than the absorbent layer is preferred be constructed of hydrophobic fibers whose diameter is relatively small, and is preferred to be constructed of hydrophobic fibers whose diameter is smaller than the diameter of the hydrophobic fibers in the absorbent layer. It is considered preferable that diameters of the hydrophobic fibers be different between the absorbent layer and the other layers in the above manner. In addition, examples of a contrivance for lowering the fiber density of the absorbent layer include a method utilizing water. When ammonium ions, absorbent resins, hydrophobic fibers, and water exist, at the same time as the absorbent resins absorbs water, the ammonium ions are arranged on outer surfaces of the absorbent resins. Accordingly, repulsion to the hydrophobic fibers acts and a space is secured.

It is preferable that the hydrophilic fibers and the absorbent resins have bonding therebetween. This bonding is preferred to be hydrogen bonding between the absorbent resins and the hydrophilic fibers or direct bonding by which the hydrophilic fibers are incorporated into surfaces or insides of the absorbent resins, and from a viewpoint that the absorption speed improves by feeding liquid to the absorbent resins, it is preferable that the hydrophilic fibers be incorporated into the surfaces or insides of the absorbent resins. Such bonding that incorporates the hydrophilic fibers into the surfaces or insides of the absorbent resins can be performed by using water and dehydrating and drying them. When the absorbent resins and the hydrophilic fibers are bonded, even small absorbent resins are apparently considered to have the same volume as that of bulky fibers, and further it is preferable because the resins become unlikely detach by utilizing entanglement of the fibers to each other.

The ratio of the hydrophilic fibers to the hydrophobic fibers by mass in the whole absorbent sheet is preferably 9:1 to 2:8, more preferably 8:2 to 3:7, further preferably 8:2 to 4:6, and most preferably 7:3 to 5:5. As the amount of the hydrophilic fibers is larger, the probability of contact between the absorbent resins and the hydrophilic fibers becomes higher and absorption speed can be increased, but the amount of the hydrophobic fibers decreases, so that there is a possibility that the detaching components increase. As the amount of the hydrophobic fibers is larger, strength as a sheet can be easily obtained, but there is a possibility that the water-absorption speed becomes slow.

The ratio of the absorbent resins to the hydrophilic fibers by mass in the whole absorbent sheet is preferably 10:1 to 1:5, more preferably 9:1 to 1:3, further preferably 8:1 to 1:2, and most preferably 8:2 to 1:1. As the amount of the hydrophilic fibers is larger, instantaneous water-retaining capability becomes higher, but there is a possibility that dry comfort becomes poor. As the amount of the absorbent resins is larger, the dry comfort becomes excellent, but there is a possibility absorbent resins whose particle size is small cannot be fixed and detach.

It is preferable to control the performance as an absorbent body into which the absorbent sheet of the present invention and another sheet-like material are combined. Depending on the intended use of the absorbent sheet, deodorants, perfumes, various inorganic powders, foaming agents, pigments, dyes, antibacterial agents, hydrophilic short fibers, plasticizers, adhesives, surfactants, fertilizers, oxidizing agents, reducing agents, chelating agents, antioxidants, thermal stabilizers, ultraviolet absorbers, light stabilizers, salts, or the like may be contained as necessary. To further increase flexibility, the surfactants are preferred to be contained.

The absorbent sheet of the present invention can be used as an absorbent product without any change by adding thereto a water-permeable sheet, a water-impermeable sheet, or gathers, for example, as necessary. This absorbent product is thin and excellent in absorption speed and dry comfort, and thus can be preferably used for disposable sanitary materials such as disposable diapers, incontinence pads, and sanitary napkins.

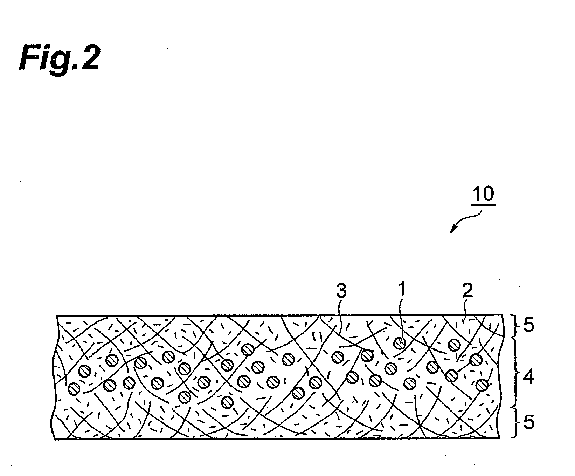

Fig. 2 is a schematic sectional view illustrating one embodiment of the absorbent sheet of the present invention. This absorbent sheet 10 depicted in Fig. 2 is constructed of absorbent resins 1, hydrophilic fibers 2, and hydrophobic fibers 3, and provided with an absorbent layer 4 containing the absorbent resins 1 and a hydrophobic fiber layer 5 containing the hydrophobic fibers 3 on one side or both sides of the absorbent layer 4. The fiber density in the absorbent layer 4 is lower than the fiber density of the hydrophobic fiber layer 5. In addition, in the absorbent layer 4, the hydrophobic fibers 3 may be substantially fused with each other. Furthermore, it is preferable that the area density of the hydrophobic fibers 3 in the absorbent layer 4 be 10 to 50 $g/m^2$ and the ratio of the area density of the hydrophilic fibers 2 to the area density of the hydrophobic fibers 3 be 10:90 to 70:30. In addition, it is preferable that the area density of the absorbent resins 1 be 10 to 50 $g/m^2$ and the thickness of the absorbent sheet 10 be 2 millimeters or less. Fig. 2 illustrates an absorbent sheet provided with the hydrophobic fiber layer 5 in plurality on both sides of the absorbent layer 4, but the hydrophobic fiber layer 5 may be provided only on one side of the absorbent layer 4 or the hydrophobic fiber layer 5 may be unprovided.

[2. Method for Producing Absorbent Sheet]

A method for producing an absorbent sheet of the present invention will now be described. The method for producing an absorbent sheet of the present invention includes at least (1) a step ("dehydration and drying step", also referred to as a "dehydration step" in the present specification) of performing dehydration in such a state that at least the hydrophilic fibers and the absorbent resins are in contact with each other and (2) a step (sheet-forming step) of continuously performing shaping in a sheet form by heating.

At the dehydration step, it is preferable to perform dehydration from such a state that the absorbent resins and the hydrophilic fibers are in contact with each other and contain water. By the dehydration step, composite compositions constructed of the absorbent resins and the hydrophilic resins are obtained. At the sheet-forming step, it is preferable to form a sheet by heating while bringing the composite compositions into contact with the hydrophobic fibers. The absorbent sheet of the present invention can be one in which the composite compositions are dispersed among the hydrophobic fibers by the above-described production method, for example.

In the absorbent sheet of the present invention, it is important that substances of the hydrophilic fibers and the absorbent resins mixed and/or bonded are arranged among the hydrophobic fibers. The hydrophilic fibers and the absorbent resins are needed to be mixed and/or bonded to obtain an improvement effect on absorption speed, and this can be performed by the dehydration and drying step of (1). It is preferable to achieve bonding by which the hydrophilic fibers are incorporated into surfaces or insides of the absorbent resins by adjusting conditions.

To fix the resins and the fibers, (2) a step (sheet-forming step) of continuously performing shaping in a sheet form while melting the hydrophobic fibers by heating is necessary. By this step, the ideal absorbent structure can be continuously formed.

The dehydration and drying step of (1) can be performed simultaneously with the sheet-forming step of (2), or can be performed separately. When performing simultaneously, it is acceptable to perform sheet forming by heating while performing dehydration and drying in such a state that the hydrophilic fibers and the absorbent resins containing water are in contact with each other. For example, it is acceptable to disperse the hydrophilic fibers, the hydrophobic fibers, and the absorbent resins on a line, spray water, and perform dehydration and drying while heating them. When performing separately, it is preferable to perform the dehydration and drying step of (1) in advance and perform the sheet-forming step of (2). It is preferable to perform the dehydration and drying step at a separate step and further to continue dehydration and drying also at the sheet-forming step because this increases productivity.

It is preferable that the contact area between the hydrophilic fibers and the absorbent resins be larger to enhance absorption capability, and it is preferable that the hydrophobic fibers be arranged uniformly over the whole area of the sheet to increase sheet strength. This can be preferably performed, for example, by contriving a way such as alternately supplying respective raw materials of the hydrophilic fibers and the absorbent resins (or composite compositions thereof) and the hydrophobic fibers onto the sheet-forming line.

At the sheet-forming step, in such a state that the hydrophilic fibers and the absorbent resins (or composite compositions thereof) are brought into contact with the hydrophobic fibers on a support, they can be heated. The support is preferred to be a fibrous support (e.g., pulp) in which the content ratio of hydrophobic fibers is 90 mass% or more.

Because the absorption speed becomes faster as the contact area between the hydrophilic fibers and the absorbent resins is larger, it is preferable that the hydrophilic fibers and the absorbent resins be uniformly mixed. In addition, it is preferable that the hydrophobic fibers be distributed more uniformly because sheet strength and absorption capability become uniform. Accordingly, the method for producing the absorbent sheet is preferred to include (3) a step ("first mixing step", also referred to as a "mixing step 1" in the present specification) of mixing the hydrophilic fibers and the absorbent resins and bringing them into contact with each other, and (4) a step ("second mixing step", also referred to as a "mixing step 2" in the present specification) of mixing the hydrophilic fibers and the absorbent resins (or composite composition thereof) and the hydrophobic fibers and bringing them into contact with each other. When performing on the sheet-forming line, it is possible to mix them by physical force using an emboss roll, a needle roll, or air, for example, before the sheet forming by heating.

It is possible to mix them on the sheet-forming line, but the sheet-forming line is preferred to move at high speed, and thus it is preferable that the method for producing the absorbent sheet separately include (3) the step (mixing step 1) of mixing the hydrophilic fibers and the absorbent resins and (4) the step (mixing step 2) of mixing the hydrophilic fibers and the absorbent resins (or composite composition thereof) and the hydrophobic fibers because these steps facilitate the mixing. The mixing step 1 of (3) and the mixing step 2 of (4) can also be performed simultaneously. The method of mixing is not particularly limited, and it is acceptable to perform mixing using air flow, perform mixing using a mixer, for example, or perform mixing using a solvent.

Timings when the mixing step 1 and the mixing step 2 are performed are not particularly limited if they are before the sheet-forming step of (2) or simultaneous. The timings may be before and after the dehydration and drying step of (1) or simultaneous. In addition, with respect to the mixing step 1 of (3) and the mixing step 2 of (4), either one of them may be performed before the other, or they may be performed simultaneously. To enhance the contact between the absorbent resins and the hydrophilic fibers, the mixing step 1 of (3) is preferred to be performed before the dehydration and drying step of (1).

To increase bonding force between the absorbent resins and the hydrophilic fibers, it is preferable to increase the water content in the absorbent resins and perform dehydration and drying at high temperature for a long period of time. In this case, to prevent excessive melting of the hydrophobic fibers, it is preferable that the mixing step 2 of (4) be performed after the dehydration and drying step of (1). When performing the dehydration and drying step of (1) and the sheet-forming step of (2) in the same line, time required for the dehydration and drying is estimated to be long compared to the heating for sheet forming. Accordingly, it is necessary to upsize a dryer for dehydration and drying or to reduce the speed of the line, which possibly deteriorates the productivity. The same applies when performing the dehydration and drying step of (1) and the sheet-forming step of (2) simultaneously. By performing heating steps for the dehydration and drying and the sheet forming in separate lines, it is possible to increase productivity of the sheet itself while enhancing the bonding between the absorbent resins and the hydrophilic fibers.

The sheet-forming step of (2) and the dehydration and drying step of (1) are preferred to be performed in the separate lines, but in this case, they do not have to be performed in the same place. For example, it is preferable to perform the dehydration and drying step of (1) in a place to produce the absorbent resins. It is also considered preferable to perform the mixing step 1 of (3) and/or the mixing step 2 of (4) in the place to produce the absorbent resins, and it is more preferable to perform the mixing step 1 of (3) before the dehydration step of (1) in particular. In the present invention, substances obtained at the dehydration and drying step (1) are defined as composite compositions, and the composite compositions can be transported and distributed similarly to the absorbent resins.

At the mixing step 2 of (4) and/or the mixing step 1 of (3), other raw materials may be mixed together. For example, it is considered preferable to mix together a functional substance that is effective near the absorbent resins such as a coloring agent, a deodorant, an antibacterial agent, a water-permeating agent depending on purposes. In addition, to enhance entanglement between the fibers, it is preferable to mix a plurality of fibrous materials. Fibers whose materials are different or fibers whose sizes or lengths are different can be combined to be used. The mixing method is not particularly limited, mixing may be performed by using air flow, mixing may be physically performed by using a mixer, for example, mixing may be performed by using a solvent, or a method of simultaneously dispersing the absorbent resins and the fibers may also be used.

At the mixing step 1 of (3), when water is present during the mixing, suitable interaction acts between the hydrophilic fibers and the absorbent resins, so that miscibility becomes excellent. However, because uniform mixing becomes difficult when a large amount of water is present, the total amount of water in the system before the mixing with respect to the mass of the absorbent resins is preferably 0.1 to 100 mass% (i.e., 0.1 to 100 parts by mass per 100 parts by mass of the absorbent resins), more preferably 1 to 40 mass%, further preferably 1.5 to 20 mass%, and most preferably 2 to 12 mass%. Water could be contained by the hydrophilic resins and/or the absorbent resins. Because absorption capability decreases in some cases when preserving the absorbent resins in a state of containing water, when preserving as raw materials, it is preferable that the hydrophilic fibers contain water. In this case, if the hydrophilic fibers are preserved in a humid atmosphere, they naturally contain water. When the absorbent resins are used immediately, the absorbent resins may contain water, and a preferable water range can be set by using resins that are not completely dried after synthesis.

Because the dehydration and drying step of (1) is preferred to be performed in such a state that the hydrophilic fibers and the absorbent resins are efficiently in contact with each other, this step is preferred to be performed after the mixing step 1 of (3). In the present specification, the term "dehydration" or "drying" means reducing the water content in the absorbent resins, the hydrophilic fibers, the composite compositions, the hydrophobic fibers, or a mixture thereof.

The dehydration method is not particularly limited, examples of the method include a method by heating, a method by decompression, and a method by air flow, and a plurality of methods may be combined. From a viewpoint of enhancing the bonding between the hydrophilic fibers and the absorbent resins, the heating is preferred to be performed. The method of the heating is not particularly limited, a method by hot air, a method using microwaves, a method using infrared rays, or other methods can be freely selected depending on facilities. The heating temperature is preferably 60 to 250°C, more preferably 80 to 200°C, further preferably 100 to 180°C, and most preferably 120 to 150°C. Drying efficiency tends to be low when the heating temperature is low, and the absorbent resins may color when the heating temperature is excessively high.

The degree of dehydration is not particularly limited, but the ratio of the water content in the absorbent resins after drying to the water content therein before the drying is preferably 70% or less, more preferably 50% or less, further preferably 30% or less, and most preferably 10% or less.

The water content before drying (i.e., the total water content in the absorbent resins and the hydrophilic fibers in a state of containing water) is not particularly limited, but is preferably 20 to 1000 mass% with respect to the mass of the absorbent resins (i.e., 20 to 1000 parts by mass per 100 parts by mass of the absorbent resins), more preferably 40 to 600 mass%, preferably 60 to 400 mass%, and most preferably 80 to 200 mass%. The bonding between the absorbent resins and the hydrophilic fibers decreases in number when the water content before drying is low, and drying time tends to become long when it is excessively high.

The water content after drying is not particularly limited, but is preferably 1 to 200 mass%, more preferably 2 to 100 mass%, further preferably 4 to 40 mass%, and most preferably 5 to 20 mass%. With excessive water, water remains in the sheet thereby possibly deteriorating the absorption capability when the dehydration and drying step of (1) and the sheet-forming step of (2) are simultaneously performed, and drying time tends to become long when the present step is performed before the sheet-forming step of (2). With insufficient water, when the sheet-forming step is provided separately from the present step, the amount of water brought into the sheet-forming step is insufficient, control of sheet-forming conditions by using the hydrophobic fibers tends to become difficult.

To form the bonding efficiently, it is preferable to increase the amount of water up to a predetermined amount after the mixing step 1 of (3) and then perform drying. The method of increasing the amount of water is not particularly limited, and examples of the method include a method of immersing in water and a method of spraying water. The water to be absorbed may contain impurities. Examples of impurities include cations such as sodium ions, ammonium ions, and iron ions; anions such as chlorine ions; and water-soluble organic compounds such as acetone, alcohols, ethers, and amines. An acidic or basic substance may be used to adjust the pH of the absorbent resins and/or absorbent composites. From a viewpoint of contact between the absorbent resins and the base material and absorption capability, it is preferable that the content of impurities be equivalent to the level of tap water, and it is more preferable to use distilled water or ion-exchange water without impurities alone.

To increase productivity of the sheet sufficiently, it is preferable that to dehydrate and dry the hydrophilic fibers and the absorbent resins in advance before the sheet-forming step of (2). The total water content in the composite compositions and the hydrophobic fibers before heating at the sheet-forming step is preferably 1 to 200 mass% with respect to the mass of the absorbent resins (i.e., 1 to 200 parts by mass per 100 parts by mass of the absorbent resins), more preferably 2 to 100 mass%, further preferably 4 to 40%, and most preferably 5 to 20%. With excessive water, the drying time tends to become long and, with insufficient water, the control of sheet-forming conditions by using the hydrophobic fibers tends to become difficult. The water content after the sheet forming is preferably 20 mass% or less with respect to the mass of the absorbent resins, more preferably 10 mass% or less, further preferably 5 mass% or less, and most preferably 1 mass% or less.

It is considered preferable to perform an absorbent product producing step after the sheet-forming step of (2). The absorbent sheet of the present invention can be used without any change by adding thereto a water-permeable sheet, a water-impermeable sheet, or gathers, for example, as necessary. This absorbent product can be preferably used for disposable sanitary materials such as disposable diapers, incontinence pads, and sanitary napkins. The producing step of the absorbent product is preferred to include a feeding step of a top sheet that is water-permeable, and/or a back sheet that is water-impermeable. Conventional methods for producing sanitary materials have a problem that it is difficult to improve productivity because the sanitary materials are mixed with supporting materials such as pulp and they need to be packed in a bag so as not to leak, but it becomes possible to dramatically improve the productivity by performing a simple producing step of the absorbent product after the producing steps of the absorbent sheet of the present invention.

[3. Device for Producing Absorbent Sheet]

Preferred examples of a device for producing an absorbent sheet of the present invention will be described below, but the device for producing an absorbent sheet of the present invention is not limited to the description below. In particular, the examples below refer to a device when producing the absorbent sheet using the absorbent resins, the hydrophilic fibers, and the hydrophobic fibers as materials, and they are different in some cases when producing the composite compositions in advance in a place to produce the absorbent resins.

(a) Device for Pulverizing Hydrophilic Fibers

When the hydrophilic fibers are in a sheet or roll shape, the fiber length and fiber size thereof can be adjusted by pulverization. A general pulverizer for pulp sheet can be preferably used. The hydrophilic fibers pulverized can be stored in a common tank.

(b) Device for Mixing Hydrophilic Fibers and Absorbent Resins

This device is preferred to have a weighing device for mixing the hydrophilic fibers stored in a tank and the absorbent resins stored in another tank at an optional ratio. To measure the mixing ratio, a general weight measuring instrument can be used. To mix the hydrophilic fibers and the absorbent resins thus measured, it is preferable to use a container in which air flow mixing can be performed, and it is possible to use a hopper, for example, on the inside of which a spiral groove is cut, for example. A mixture accumulating in a bottom portion can be sent to the next step, for example, by controlling the rotational speed using a screw, for example.

(c) Device for Increasing Water Content

When the amount of water in the absorbent resins is small, it is preferable to increase the water content. To make the absorbent resins contain water uniformly, it is preferable to supply the hydrophilic fibers and the absorbent resins to a conveying device such as a conveyor and to increase the water content while conveying them thereon. As a device for increasing the water content, a water spraying device can be preferably used. As the water spraying device, a sprayer can be used, for example.

(d) Device for Dehydration and Drying

For dehydration and drying, a hot air dryer can be preferably used. This dryer may be installed on the above-mentioned conveyor, or may be a fixed dryer. When drying time is long, the fixed dryer is preferred. The composite compositions dried can be stored in a tank, for example.

(e) Device for Mixing Composite Compositions and Hydrophobic Fibers

A device similar to one of the above-mentioned step (b) can be preferably used, but is preferred to have a structure for flowing air to feed mixed substances to the sheet-forming step. By controlling the amount of air flow, a specific amount can be supplied to the sheet-forming step.

(f) Sheet-Forming Device

Sheet forming can be performed by a conveyor method, for example, in which a metal net conveyor or other conveyors excellent in heat conductivity can be preferably used. To uniformize the thickness of raw materials supplied to the conveyor, the sheet-forming device is preferred to have a device for sucking from underneath the net. When using a sheet, the sheet-forming device is preferred to have a sheet-feeding unit. In addition, the sheet-forming device may have a device for uniformly supplying the hydrophobic fibers, the hydrophilic fibers, and the absorbent resins. It is preferable that pressing be performed to unformize the thickness after all raw materials are supplied. For the pressing, a roller press can be preferably used, for example, and a surface of the roller press may be embossed, for example. To increase the efficiency at the subsequent heating step, the temperature of the press may be raised. As a device for melting and forming a sheet of the hydrophobic fibers by heating, a common hot air dryer can be used, which can continuously apply heat and perform sheet forming. This dryer may be of a tunnel-dryer type in which a conveyor passes through the dryer, or may be of a drum type. The sheet-forming device is preferred to have a pressing device to make the thickness uniform after heating. When a sheet thus formed is used as a product without being additionally processed, the sheet can be wound up around a wind-up device. A slitting device for making the width uniform may be provided. When the hydrophilic fibers, and/or the absorbent resins, and/or the hydrophobic fibers are not mixed in advance, respective raw materials can be directly supplied onto the conveyor and formed in a sheet by similar operation.

(g) Device for Producing Absorbent Product

The absorbent sheet formed at step (f) can be sent to a general absorbent product producing step without being wound up or additionally processed. For example, with a device for feeding a permeable sheet referred to as what is called a top sheet, a device for feeding an impermeable sheet referred to as what is called a back sheet, and an adhesion device in their original conditions, the absorbent product can be produced, which can significantly facilitate steps for already available absorbent products. As a matter of course, it is possible to provide also a device for adding gathers and a tape, for example, used in general absorbent product production and a clicker cutter.

The device for producing an absorbent product is not particularly limited in the present invention, and a device used for producing a conventional absorbent sheet can be used. More specifically, while a continuum of material for the back sheet is being conveyed along a circumferential surface of an operating drum, the absorbent sheet of the present invention is supplied and fixed onto the continuum of material for the back sheet positioned on the circumferential surface of the operating drum, and then the continuum of material for the back sheet is cut into lengths each corresponding to a dimension of the absorbent product. It is preferable to provide the top sheet in the absorbent product of the present invention, and the absorbent product can be produced by supplying a continuum of material for the top sheet to the operating drum and fixing it to the continuum of the material for the back sheet and the absorbent sheet before cutting the continuum of the material for the back sheet into lengths each corresponding to the dimension of the absorbent product.

[4. Absorbent Resins]

In the absorbent resins contained in the absorbent sheet of the present invention, the residual monomer concentration with respect to the whole quantity of the absorbent resins is preferably 200 ppm by mass (hereinafter, also referred to as simply "ppm") or less, more preferably 100 ppm or less, further preferably 50 ppm or less, and most preferably 10 ppm or less. It is not preferable for performance that residual monomers be present in high concentration because they elute in large quantity during liquid absorption.

The residual monomers can be reduced by completing polymerization by heat treatment during or after producing the absorbent sheet, but the residual monomer concentration in material of the absorbent resins before producing it is preferably 5% or less, more preferably 1% or less, further preferably 0.1% or less, and most preferably 0.05% or less. It is not preferable to use the absorbent resins with many residual monomers as starting materials because it becomes difficult to complete the polymerizing reaction of the residual monomers during producing the sheet and a large quantity of residual monomers are likely to remain at the end. In addition, the texture is adversely affected by the reaction method in some cases.

For such reasons, in the present invention, it is preferable to use absorbent resins that are polymerized in advance. The shape of the absorbent resins is not particularly limited, but is preferred to be a shape of particle. Although not particularly limited, examples of the absorbent resins include spherical ones produced by suspension polymerization, amorphous ones into which an aqueous solution polymer is pulverized, porous ones each having an increased specific surface area, and ones each of which is formed with a plurality of spherical particles flocculated. The particle size is not particularly limited, but is preferably 10 micrometers or more because generation of fine powders causes a problem in handling of the absorbent resins. It is more preferably 40 micrometers or more and further preferably 80 micrometers or more. The absorbent resins can be appropriately classified with a sieve, for example.

The amount of residual monomers can be measured by the following method. The absorbent resins are added to 0.9% saline in the amount of 250 times the weight of the resins, and the residual monomers are extracted for about 6 hours by stirring at room temperature, and filtered. The amount of residual monomers in the filtrate is determined by liquid chromatography.

In the present invention, the type of absorbent resin is not particularly limited, and any kind of absorbent resin can be used, but an absorbent resin having acid groups in the side chains is preferred, and a resin having carboxy groups in the side chains is further preferred. Examples of carboxy group-containing units include units derived from polymers such as acrylic acid, methacrylic acid, itaconic acid, maleic acid, crotonic acid, fumaric acid, sorbic acid, cinnamic acid, and anhydrides thereof and neutralized salts thereof.

In the absorbent resin having acid groups in the side chains, 30% or more of the acid groups are preferred to be neutralized in the form of salts, 50% or more thereof are more preferred, 70% or more are further preferred, and 90% or more are most preferred. The type of neutralized salts is not limited, but 30% or more of the salts are preferred to be neutralized in the form of ammonium salts, 50% or more thereof are more preferred, 70% or more thereof are further preferred, and 90% or more thereof are most preferred.

The absorbent resin having acid groups in the side chains is preferred because the absorption speed is increased due to static repulsion between the acid groups during liquid absorption. It is preferable for the acid groups to be neutralized because thereby the liquid is absorbed into the inside of the absorbent resin by osmotic pressure and also because thereby bonding to the hydrophilic fibers can be easily formed with the salts. It is preferable that the acid groups are neutralized in the form of ammonium salts because ammonium salts have a high affinity for water thereby increasing the absorption amount and strong bonding to the hydrophilic fibers can be easily formed. However, because the pH is likely to become basic when the acid groups are neutralized, when using absorbent resins for sanitary materials, the percentage of acid groups neutralized as salts in the whole acid groups contained in the absorbent resins after forming the absorbent sheet is preferably 30 to 90% and more preferably 50 to 80%. In other words, it is particularly preferable that the percentage of neutralized salts decrease at around the mixing step 1 with the hydrophilic fibers and the hydration and drying step.

The absorbent resin may have a unit that does not contain a carboxy group, and examples thereof include hydrophilic units derived from nonionic compounds such as acrylamide, methacrylamide, N-ethyl (meth)acrylamide, N-n-propyl (meth)acrylamide, N-isopropyl (meth)acrylamide, N,N-dimethyl (meth)acrylamide, 2-hydroxyethyl (meth)acrylate, 2-hydroxypropyl (meth)acrylate, methoxypolyethylene glycol (meth)acrylate, polyethylene glycol mono(meth)acrylate, N-vinylpyrrolidone, N-acryloylpiperidine, and N-acryloylpyrrolidine; and hydrophobic units derived from compounds such as (meth)acrylonitrile, styrene, vinyl chloride, butadiene, isobutene, ethylene, propylene, stearyl (meth)acrylate, and lauryl (meth)acrylate.

The absorbent resin may also contain a unit to be a crosslinking agent between polymer molecular chains. Examples thereof include units derived from compounds such as diethylene glycol diacrylate, N,N'-methylenebisacrylamide, polyethylene glycol diacrylate, polypropylene glycol diacrylate, trimethylol propane diallyl ether, allylglycidyl ether, pentaerythritol triallyl ether, pentaerythritol diacrylate monostearate, bisphenol diacrylate, isocyanuric acid diacrylate, tetraallyloxyethane, and diallyloxyacetic acid salt.

The absorbent resin may also contain a unit to be a crosslinking agent by condensation with the carboxy group-containing units. Examples thereof include glycidyl ether compounds such as ethylene glycol diglycidyl ether, trimethylol propane triglycidyl ether, (poly)glycerine polyglycidyl ether, diglycerine polyglycidyl ether, and propylene glycol diglycidyl ether; polyvalent alcohols such as (poly)glycerine, (poly)ethylene glycol, propylene glycol, 1,3-propanediol, polyoxyethylene glycol, triethylene glycol, tetraethylene glycol, diethanolamine, and triethanolamine; polyvalent amines such as ethylenediamine, diethylenediamine, polyethylenimine, and hexamethylenediamine; and polyvalent ions such as zinc, calcium, magnesium, and aluminum.

Many types of absorbent resin are known, including crosslinked partially neutralized polyacrylic acid (see Japanese Patent Application Laid-Open Publication No. S55-84304, for example), hydrolyzed starch-acrylonitrile graft polymer (see Japanese Examined Patent Application Publication No. S49-43395, for example), neutralized starch-acrylic acid graft polymer (see Japanese Patent Application Laid-Open Publication No. S51-125468, for example), saponified vinyl acetate-acrylic acid ester copolymer (see Japanese Patent Application Laid-Open Publication No. S52-14689, for example), hydrolyzed acrylonitrile copolymer or acrylamide copolymer (see Japanese Examined Patent Application Publication No. S53-15959, for example), and polyglutamic acid salts (see Japanese Patent Application Laid-Open Publication No. 2003-192794, for example). Polyacrylic acid salt copolymers and crosslinked partially neutralized polyacrylic acid that are commonly used for sanitary materials are preferred from a viewpoint of absorption capability and cost, for example.

Crosslinked polyacrylic acid as a preferred example of absorbent resin to be used and a production method thereof will be explained below. In crosslinked polyacrylic acid, preferably 50 mol% or more, more preferably 80 mol% or more, or further preferably 90 mol% or more of the repeating units in the polymer molecule chains are carboxy group-containing units. When the percentage of the carboxy group-containing units in the repeating units is insufficient, absorption capability deteriorates in some cases. It is preferable that parts of the carboxy groups in the polymer molecule chains are neutralized (partially neutralized), and examples of the salts include alkali metals such as sodium, potassium, and lithium; and nitrogen-containing basic substances such as ammonia. Preferably 30% or more, more preferably 50% or more, further preferably 70% or more, or most preferably 90% or more of the carboxy groups are neutralized. However, when using for sanitary materials, the percentage of neutralized one in the carboxy groups that the absorbent resins in the absorbent sheet have is preferred to be 50 to 80%. In terms of the kind of salt from a viewpoint of the pH, it is preferable that the carboxy groups be partially neutralized with at least one kind including sodium, potassium, ammonia, and lithium, it is more preferable that the carboxy groups be partially neutralized with sodium and/or ammonia, and it is most preferable that the carboxy groups be partially neutralized with ammonia alone. From a viewpoint of absorption capability, 50 mol% or more of the carboxy group neutralized salts in the polymer molecule chains are preferred to be ammonium salts, and more preferably 70 mol% or more thereof, further preferably 90 mol% or more thereof, or most preferably all thereof are neutralized with ammonium salts. It is preferable that the percentage of ammonium salts is high from a viewpoint of absorption capacity and adhesiveness to the hydrophilic fibers. The percentage of ammonium salts in the absorbent resin can be calculated from the total amount of nitrogen atoms in the absorbent resin. The total amount of nitrogen atoms in the absorbent resin can be obtained by the Kjeldahl method.

It is preferable that the resins in the absorbent sheet have a distributed structure in which the ammonia neutralization rate of the carboxy groups in the insides of the resins is higher than the ammonia neutralization rate of the carboxy groups at resin outer surfaces. The ammonia neutralization rate of the carboxy groups in resin central parts is preferably 50 mol% or more, more preferably 60 mol% or more, and most preferably 70 mol%, and the ammonia neutralization rate of the resin outer surfaces is preferably lower than 50 mol%, more preferably lower than 45 mol%, and most preferably less than 40 mol%. In addition, the difference between the neutralization rates of the resin central parts and the resin outer surfaces is preferably 5 mol% or more and further preferably 10 mol% or more. It is preferable that the ammonia neutralization rate in the resin central parts fall into the above ranges because decrease in water-absorption capacity under no pressure is unlikely to occur. In addition, it is preferable that the ammonia neutralization rate at the resin outer surfaces fall into the above ranges because the water-absorption capacity under pressure is unlikely to decrease. Note that this represents a state of absorbent resins in final products.

As the salt concentration at the outer surface of the absorbent resins (also referred to as "surface salt concentration") is higher, adhesiveness to the hydrophilic fibers can be increased, and thus it is preferable that the surface salt concentration before adhesion to the hydrophilic fibers (i.e., before dehydration in the dehydration step) be high and the surface salt concentration after adhesion be low. In addition, when performing the dehydration step and the sheet-forming step simultaneously, it is preferable that the surface salt concentration before adhesion to the absorbent resins be high and the surface salt concentration of the absorbent resins after adhesion be low. Note that in the present specification, the salt concentration is synonymous with the neutralization rate of acid groups (carboxy groups). The resin outer surfaces represent parts exposed outside the resins. In addition, the resin central parts represent parts that are most inner from resin outer surfaces of the resins. It is preferable that the absorbent resins each have a core-shell structure inside each resin, but the carboxy group neutralization rate that is an average for the resins as a whole is preferably 30 mol% or more, and more preferably 50 mol% or more. When the average carboxy group neutralization rate of the resins as a whole extremely decreases, decrease in water-absorption capacity under no pressure occurs in some cases.

The salt concentration of the resins can be obtained by measuring the carboxy group neutralization rate by the microscopic ATR method that is one of infrared absorption analysis methods.

The carboxy group neutralization rate at the resin outer surfaces is measured by directly examining the resin outer surfaces by the microscopic ATR method. The carboxy group neutralization rate in the resin central parts is measured by the microscopic ATR method after cutting open the resins and exposing the central parts by using an ultramicrotome (ULTRACUT N manufactured by Reichert). As a measurement device, FTS-575 manufactured by Bio-Rad Laboratories, Inc. can be used, for example. As an index defining the composition ratio of carboxyic acid and carboxyate, the peak area ratio (1695/1558 cm$^{-1}$) of peak area at 1695 cm$^{-1}$ (carboxyic acid $v$C=O baseline 1774 to 1616 cm$^{-1}$) and peak area at 1558 cm$^{-1}$ (carboxyate $v$COO$^-$ baseline 1616 to 1500 cm$^{-1}$) was calculated. The composition ratio is determined based on a calibration curve that was separately prepared by measuring partially crosslinked polyacrylic acids in which 10 mol%, 30 mol%, 50 mol%, 70 mol%, 90 mol%, and 100 mol% of whole carboxyic acid are neutralized with ammonia as standard samples.

Water-absorption capability of the absorbent resins is preferred to be higher, and the water-absorption capacity in water-absorption capacity measurement under no pressure is preferably 50 g/g or more, and more preferably 55 g/g or more. In addition, the water-retaining capacity in water-retaining capacity measurement is preferably 33 g/g or more, more preferably 36 g/g or more, and most preferably 39 g/g. In addition, the water-absorption capacity under pressure at 57 g/cm$^2$ is preferred to be 10 g/g or more.

The absorption capacity of absorbent resin particles is preferred to be higher because the quantity of absorbent resin particles used can be reduced. Note that in the present specification, the absorption capacity of absorbent resin particles represents the amount of 0.9% saline that the absorbent resin particles can freely absorb by swelling in a state without any load thereon. The absorption capacity of the absorbent resin particles is measured by the following method. Put 0.05 gram of absorbent resin particles uniformly into a tea-bag type nonwoven pouch (60x40 mm), and immerse the bag in 0.9% saline at 23°C. After 180 minutes, take out the tea bag, and obliquely suspend the bag with its corners fixed for 10 minutes to drain water, and measure the weight. Perform the same measurement for the sample without absorbent resin particles, and measure the weight as a blank value. Calculate the absorption capacity using (Formula 4). Measure the value three times, and determine the average of them as the absorption capacity.

$$(\text{Formula 4}) \quad \text{Absorption capacity of absorbent resin particles}$$
$$(g/g)=\{(\text{weight of tea bag after absorption})-(\text{weight of blank tea bag}$$
$$\text{after absorption})-(\text{weight of absorbent resin particles})\}/(\text{weight of}$$
$$\text{absorbent resin particles})$$

The water-absorption capacity under pressure of the absorbent resin particles of the present invention is measured by the following method. Place 0.02 gram of absorbent resin particles in an acrylic resin tube with an inner diameter of 25 millimeters and a height of 30 millimeters having a 250-mesh nylon nonwoven fabric on the bottom, place a smoothly moving cylinder in the tube to make it the measurement device, and measure the weight. Apply load by placing a 278.33 gram load (corresponding to 0.8 psi) on top of the cylinder of the measurement device, and place the device in a 120 mm Petri dish containing 60 grams of 0.9% saline. After 60 minutes, take out the measurement device and let it stand for 3 seconds on a Kimtowel to drain water, weigh the device after unloading the load, and calculate the water-absorption capacity under pressure according to (Formula 5).

$$(\text{Formula 5}) \quad \text{Water-absorption capacity of absorbent resin}$$
$$\text{particles under pressure } (g/g)=(\text{weight of device after absorption}$$
$$(g))-\text{weight of device before absorption } (g))/(\text{weight of absorbent resin}$$
$$\text{particles})$$

One example of a preferred method for producing the absorbent resin used in the present invention is a method of polymerizing a monomer solution containing unsaturated carboxyic acid ammonium salts with a radical polymerization initiator and drying the resins obtained. This example will be described hereinafter.

The unsaturated carboxyic acid ammonium salt monomer is not particularly limited, examples thereof include ammonium salts such as (meth)acrylic acid, itaconic acid, (dehydrated) maleic acid, crotonic acid, fumaric acid, 2-(meth)acryloyl ethanesulfonic acid, and 2-(meth)acrylicamide-2-methylpropanesulfonic acid, and preferably ammonium salt of (meth)acrylic acid is used. A method for producing the unsaturated carboxyic acid ammonium salts may be a method for derivatization such as neutralization of carboxy acid or hydrolysis of unsaturated amide compounds or unsaturated nitrile compounds with microorganisms.

Monomers other than that of unsaturated carboxyic acid ammonium salts can be added. Examples of other monomer components include (meth)methyl acrylate; (meth)ethyl acrylate, (meth)acrylamide, (meth)acrylonitrile, vinyl acetate, hydroxyethyl (meth)acrylate, methoxyethyl (meth)acrylate, and hydroxypropyl (meth)acrylate, and one of these or two or more thereof can be added at a concentration of 50 mol% or less in all monomer components. A compound to be a condensation crosslinking agent for the carboxy group added as a crosslinking agent may be added. Examples of the condensation crosslinking agent include glycidyl ether compounds such as ethylene glycol diglycidyl ether, trimethylol propane triglycidyl ether, (poly)glycerine polyglycidyl ether, diglycerine polyglycidyl ether, and propylene glycol diglycidyl ether; polyvalent alcohols such as (poly)glycerine, (poly)ethylene glycol, propylene glycol, 1,3-propanediol, polyoxyethylene glycol, triethylene glycol, tetraethylene glycol, diethanolamine, and triethanolamine; polyvalent amines such as ethylenediamine, diethylenediamine, polyethylenimine, and hexamethylenediamine; and polyvalent ions such as zinc, calcium, magnesium, and aluminum.

It is acceptable to copolymerize a polymerizable crosslinking agent as another crosslinking agent. Examples of the polymerizable crosslinking agent include diethylene glycol diacrylate, N,N'-methylenebisacrylamide, polyethylene glycol diacrylate, polypropylene glycol diacrylate, trimethylol propane diallyl ether, allylglycidyl ether, pentaerythritol triallyl ether, pentaerythritol diacrylate monostearate, bisphenol diacrylate, isocyanuric acid diacrylate, tetraallyloxyethane, and diallyloxyacetic acid salt. In these polymerizable crosslinking agents, N,N-methylenebisacrylamide or trimethylol propane triacrylate is particularly desirable.

For the purpose of extending surface areas of the absorbent resins, it is preferable to add a foaming agent. As a foaming agent, known carbonate can be used. As carbonate, any carbonate or hydrogen carbonate containing salt or mixed salt can be used, but examples of carbonates more preferred for the present invention include sodium carbonate, sodium hydrogen carbonate, potassium hydrogen carbonate, ammonium carbonate, ammonium hydrogen carbonate, magnesium carbonate, calcium carbonate, barium carbonate, and hydrates thereof, and one of them or two or more thereof are used. Carbonates particularly preferred for the present invention are monovalent cations such as carbonate or hydrogen carbonate of sodium, potassium, and ammonium. When carbonates constructed of polyvalent cationic species are used, polymers having carboxy groups are metal-crosslinked by the polyvalent cationic species, whereby water-absorption capability is adversely affected.

The rate of the carbonate added to the monomer aqueous solution with respect to the monomer components is preferably 0.01 to 10 mass% and more preferably 0.1 to 5 mass%. When the addition rate of the carbonate is lower than 0.01 mass%, a water-absorbing gel obtained by the polymerization is not porous. In addition, when the carbonate is added at a rate higher than 10 mass%, water-soluble components increase and also water-retaining capability is deteriorated. The carbonate is preferred to be added before ultraviolet irradiation, and as an addition method, the carbonate may be added without being processed, or the carbonate may be dissolved in any solvent to be added as a carbonate solution.

For the purpose of controlling the foaming agent and the foaming timing thereof, an anti-foaming agent can be used. As an anti-foaming agent, known one such as a foam-breaking agent, a foam-suppressing agent, and a foam-adjusting agent can be used, and one of them or two or more thereof in combination can be used. Specific Examples of the anti-foaming agent include oils and fats, fatty acids, lower alcohols, higher alcohols, metal soaps, silicones, hydrophobic silica-silicone compounds, fatty acid esters, polyglycols, polyglycol esters, polyethers, modified silicones, oil-soluble polymers, organic phosphorus compound, sulfated fatty acids, polyether derivatives, and silica-modified silicone compounds.

The solvent for the monomer solution is not particular limited as long as it has excellent dissolving properties for monomers. Water alone is particularly preferred, but hydrophilic solvents such as ethanol, methanol, and acetone may also be used either singly or in combination of two or more. Salts such as sodium chloride and a basic compound such as ammonia for controlling pH may also be added. As a method for polymerization, a known method such as solution polymerization can be used, and aqueous solution polymerization is preferred from a viewpoint of saving energy because an organic solvent does not have to be used. As for the type of a reactor, one that initiates polymerization by heating and/or ultraviolet irradiation, and either of batch-type and continuous-type may be used. A device using an endless belt that is a known reaction device may also be used. The method of polymerizing the unsaturated monomers is not particularly limited, and a commonly used method such as aqueous solution polymerization, reverse-phase suspension polymerization, reverse-phase emulsion polymerization, spray polymerization, belt polymerization or the like may be used. The polymerization initiation method is also not particularly limited, and polymerization may be initiated by using a radical polymerization initiator, by exposure to radiation or electron beams, for example, or by using a photosensitizer in ultraviolet polymerization. The initiator used in radical polymerization may be a known initiator examples of which include persulfate such as potassium persulfate, ammonium persulfate, and sodium persulfate; hydrogen peroxide; and organic peroxide such as cumene hydroperoxide, t-butyl hydroperoxide, and peracetic acid. When using an oxidizing radical polymerization initiator, a reducing agent such as L-ascorbic acid or Rongalite may also be added.

It is preferable that polymerization be initiated by ultraviolet irradiation using a radical photopolymerization initiator and peroxide as the polymerization initiation method. Examples of the radical photopolymerization initiator include benzoin, benzyl, acetophenone, benzophenone, and derivatives thereof. In addition, examples of the derivatives include benzoin derivatives such as benzoin methyl ether, benzoin ethyl ether, benzoin isopropyl ether, and benzoin isobutyl ether; acetophenone derivatives such as diethoxyacetophenone, 2,2-dimethoxy-1,2-diphenylethane-1-on, 1-hydroxycyclohexyl phenyl ketone, 2-methyl-1-(4-(methylthio)phenyl)-2-morpholinopropane-1, 2-benzyl-2-dimethylamino-1-(4-morpholinophenyl)-butanon-1, and 2-hydroxy-2-methyl-1-phenylpropane-1-on; and benzophenone derivatives such as methyl o-benzoylbenzoate, 4-phenyl benzophenone, 4-benzoyl-4'-methyl diphenyl sulfide, 3,3',4,4'-tetra(t-butylperoxycarbonyl)benzophenone, 2,4,6-trimethylbenzophenone, 4-benzoyl-N,N-dimethyl-N-[2-(1-oxo-2-prophenyloxy)ethyl]benzene metanaminium bromide, (4-benzoylbenzyl)trimethylammonium chloride, 4,4-dimethylamino benzophenone, and 4,4-diethylamino benzophenone.

As other radical photopolymerization initiators, azo compounds can also be used, and an azo nitrile compound, an azo amide compound, an alkyl azo compound, and the like can be used. In this case, however, because a relatively large amount of compound thereof needs to be added and thus it is difficult to achieve high polymerization, it is preferable to use a radical photopolymerization initiator having benzoyl groups.

The addition rate of the polymerization initiator with respect to the monomer components is preferably 0.0001 to 0.1 mass%, and more preferably 0.001 to 0.01 mass%. The degree of polymerization becomes extremely low when the addition rate of the photopolymerization initiator is lower than 0.0001 mass%, and the amount of oligomers tends to increase and the amount of water-soluble components tends to increase when it is higher than 0.1 mass%.

To reduce the amount of residual monomers, it is preferable to use a peroxide. Examples of preferred peroxides as known initiators include persulfate such as potassium persulfate, ammonium persulfate, and sodium persulfate; hydrogen peroxide; and organic peroxide such as cumene hydroperoxide, t-butyl hydroperoxide, and peracetic acid. One of the above peroxides or two or more thereof in combination can be used. The addition rate of the peroxide with respect to the monomer components is preferably 0.001 to 10 wt%, more preferably 0.01 to 1 wt%. It becomes difficult to sufficiently reduce the residual monomers when the addition rate of the peroxide is lower than 0.001 wt%, and the amount of water-soluble components increases and also the absorbent resins obtained color in some cases when 10 wt% or more thereof is added.

It is preferable to perform oxygen removal operation in the monomer solution in advance before initiating polymerization. More specifically, dissolved oxygen is removed by bubbling with an inactive gas for a sufficient period of time. In addition, it is desired that an inactive gas such as nitrogen or helium be substituted for the atmosphere in the reactor. In the present invention, the concentration of dissolved oxygen in the monomer aqueous solution is preferably 4 ppm or less, and further preferably 1 ppm or less. When the concentration of dissolved oxygen exceeds 4 ppm, polymerization initiation time delays and reaction is not completed, and thus the amount of residual monomers increases in some cases. Furthermore, the inside of the reactor may be under reduced pressure, normal pressure or increased pressure.

It is preferable to initiate polymerization by ultraviolet irradiation from a viewpoint of controlling polymerization. In this case, it is desirable to make sufficient ultraviolet rays pass through the unsaturated monomer aqueous solution. The thickness of the monomer aqueous solution is preferably 50 millimeters or less, and further preferably 20 millimeters or less to control the reaction temperature (maximum reaching temperature of the polymer) and to sufficiently secure the passage of the ultraviolet rays. When the thickness of the monomer aqueous solution exceeds 50 millimeters, ultraviolet irradiation cannot be performed uniformly, and thus the polymer becomes uneven in some cases. The lower limit of the thickness of the monomer aqueous solution is not particularly limited, but is preferred to be 1 millimeter or more in consideration of productivity.

The light quantity of ultraviolet rays is not particularly limited, but is generally preferred to be 10 to 10000 mJ/cm$^2$. Polymerization becomes insufficient in some cases when the light quantity is smaller than this range, and crosslinking points in the polymer obtained are cut due to excessive irradiation and the amount of water-soluble components increases in some cases when the light quantity is larger than this range, both of which are not preferable. As a light source used for the ultraviolet irradiation, conventional known light sources can be used, and a mercury lamp, a metal halide lamp, or the like may be used in consideration of reaction conditions, for example. The wavelength of the irradiation light is not particularly limited, and light having wavelength of 200 to 450 nanometers are generally used. The period of time for ultraviolet irradiation is determined to obtain the above light quantity and, under the above-described conditions, polymerization is initiated immediately after the irradiation is started, and the polymerization is generally completed sufficiently with irradiation for a short time period of 10 to 180 seconds.

The polymerization initiation temperature is preferably 0 to 30°C. The temperature of the unsaturated monomer aqueous solution before ultraviolet irradiation is preferably maintained at 30°C or lower, and more preferably maintained at 0 to 20°C. When the temperature of the monomer aqueous solution exceeds 30°C, the temperature of the reaction system becomes excessively high, so that there is a possibility that molecular weight is lowered, water-retaining capability is reduced, and the amount of water-soluble components is increased. The lower limit of the monomer aqueous solution temperature is not particularly limited as long as the monomer aqueous solution does not freeze at the temperature and generally it is 0°C or higher.

The concentration of the monomer aqueous solution is not particularly limited as long as it is within a range in which the monomers can dissolve, but the concentration is preferably 10 to 70 mass% and, when particularly using ammonium acrylate as the monomers, the concentration is most preferably 30 to 65 mass% from a viewpoint of cost efficiency and reaction controllability.

When the water-soluble unsaturated monomers start polymerization, the temperature in the system increases, but the maximum reaching temperature in the system is controlled preferably at 120°C or lower, and more preferably at 100°C or lower to obtain excellent absorbent resins. When the maximum reaching temperature in the system exceeds 120°C, the amount of water-soluble components in the polymer obtained by polymerization of the monomer aqueous solution increases in some cases also water-retaining capability becomes inferior in some cases. Various methods can be considered as a method for controlling the maximum reaching temperature during polymerization, for example, a method of cooling the contact portions of the polymer from outside or a method of blowing cold air onto the polymer, for example, can be considered, but these method requires a large facility, and accordingly, it is desirable to adopt the above-described conditions that are conditions that the concentration of the monomer aqueous solution is 30 to 65%, the temperature of the monomer aqueous solution is 30°C or lower, and the thickness of the monomer aqueous solution is 50 millimeters or less, preferably 1 to 20 millimeters, to control the maximum reaching temperature in the system at 120°C or lower.

After polymerization, hydrous gelled resin is generated in solution polymerization. It is preferable to coarsely grind and dry this resin. The resin can be pulverized into particles of about some hundreds of micrometers after drying. The particle size distribution ranges desirably from 3000 micrometers to 1 micrometers, particularly desirably from 1000 micrometers to 30 micrometers, and further desirably from 600 to 100 micrometers. As a method for the coarsely grinding, a device that can cut and extrude a rubber-like elastic body can be used, which can be easily achieved by a known technique with a cutter-type cutting machine, a chopper-type cutting machine, a kneader-type cutting machine, and the like. It is preferable to use a cutter-type cutting machine because degradation of polymer is little due to its share when cutting the gel. To pulverize a dried gel, a conventionally known method of pulverization can be adopted. For example, the dried gel can be pulverized in a desired particle size with a vibration-type pulverizer, an impact-type pulverizer, a friction-type pulverizer, or the like. The hydrophilic fibers may be mixed with the resin particles at the same time during pulverization and/or after pulverization.

The drying method is not particularly limited, but vacuum drying or hot-air drying is desired. As a dryer that can be used in the present invention, a general dryer or furnace can be used, examples of which include a hot-air dryer, a fluidized-bed dryer, a pneumatic conveying dryer, an infrared dryer, and a dielectric heating dryer. The drying temperature is in a range preferably from 70°C to 180°C, and more preferably from 100 to 120°C.

If hydrophilic fibers are mixed into the gel before drying, composite compositions of the hydrophilic fibers and absorbent resins can be formed. This mixing can be performed any time, but it is preferable to perform the mixing after shapes of particles are made uniform to a certain degree because the mixing can be performed more uniformly. More specifically, it is preferable to coarsely grind the hydrous gel, perform preliminary drying, perform pulverization, and then mix the resulting particles. The preliminary drying herein means drying to obtain dryness allowing preferable pulverization.

After drying, heat treatment may be on the absorbent resins. By the heat treatment, it is possible to promote polymerization and adjust the degree of crosslinking. It is acceptable to perform heating in the same dryer continuously after the heating, or to perform heating at a step separate from the drying step. This heat treatment is preferred to be performed with the hydrophilic fibers present.

The surface strength of the absorbent resins before the heat treatment is preferably 0.1 to 5.5 N, more preferably 0.1 to 5 N, further preferably 0.2 to 4 N, or most preferably 0.2 to 3 N. Resins having such a low surface strength cannot be used in a conventional absorbent structure because blocking is severe therein. However, it is advantageous in the present invention that these resins can easily form strong bonds with the hydrophilic fibers.

By performing heating with the hydrophilic fibers and water present, composite compositions having many bonds can be formed. It is preferable to continue to perform the heating even after bonds are formed and the amount of water is reduced, to promote crosslinking of the absorbent resins, and to enhance the surface strength. In this case, it is preferable that a condensation crosslinking agent, for example, exists because thereby the degree of crosslinking on the surfaces of the absorbent resins increases, effects of what is called a surface crosslinking can be obtained, and the bonding force with the hydrophilic fibers increases. The condensation crosslinking agent, for example, may be contained in advance in the absorbent resins, or may be added before the hydration and drying when forming the composite compositions.

The surface strength is a parameter indicating the tendency of the particle surfaces to be deformed. When absorbent resin particles that have absorbed to certain times and have swollen are placed in a container and subjected to load, the gel moves and deforms so as to fill gaps between the absorbent resin particles that are packed in the container with the gaps between them. Because the surface strength is the elastic modulus of the absorbent resin particles when they have absorbed liquid and reached their actual volumes, it signifies the degree of interaction between gel particles and the tendency of the surfaces to be deformed. High surface strength of the absorbent resin particles means that the absorbent resin particles are not easily deformed. This not being easily deformed means that there are cases in which bonds between the resins and the hydrophilic fibers are not easily formed. The surface strength of the absorbent resin particles of the present invention is determined as follows.

Equipment: Shimadzu Autograph AG-1

Sample: Precisely weigh 0.10 gram of absorbent resin particles and place them uniformly on the bottom of a cylindrical container with an inner diameter of 20.5 millimeters and a height of 50 millimeters on the bottom of which a nylon sheet with a pore size of 75 micrometers is pasted beforehand. Prepare a Petri dish with a diameter of 50 millimeters and fill it with 0.90 gram of saline, and let the cylindrical container containing the absorbent resin particles stand in the Petri dish to cause them to absorb the saline and swell for 1 hour.

Measurement: Prepare a 1 kN load cell, and attach thereto a cylindrical shaft with a diameter of 19.7 millimeters. Set the measurement range at 0.2 kN, adjust the position of the load cell at the height where no load is applied thereon, and set the load cell to descend at a fixed rate of 0.6 mm/min. Measure the pressure loaded on the load cell over time. The surface strength herein is indicated by the load (N) at the point when the particles reach their actual volume. The actual volume of the absorbent resin particles was calculated based on the relative density of saline of 1.010 g/cm$^3$ and the relative density of the absorbent resin particles.

As a condition for the absorbent resin to form bonding easily, the salt concentration near the surfaces of the absorbent resin particles is preferably 50 mol% or more, more preferably 60 mol% or more, further preferably 70 mol%, sill further preferably 80 mol% or more, and most preferably 85 mol% or more. The bonding strength increases as the surface salt concentration becomes higher. The surface salt concentration of the absorbent resin particles in the final absorbent sheet is not particularly limited, but it is preferably 90 mol% or less, more preferably 80 mol% or less, and further preferably 60 mol% or less. It is advantageous that the surface salt concentration of the absorbent resin particles in the final sheet is low because stickiness of the sheet is unlikely to occur even when the sheet is exposed to humid air. This is also especially preferable because the diffusivity of aqueous solution in the absorbent composite can thus be maintained high even if the particles come in contact with each other during swelling after absorbing liquid.

To maintain high absorption capacity, it is necessary to increase the salt concentration of the absorbent resin particles as a whole, but to maintain high liquid diffusivity in the composite compositions, it is desirable to lower the salt concentration near the surface. In other words, it is preferable to increase the internal salt concentration while reducing only the surface salt concentration. More specifically, the surface salt concentration is reduced by preferably 10 mol% or more, more preferably 20 mol% or more, and further preferably 30 mol% or more with respect to the salt concentration at the resin central part. The term "near the surface" means the outer layer of a thickness of about 1 micrometer from the surface of the resin.

It is preferable to adjust the salt concentration near the surface because the adjustment is performed by heating at the same time as the formation of bonds with the hydrophilic fibers whereby a high level of balance between bonding strength and absorbability can be obtained. The temperature in this heat treatment is preferred to be higher than the temperature in the drying treatment by 10°C or higher. The heating conditions include two elements of heating temperature and heating time, which significantly affect absorption capability of the absorbent resins and/or the composite compositions. They vary depending on required properties, but the heating temperature is higher than that of the drying condition by preferably 10°C or higher, further preferably 20°C or higher, and most preferably 30°C or higher, and also the heating temperature range is preferably 100 to 250°C, more preferably 115 to 200°C, and further preferably 130 to 170°C. The heating time is preferably 10 seconds to 5 hours, more preferably 30 seconds to 1 hour, and further preferably 1 minute to 30 minutes. Crosslinking proceeds slowly and it takes time when the heating temperature is insufficient, and it is difficult to control crosslinking in some cases when the heating temperature is excessively high.

In a case that the absorbent resins are formed mainly by ammonium salts, when heat treatment is performed under the above conditions, the neutralization rate at the resin outer surfaces of the absorbent resins and the neutralization rate in the resin central parts thereof can be adjusted within preferred ranges. As these preferred ranges, the ammonia neutralization rate of the carboxy groups in the resin central parts is 50 mol% or more, preferably 60 mol% or more, and most preferably 70 mol% or more, and the ammonia neutralization rate of the carboxy groups at the resin outer surfaces is less than 50 mol%, preferably less than 45 mol%, and most preferably less than 40 mol%. Difference between the neutralizations of the resin central parts and the resin outer surfaces is preferably 5 mol% or more, and further preferably 10 mol% or more. It is preferable that ammonia be present because this presence facilitates bonding between the hydrophilic fibers and the absorbent resins. Note that the heat treatment device is not particularly limited, and a known device such as a hot air dryer, a fluidized-bed dryer, or a Nauta-type dryer can be used.

The weight-average particle size of the absorbent resin particles used in the present invention is preferably 30 to 500 micrometers, more preferably 50 to 400 micrometers, further more preferably 80 to 300 micrometers, and most preferably 100 to 250 micrometers. When the average particle size is insufficient, the amount of absorbed water decreases in some cases. When it is excessively large, the absorption speed becomes slow in some cases.

In the present specification, the particle size of the absorbent resin particles is determined by sieve classifying with sieves having sieve openings of 20 micrometers, 25 micrometers, 32 micrometers, 38 micrometers, 45 micrometers, 53 micrometers, 63 micrometers, 75 micrometers, 90 micrometers, 106 micrometers, 212 micrometers, 300 micrometers, 425 micrometers, 500 micrometers, 600 micrometers, 710 micrometers, 850 micrometers, 1000 micrometers, 1180 micrometers, 1400 micrometers, 1700 micrometers, and 2500 micrometers. In the present specification, the intermediate value between the value for the sieve openings of one sieve through which the particles pass and the value for the sieve openings of another sieve through which they cannot pass is determined to be the particle size. Note that the particle size of particles that pass through a sieve having sieve openings of 20 micrometers is determined to be 10 micrometers, and the particle size of particles that remain on a sieve having sieve openings of 2500 micrometers is determined to be 2700 micrometers.

In addition, the percentage of the absorbent resin particles that can pass through a sieve having sieve openings of 90 micrometers is preferably 50 mass% or less, more preferably 30 mass% or less, and further preferably 10 mass% or less. In addition, the percentage of particles that cannot pass through a sieve having sieve openings of 425 micrometers is preferably 50 mass% or less, and more preferably 30 mass% or less. Furthermore, the percentage of particles that cannot pass through a sieve having sieve openings of 300 micrometers is preferably 70 mass% or less, more preferably 50 mass% or less, and further preferably 30 mass% or less. It is preferable to use the absorbent resins having such relatively sharp particle size distribution because it becomes easy for them to be uniformly arranged in a grid formed by the fibers.

The amount of the absorbent resins with respect to the absorbent sheet is preferably 20 to 300 $g/m^2$, more preferably 50 to 250 $g/m^2$, further preferably 70 to 200 $g/m^2$, and most preferably 90 to 180 $g/m^2$. Dry comfort deteriorates in some cases when the amount is insufficient, and a further effect cannot be obtained when the amount is excessively large.

[5. Hydrophilic Fibers]

The hydrophilic fibers in the present invention are not particularly limited as long as they can retain a liquid, and any hydrophilic fibers can be used. The hydrophilic fibers are not particularly limited as long as they can retain a liquid, but particularly cellulosic fibers are preferred. The cellulosic fibers in the present invention mean fibers mainly made from cellulose. As the cellulose, one derived by treatment such as esterification or etherification may be used. One that is mixed with other fibers may also be used. Examples of cellulose include cotton, hemp, rayon, polynosic, lyocell, cupra, and pulp. Among these, pulp is preferred. As the pulp, either of wood pulp and non-wood pulp may be used. Examples of the non-wood pulp include bagasse, grass, straw, and bamboo. Alternatively, recycled old newspaper may be used as pulp, but virgin pulp directly produced from wood, for example, is preferred when used as sanitary materials.

The average fiber length of the hydrophilic fibers with which the absorbent resins form composite compositions in the present invention is preferably 20 to 1000 micrometers, more preferably 30 to 500 micrometers, and further preferably 100 to 300 micrometers. Effect of capturing a liquid is not sufficient when the average fiber length of the hydrophilic fibers is small, and blocking is more likely to occur in producing the composite compositions when it is large, both of which are not preferable. The ratio of the average fiber length of the hydrophilic fibers to the average particle size of the absorbent resins that form the composite compositions in the present invention is preferably 0.05:1 to 2:1, more preferably 0.1:1 to 1.5:1, and further preferably 0.5:1 to 1:1. The effect of capturing a liquid is not sufficient when the ratio of the average fiber length of the hydrophilic fibers to the average particle size of the absorbent resins is small, and blocking is more likely to occur in producing the composite compositions when it is large, both of which are not preferable.

The ratio of the absorbent resins to the hydrophilic fibers in the absorbent sheet is preferably 10:1 to 1:5, more preferably 9:1 to 1:3, further preferably 8:1 to 1:2, and most preferably 8:2 to 1:1. As the amount of the hydrophilic fibers is larger, instantaneous water-retaining capability becomes higher, but dry comfort may be inferior. As the amount of the absorbent resins is larger, dry comfort becomes excellent, but there is a possibility that absorbent resins having a small particle size cannot be fixed and detach from the absorbent sheet.

The amount of the hydrophilic fibers with respect to the absorbent sheet is 10 to 150 $g/m^2$, more preferably 20 to 100 $g/m^2$, further preferably 30 to 80 $g/m^2$, and most preferably 40 to 60 $g/m^2$. Absorption speed may be inferior when the amount of the hydrophilic fibers are insufficient, and dry comfort is inferior in some cases when the amount thereof is excessively large.

[6. Hydrophobic Fibers]

It is preferable that the hydrophobic fibers in the present invention have thermoplasticity. Any material may be used, and general polyethylene, polyester, or polypropylene, for example, can be favorably used. It is considered preferable to use fibers having a core-in-sheath structure in which the amount of fusion can be easily controlled. In this case, it is preferable that the glass-transition temperature and/or the melting point of the sheath be lower than the glass-transition temperature and/or the melting point of the core. Difference between the melting points of the core and the sheath is preferably 10°C or higher, more preferably 20°C or higher, and further preferably 30°C or higher.

The thickness of the fibers is not particularly limited, but is preferably 0.01 to 200 denier, more preferably 0.1 to 100 denier, further preferably 0.5 to 50 denier, and most preferably 1.2 to 15 denier. Space between the hydrophobic fibers becomes too small and absorption capability is deteriorated in some cases when the thickness is too small, and the feeling becomes worse in some cases when the thickness is excessively large. The fiber length is not particularly limited, but is preferably 0.1 to 20 millimeters, more preferably 0.5 to 10 millimeters, and further preferably 1 to 5 millimeters. Adhesion becomes weak in some cases when the fiber is too short, and miscibility decreases in some cases when the fiber is excessively long.

It is preferable that the hydrophobic fibers contained in the hydrophobic fiber layer and the hydrophobic fibers contained in the absorbent layer have a layer including organic resin on each of their surfaces. The organic resin herein means resin that has carbon and hydrogen as a basic skeleton.

The ratio of the hydrophilic fibers to the hydrophobic fibers in the absorbent sheet is preferably 9:1 to 2:8, more preferably 8:2 to 3:7, further preferably 8:2 to 4:6, and most preferably 7:3 to 5:5. As the amount of the hydrophilic fibers is larger, probability of contact between the absorbent resins and the hydrophilic fibers increases and absorption speed can be increased, but the amount of the hydrophobic fibers decreases and thus the number of detaching components may increase. As the amount of the hydrophobic fibers is larger, strength as a sheet can be easily obtained, but water-absorption speed may decrease.

The amount of the hydrophobic fibers with respect to the absorbent sheet is preferably 5 to 100 g/m$^2$, more preferably 10 to 80 g/m$^2$, further preferably 15 to 60 g/m$^2$, and most preferably 20 to 40 g/m$^2$. The sheet strength becomes low in some cases when the amount of the hydrophobic fibers is insufficient, and the water-absorption speed decreases in some cases when the amount is excessively large.

[7. Absorbent Product and Method for Producing Same]

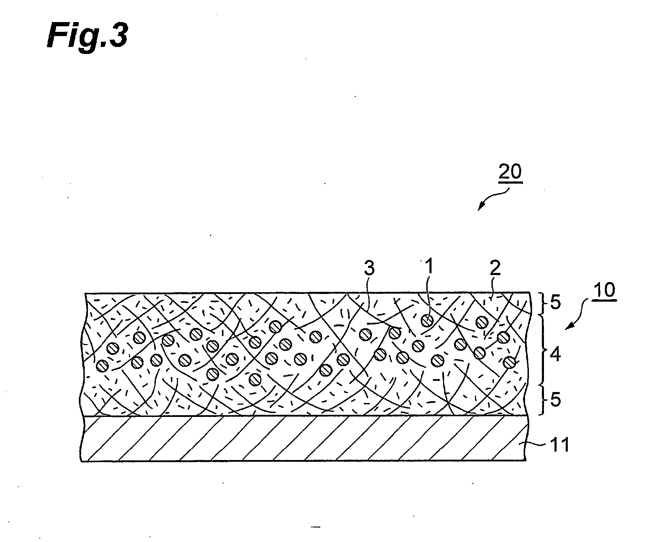

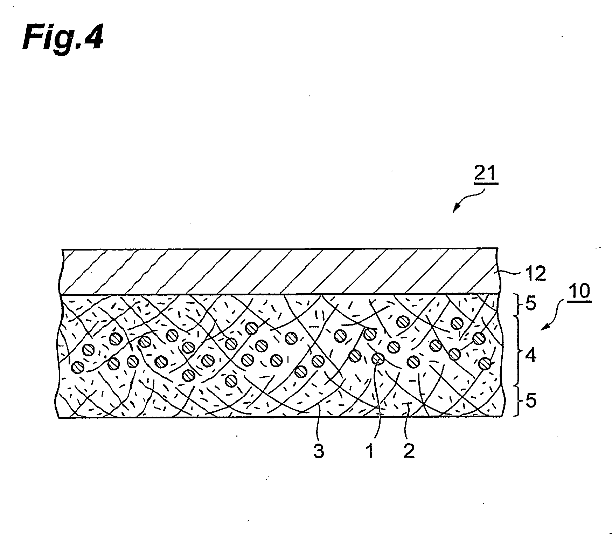

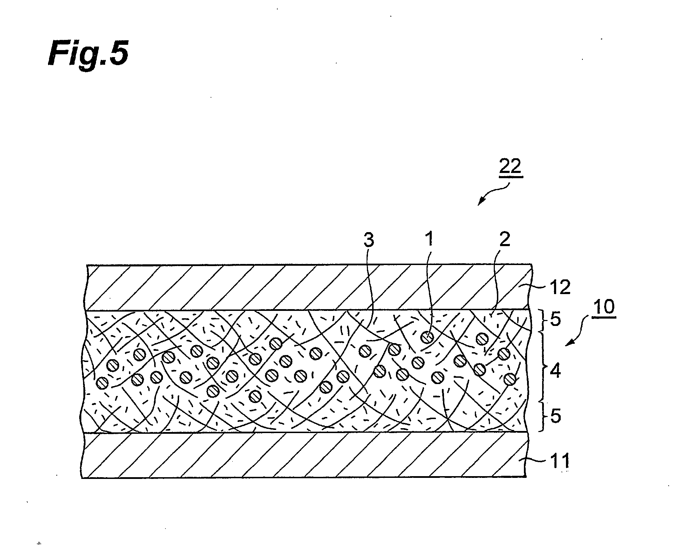

The absorbent product according to the present invention has the absorbent sheet of the present invention, a top sheet provided on one side of the absorbent sheet, and/or a back sheet provided on the other side of the absorbent sheet. Figs. 3 to 5 are schematic sectional views illustrating absorbent products according to embodiments of the present invention. An absorbent product 20 depicted in Fig. 3 includes a back sheet 11 and the absorbent sheet 10 according to the present invention provided on the back sheet 11. An absorbent product 21 depicted in Fig. 4 includes a top sheet 12 and the absorbent sheet 10 according to the present invention provided on the top sheet 12. An absorbent product 22 depicted in Fig. 5 includes the back sheet 11, the absorbent sheet 10 according to the present invention provided on the back sheet 11, and the top sheet 12 provided on the absorbent sheet 10.

The absorbent product can be produced by a production method including at least the step of adhering the absorbent sheet of the present invention to the top sheet that is continuously fed and/or the back sheet that is continuously fed.

In addition, the production method of the absorbent product can include the step of supplying and fixing an absorbent sheet that is obtained by a production method having the following steps (1) to (5) or an absorbent sheet that is obtained by a production method having the following steps (6) to (9) onto a continuum of material for the back sheet that is conveyed along the circumferential surface of the operating drum, and the step of cutting the continuum of material for the back sheet to which the absorbent sheet is fixed into lengths each corresponding to a dimension of the absorbent product:

(1) mixing hydrophilic fibers and absorbent resins;
(2) humidifying a mixture of the hydrophilic fibers and the absorbent resins;
(3) performing dehydration in such a state that the hydrophilic fibers and the absorbent resins are in contact with each other;
(4) mixing composite compositions constructed of the hydrophilic fibers and the absorbent resins with hydrophobic fibers;
(5) continuously performing shaping in a sheet form by heating;
(6) mixing hydrophilic fibers, absorbent resins, and hydrophobic fibers;
(7) humidifying a mixture of the hydrophilic fibers, the absorbent resins, and the hydrophobic fibers;
(8) performing dehydration in such a state that the hydrophilic fibers and the absorbent resins are in contact with each other; and
(9) continuously performing shaping in a sheet form by heating.

In this case, the production method can further include the step of supplying and fixing a continuum of material for the top sheet to the continuum of material for the back sheet to which the absorbent sheet is fixed before the step of cutting the continuum to which the absorbent sheet and the back sheet are fixed.

In the absorbent product according to the present invention, it is preferable that the absorbent sheet be obtained by changing the mixing ratio of the composite compositions to the hydrophobic fibers or the mixing ratio of the hydrophilic fibers, the absorbent resins, and the hydrophobic fibers in a width direction and/or a length direction of the absorbent sheet, and the content of the absorbent resins in the absorbent sheet differ depending on positions.

[8. Applications of Absorbent Sheet]

The absorbent sheet of the present invention can be preferably used for absorbent members of disposable sanitary materials such as disposable diapers, incontinence pads, and sanitary napkins, absorbent members of excretion treatment materials such as sheets for animals and pets; absorbent sheets that prevent marine products from getting wet with thawing water when frozen marine products are transported; absorbent sheets that cover potted plants to prevent water evaporation, absorbent sheets that are laid under potted plants; absorbent sheets that are arranged around a water tank; absorbent sheets that are used for sheets for dew condensation preventing material, for example; waterdrop absorbing mats that are arranged in a part such as a water receiver of an umbrella stand and absorb waterdrops dripping from an umbrella, for example; mats for a headrest cover for a vehicle; mats for preventing one's head from becoming sweaty in a helmet or a hat; toilet paper sheets that are used after defecation at an electric toilet seat with a warm-water spray feature (e.g., Washlet manufactured by TOTO, Ltd.), for example; absorbent mats that prevent a floor of an open event site from getting wet when it rains; absorbent mats that prevent a floor of a vehicle such as a car, a train, or a plane from getting wet on a rainy day; absorbent mats that prevent a floor of facilities such as a hospital, a rest area, a department store, a hotel, a store, an office building, or a recreational facility from getting wet on a rainy day; absorbent mats that prevent the inside of a refrigerator from getting wet; absorbent mats that prevent a floor of a kitchen from getting wet, and absorbent sheets that absorb drips from raw garbage in a kitchen or a cooking place; absorbent mats that prevent a floor equipped with sanitary fixtures such as a water supply system, a hot-water supply system, a toilet bowl, or a washstand from getting wet; absorbent mats that prevent a floor around a refrigerator from getting wet; a mat for leisure or a mat for massotherapy, and an auxiliary mat for a bed; packaging materials that have a water retention or humidity control function for vegetables, fruits, or flowers and ornamental plants; packaging materials that have a water retention or humidity control function for fresh fish, raw meat, a daily dish, or a packed lunch, for example; packaging materials for seeds, bacterial strains, seedlings, or bulbs; waste rags or clothes for cleaning machinery or windows, or for wiping dew condensation water and other water off ceilings, walls, floors, or windows; or sheets that prevent water evaporation when growing garden plants. Particularly, the absorbent sheet can be preferably used for disposable sanitary materials such as disposable diapers, incontinence pads, and sanitary napkins because of its excellent absorption speed and dry comfort.

The absorbent sheet of the present invention is preferred as an absorbent sheet for a grill drip pan. A grill in the present invention means a cooking device for a cooking method of grilling in which heat radiation is a major heat source of the grilling. The grill may be, other than one using a direct flame, one using an electromagnetic wave, one that heats a metal plate or a far-infrared ray ceramics by gas combustion up to high temperature and uses infrared rays radiated therefrom, or one using Joule heat by an electric heater as a heat source. Examples of the grill include a fish grill. The fish grill is generally a system that is built into a gas range and used to grill fish, for example. Foodstuffs to grill are not limited to fish. Although the fish grill have a smaller inner volume than that of a general grill or an oven, it can grill all kinds of grilled dishes whose sizes are not so large for one plate, and can handle a wide range from western dishes such as gratin, hash browns, and pizza to Japanese dishes such as grilled fish (sauries or dried marine products). However, it has been problems that when cooking without cleaning oil sticking on the inside and without placing water into the grill drip pan, the oil could catch fire and burst into flames. In a case of using water, there are problems that oil splashes when it falls into water directly and that it takes a lot of time and effort to clean up the ingrained oil. When the absorbent sheet of the present invention is used in a state of being spread in a grill pan and absorbing water, the grill pan will not get dirty because the absorbent sheet absorbs the oil. It is difficult for common absorbent sheets to absorb oil, but in the structure of the absorbent sheet of the present invention, because the sheet has voids and the hydrophobic fibers are present in the sheet, oil can be contained among the hydrophobic fibers in the sheet structure. There is an advantage that retaining oil inside reduces generation of odor. When cooking needs a large amount of oil, usage can be adopted in which a large amount of water is placed into the grill pan and then the absorbent sheet of the invention is placed thereon. In this case, the absorbent sheet floats on water, making it easier to absorb oil. Because the absorbent sheet of the present invention does not lose the shape even after being used, it can be easily removed. Accordingly, when detachment of the absorbent resins occurs, it becomes difficult to wash the grill, but detachment of the resins does not occur, and thus it is easy to wash the grill. Furthermore, because there is also an effect that the sheet absorbs odor associated with grilling, there is an advantage that it is not necessary to care about odor in the room even without turning on a fan. Furthermore, the absorbent sheet of the present invention can be used a plurality of times. Once after removing the sheet and washing the grill, the same sheet may be placed back, or the sheet can be used without being washed. When removing the sheet, it may be dried and preserved. When using the sheet without washing it, there is a possibility that pouring water might be forgotten, but generally a grill has a sealed structure, and thus the water absorbed in the absorbent sheet is easily released. Once the shape of the sheet has been deformed, fibers separated from the absorbent resins may be present, and the deformed part may get burned and an odor may stick to the food. However, because the absorbent sheets of the present invention can maintain the relation between the resins and the fibers, the above-mentioned problems will not happen. In a conventional sheet whose resins detach after absorbing water, far from being easy, cleaning after use is even troublesome, and when resins stick to food, they absorb water of the food thereby spoiling its deliciousness. Such a conventional sheet cannot be used a plurality of times. There is an example (e.g., Japanese Patent Application Laid-Open Publication No. 2005-124833) in which a conventional absorbent sheet is set in an absorbent tray, and the absorbent sheet is supposed to be disposable together with the tray, but the tray is a waste and increases the amount of garbage in itself, and also there are cases that the sheet cannot be used because of bulkiness depending on kinds of grills. Furthermore, resins may detach and spill. Because performance of a sheet of this type varies once it is used, it is difficult to reuse the sheet.

The absorbent sheet of the invention can be preferably used as an absorbent sheet for a coffin. A coffin is for a dead body to be laid to rest therein and is used for both a human and an animal. Nowadays, when a body (regardless of human or animal) is laid to rest, regardless of (coffin,) summer or winter, pieces of "dry ice" are placed beside or under the body (6 to 8 pieces in summer) and are used to absorb secretions from the body and to prevent (delay) decomposition. Because the absorbent sheet of the present invention is excellent in spreading capability of the sheet in comparison with a conventional absorbent sheet, a liquid or odor is absorbed uniformly by the whole sheet, and thus the efficiency of using the sheet is excellent. In addition, in such a conventional absorbent sheet, a dead body may be soiled or lose shape because absorbing portion does not become bloated or peeling of the sheet does not occur, for example, but such things do not happen with the absorbent sheet of the present invention. Furthermore, the absorbent sheet of the present invention is a preferred sheet that can achieve an original goal with a small amount of resources.

The absorbent sheet of the present invention can be preferably used as an absorbent sheet for dew condensation preventing material. By attaching the absorbent sheet of the present invention, dew condensation water formed on surfaces on which due condensation occurs such as window glass, window frames, and walls of buildings can be quickly and surely absorbed, and inconvenience that dew condensation water remains on window glass or frame portions can be avoided. As an absorbent sheet for dew condensation preventing material, conventionally, various ones have been available such as one utilizing absorbability of acid paper, one in which silica gel powders are compounded into neutralized paper, and one used as silica gel alone, but absorption speed of silica gel and absorption capacity of paper are limited. The absorbent sheet of the present invention is characterized in that total capacity of absorption that the hydrophilic fibers, the hydrophobic fibers and the absorbent resins have is large (at least several times larger than silica gel). In addition, even when the absorbent sheet absorbs excessive water, the water does not become solution therein. Furthermore, because rewetting is less likely to occur, water will not leak even when the sheet is in contact. The absorbent sheet can be used at a desired position to place it because it is flexible and thin by combining the respective materials in a balanced manner, and thus the absorbent sheet is preferable for a sheet for dew condensation preventing material. With only a sheet of conventional general absorbent resins, when they absorb water, the absorbent resins swell, become pasty, and thus are likely to detach from the sheet. Accordingly, the sheet of the absorbent resins need to be covered by nonwoven fabric, for example, thereby causing a disadvantage that the thickness becomes larger or costs of a production facility and raw material becomes higher. In addition, it is common practice that the sheet will not be removed for a long period of time once after it is attached on a surface of glass and the absorption capability is recovered by evaporating absorbed water by natural drying. A conventional absorbent resin sheet absorbs a large amount specifically at portion thereof that dew condensation water touches, and a liquid that overflows after saturation diffuses. Accordingly, there have been a problem that such overflowing liquid is likely to leak and a problem that it is difficult to recover the absorption capability because surface areas of the resins absorbing the liquid are small. Furthermore, there has been a problem that particularly when the amount of dew condensation water formed on a surface of glass is large and exceeds the acceptable amount of water absorption of an absorbent sheet for dew condensation preventing material before the absorption capability is recovered, the dew condensation water falls and remains in the frame. The absorbent sheet of the present invention has an advantage that absorbed water is less likely to overflow because of being absorbed uniformly by the whole sheet, and an advantage that the absorption capability can be easily recovered by transpiration due to its large surface area because the whole absorbent resins are used for water absorption. In addition, because pressure is applied on the absorbent resins that have absorbed water by the hydrophobic fibers, the resins easily release the liquid, and thus the absorbent sheet has an advantage that recovery of the absorption capability is quick.

The absorbent sheet of the present invention can be preferably used as a sweat-absorbent pad. By placing the absorbent sheet of the present invention as a sweat-absorbent pad to an armpit, a forehead, a neck, and a back or other body parts, the pad can absorb sweat and keep the body part clean. Required for a sweat-absorbent pad are water-absorption speed, dry comfort, and fit feeling. Sweat-absorbent sheets aiming at water-absorption speed and dry comfort are available, but there is no pad considered for feeing after water absorption. The absorbent sheet of the present invention has an advantage that fit feeling does not change before and after sweat-absorption because the sheet absorbs uniformly with the whole sheet. Furthermore, the absorbent sheet of the present invention is excellent in flexibility, and is thus excellent in fit feeling. Because resins do not detach, naturally, a problem of bad feeling caused by touching the absorbent resins does not occur. The absorption speed of the absorbent sheet of the present invention is sufficient because the hydrophilic fibers capture sweat quickly into its absorbent sheet structure, and also the absorbent sheet is excellent in fit feeling because the hydrophilic fibers send a liquid into the absorbent resins. There is an effect of preventing odors by absorbing ammonia. In typical production methods of conventional sweat-absorbent pads for an armpit, pads are produced in a facility for lactation pads that prevent leakage of (mother's milk) with improvement or adaptation applied, but the facility is very large and requires a large amount of investment and is moreover nonproductive (speed is slow). Conventional facilities use a production method including steps of (1) film step, (2) under-sheet supply, (3) pulverizing step, (4) absorbent resin dispersing step, (5) folding step (two or three foldings), (6) nonwoven cloth rolling-up step, (7) sealing and punching step, (8) counting, and (9) lining-up, and are very complicated, nonproductive, and money- consuming. Conventional facilities for a sweat-absorbent pad (sheet) that is produced based on the same specifications as the ones for sanitary napkins and only has a narrower width (for the forehead, the neck, the back, etc.) require equipment that costs very high. Because the absorbent sheet of the present invention can be easily produced by sending a sheet in a rolled shape to constructing equipment and simply slitting or punching the sheet in an optional shape (a sheet having a laminate on one side thereof is used when a water-preventing body is necessary), the sheet can be produced with low material cost and low production cost, making it possible to produce more excellent sweat-absorbent products than conventional ones.

The absorbent sheet of the present invention can be preferably used as a sweat-absorbent sheet for antifouling pad for clothes. The sweat-absorbent sheet for antifouling pad for clothes is an antifouling sheet for clothes that is pasted to a collar or a sleeve, for example, to prevent soiling of the clothes. Especially it is effective when used on a white shirt. Increase in temperature causes sebum dirt to be oxidized whereby clothes get dirty. Because sebum dirt is discharged from the skin with sweat, it becomes possible to prevent dirt of clothes by absorbing sebum and sweat at the same time. Because the absorbent sheet of the present invention absorbs sweat easily and also capture oil easily into the absorbent sheet, it is excellent in antifouling effect. Furthermore, the absorbent sheet is characterized by being soft and excellent in fitting, and also excellent in fit feeling even when absorbing sweat. Feeling becomes worse when detachment of resins occurs, but the absorbent sheet of the present invention does not have such problems. The absorbent sheet exhibits an odor preventing effect by absorbing ammonia.

The absorbent sheet of the present invention can be preferably used as a absorbent sheet for a bedding cover. Bedding refers to a pillow, a mattress, a comforter, a bed, and a sheet, for example. While sleeping, a person sweats a lot and sebum dirt is discharged at the same time, bedding easily gets dirty. By absorbing sebum and sweat at the same time, it becomes possible to prevent bedding from getting dirty.-The absorbent sheet of the present invention absorbs sweat easily and also capture oil easily into the absorbent sheet, and thus is excellent in antifouling effect. Furthermore, the absorbent sheet is characterized in that it is soft and excellent in fitting and also the fitting does not become worse even when absorbing sweat. When resins detach, not only the feeling becomes bad, but also a problem of cleaning occurs, but the absorbent sheet of the present invention does not have such problems. The absorbent sheet exhibits an odor preventing effect by absorbing ammonia.

The absorbent sheet of the present invention can be preferably used around foods. For example, it can be preferably used as an absorbent sheet for a thawing pad when thawing a frozen food, and as an absorbent sheet for a water removing pad for liquid dripping food materials. Dripping of a liquid from foods gives an unpleasant image and causes the foods to look less delicious. Furthermore, it gives a bad impression when a surface of absorbed sheet deforms with projections and depressions. Especially, appearance of commercial goods is important. When an absorbent resin comes in contact with foods, it takes a liquid out of foods, which causes the foods to taste less delicious. Therefore, it is essential for an absorbent resin not to detach and not to come to the surface, and it is preferable that water be not exchanged between an absorbent resin in an absorbent structure and foods. With the absorbent sheet of the present invention, this can be achieved by providing a layer with no absorbent resin to the surfaces that contact with foods. Furthermore, it can also reduce odors.

The absorbent sheet of the present invention can be preferably used as an absorbent sheet for a mask. Various kinds of masks are available on the market, but they usually have a structure of stopping foreign substances by fineness of fibers, and thus it is difficult for them to completely stop fine particles like pollen. Using the absorbent sheet of the present invention that contains water enhances the ability of the mask to stop pollen and the like. A mask having parts without a water-absorbent resin or having varying degrees of water absorption with location is not preferable because the ability to stop the pollen and the like vary. The absorbent sheet of the invention can be used preferably because it absorbs water uniformly over the whole sheet. If water-absorbent resins detach, for example, there is a problem that feeling becomes bad, but such a problem does not occur in the absorbent sheet of the invention.

The absorbent sheet of the present invention can be preferably used as an absorbent sheet for a wet proof pad of electronic equipment. Because electronic equipment such as a personal computer or a digital camera has problems that water causes not only a hardware failure of a main unit but also loss of data, it is especially required to prevent wetting when a liquid is accidentally spilled. Because it is difficult to cover all of the precision parts by an impermeable sheet, the water-absorbent sheet can be preferably used. Conventional water-absorbent sheets may cause a problem that detached resins come into parts, but the absorbent sheet of the present invention does not have such detaching components, and thus can be preferably used. In electronic equipment, because downsizing has some kind of value, there is little space for absorbing a liquid and swelling. If a part of a sheet absorbs a liquid and becomes a dam, a liquid is not absorbed any more, and accordingly it is required that the whole sheet absorbs a liquid as in the absorbent sheet of the invention.

[9. Base Material]

In the present invention , a base material may be used. Strength of the sheet is increased by the base material, whereby falling out of fibers or resins can be prevented during production, and thus waste of raw materials is reduced. The base material in the present invention means a material that can maintain the shape of the sheet.

In the present invention, the base material may be of any material that is in a sheet form, but preferably it is of paper and/or fabric. The paper herein means paper broadly defined by JIS P 0001, and fabric is a general term for sheet-shaped fiber products as defined by JIS L 0206. Fabric is classified into woven fabric, knitted fabric, braided fabric, lace, mesh, and nonwoven fabric depending on the means of forming the sheet. Fabric used in the present invention is preferably woven, knitted, or nonwoven fabric, and more preferably nonwoven fabric. Paper and/or fabric is preferred because of excellent morphologic stability, unlike pulp and other short fibers. Nonwoven fabric is defined by JIS L 0222.

The raw material of the base material is not particularly limited, and a plurality of materials may be combined into the base material. The base material fibers may be natural fibers or synthetic fibers, and a plurality of types of fibers may also be combined. The fibers may either be long fibers or short fibers. They may also be treated to increase strength or to give hydrophilicity.

Hydrophilic one is characterized by being excellent in liquid absorbability and water permeability and having a high affinity for with hydrophilic fibers and absorbent resins. Hydrophobic one is characterized by being excellent in dry comfort and having a high affinity with hydrophobic fibers. Because both of these performances are preferred as an absorbent body, a base material having both hydrophilicity and hydrophobicity is considered preferable. The base material having both hydrophilicity and hydrophobicity may be one in which two or more kinds of hydrophilic raw materials and hydrophobic raw materials may be mixed, one formed by subjecting hydrophilic raw material to hydrophobic treatment, or one formed by subjecting hydrophobic materials to hydrophilic treatment. In addition, because continuous long fibers are more excellent in liquid permeability than short fibers, hydrophilic portion is preferred to be continuous long fibers.

The shape of the base material is not particularly limited, and the thickness is preferably 0.001 millimeter to 1 centimeter, more preferably 0.01 millimeter to 5 millimeters, further preferably 0.05 millimeter to 3 millimeters, and most preferably 0.1 millimeter to 1 millimeter. The weight is 0.1 $g/m^2$ to 200 $g/m^2$, more preferably 0.5 $g/m^2$ to 100 $g/m^2$, further preferably 1 $g/m^2$ to 60 $g/m^2$, and most preferably 2 $g/m^2$ to 30 $g/m^2$. One that is too small or too light is not preferred from a viewpoint of strength.

In the present invention, the tensile breaking strength after absorption of saline is preferably 0.6 N/20 mm or more, more preferably 0.6 to 5000 N/20 mm, further preferably 0.7 to 500 N/20 mm, and still further preferably 0.85 to 100 N/20 mm, and most preferably 1 to 100 N/20 mm.

[10. Features of Absorbent Sheet of Present Invention]

The absorbent sheet of the present invention is constructed of at least the absorbent resins, the hydrophilic fibers, and the hydrophobic fibers. The absorbent sheet is in a shape of sheet, and does have almost no components that detach. The absorbent sheet can be used in combination with additional particulate materials or fibrous materials, for example, but in this case, only a part except parts that easily detach is used as an absorbent sheet. The parts that easily detach means objects that have detached from the sheet after an end of the sheet is held by hand and the sheet is reciprocated ten times in a state of being held vertically at such a speed that it takes one second for one stroke movement between a distance of 30 centimeters.

With the absorbent sheet of the present invention, for example, the absorbent sheet is cut into a rectangle of 160 millimeters long and 70 millimeters wide and let stand on a table, and 15 grams of saline is dripped thereon over 1.5 seconds. In this case, the time period (absorbent speed (sec.)) from the start of dripping to the time when the liquid becomes invisible on the surface of the absorbent sheet after absorption is preferably 13 seconds or less, and more preferably 10 seconds or less. In addition, after 5 minutes from the start of the dripping, a square of filter paper of 100 millimeters long and 100 millimeters wide is placed on the position of the dripping, a weight of 3.5 kilograms having the same bottom area as the filter paper is placed thereon, the weight is removed in 3 minutes after placing the filter paper, and then the filter paper is weighed. The increase of weight of the filter paper (rewet amount (g)) thus weighed is preferably 0.2 gram or less, and more preferably 0.1 gram or less.

[11. Composite Compositions, and Method for Producing Same]

In the present invention, the production of the composite compositions and the production of the absorbent sheet can be performed separately. Because the composite compositions can be produced in an existing facility for absorbent resins, it is preferable to produce the composite compositions in the same place to produce the absorbent resins. Accordingly, the sheet production facility can be further simplified. The composite compositions are obtained by dehydrating and drying the hydrophilic fibers and the absorbent resins in a state of being in contact with each other. The absorbent resins are obtained, for example, as hydrous gels by polymerization in many cases. In the case of aqueous solution polymerization, these hydrous gels are coarsely ground, preliminarily dried, and pulverized to make particle size uniform. During pulverization, water remains because it cannot completely vaporize. These particles and the hydrophilic fibers are mixed, and dehydrated and dried, whereby the composite compositions can be formed. In other words, the composite compositions can be obtained by a production method that includes the step of mixing the absorbent resins that are hydrous gels and contain water with the hydrophilic fibers to bring them into contact with each other and performing dehydration.

Fine particles are made through pulverization and disposed in some cases, but the disposal volume can be reduced by being combined with the hydrophilic fibers, and the production is therefore preferable. The mixing of the hydrophilic fibers may be performed by putting the hydrophilic fibers into the pulverizer. In the case of suspension polymerization, the particle sizes can be made uniform during polymerization. Generally, the absorbent resin particles containing water are recollected by filtration or centrifugation. They may be mixed with the hydrophilic fibers after being collected, but it is preferable to put the hydrophilic fibers in the pulverizer after polymerization with the solvent present because thereby uniform mixing can be achieved.

In general absorbent resins, surface crosslinking treatment is performed. Because the surface crosslinking treatment is performed by heating, the absorbent resins mixed with the hydrophilic fibers can be dehydrated and dried by using a surface crosslinking device. When adding a surface crosslinking agent, it is preferable to do so before mixing the absorbent resins with the hydrophilic fibers because of effective utilization. For example, in the case of aqueous solution polymerization, it is preferable that the particle sizes be adjusted, the surface crosslinking agent be added, and the absorbent resins be mixed with the hydrophilic fibers. For example, in the case of suspension polymerization, because the surface crosslinking agent can be dispersed uniformly on the surfaces of the particles by adding the surface crosslinking agent into the solvent after polymerization, it is preferable to subsequently add the hydrophilic fibers. It is possible to circulate the absorbent resins and the hydrophobic fibers as their composite compositions, and it is further possible to circulate them in a form of mixture with the hydrophobic fibers by performing the mixing step 2 of (4). As the surface crosslinking agent, a condensation crosslinking agent can be used.

**Examples**

Specific examples of the present invention and comparative examples will be given below, but the present invention is not limited to these examples.

(1) Measurement of water-absorption capacity of resins w/o pressure: Tea-bag method

Absorbent resins A (g) (about 0.5 g) were put uniformly into a tea-bag type nonwoven pouch (7x9 cm), and were immersed in 500 cc saline at 25°C for 1 hour. The tea-bag type pouch was taken out after the predetermined period of time, water was naturally drained for 10 minutes, and then weight B (g) of the tea-bag type pouch was measured. As a blank test, the same operation was performed with only the tea-bag type pouch without adding the absorbent resins, and weight C (g) was measured. From these values, the water-absorption capacity was determined according to the following formula.

$$\text{Water-absorption capacity (g/g)}=(B\ (g)-C\ (g))/A\ (g)$$

Measurement of absorption speed of resins: Vortex method

Fifty grams of 0.9% saline adjusted at 25°C was measured off into a 100 ml glass beaker. A stir bar of 30x8 mm was put therein, the beaker was placed on a magnetic stirrer having a tachometer, and the solution was stirred at 600 rpm. The number of revolutions was checked with a non-contact tachometer. Into the beaker, 2.00 grams of absorbent resins were measured out and placed. An absorption time was determined as a period of time from the placing of the absorbent resins to the time when the surface of the liquid becomes flat.

(3) Measurement of carboxy group neutralization rate at resin outer surface and central part

(i) Measurement device

As a measurement device, FTS-575 manufactured by Bio-Rad Laboratories, Inc. was used.

(ii) Measurement condition

Microscopic ATR method (Ge crystal plate, single reflection)
Back Ground: Air, measurement at room temperature
Aperture: $50 \times 50$ micrometers
Cumulative number of times: 100 times
The peak area ratio (1695/1558 cm$^{-1}$) of 1695 cm$^{-1}$ (carboxyic acid $vC=O$ baseline 1774 to 1616 cm$^{-1}$) to 1558 cm$^{-1}$ (carboxyate $vCOO^-$ baseline 1616 to 1500 cm$^{-1}$) was calculated based on spectral data obtained under the above-described conditions.

(iii) Preparation of calibration curve

Partially crosslinked polyacrylic acids in which 10 mol%, 30 mol%, 50 mol%, 70 mol%, 90 mol%, and 100 mol% of whole carboxyic acid are neutralized with ammonia were used as samples for preparing the calibration curve. These calibration curve samples were cut, and their central parts are measured five times for one sample by the microscopic ATR method. The calibration curve (quintic polynomial approximation curve) was prepared based on -COOH/-COO$^-$ peak area ratio. The resins were cut open with an ultramicrotome (ULTRACUT N manufactured by Reichert).

(iv) Measurement of samples

Measurement was performed in the same manner as that for the calibration curve. The resin outer surfaces were directly measured by the ATR method, and the resin central parts were measured by the ATR method after cutting open the resins with the ultramicrotome. The resin outer surfaces were measured three times for one sample, the resin central parts were measured five times for one sample, and the averages thereof were determined as measurement results.

(4) Measurement of residual monomers of resins

One gram of absorbent resins were weighed into a 300 ml beaker, 250 grams of saline was added therein, and the solution was stirred for 2 hours. After a predetermined period of time, the solution was filtered with a membrane filter, and the filtrate was analyzed by high-performance liquid chromatography. The analysis conditions of the high-performance liquid chromatography were as follows:

Column: ODS80Ts manufactured by Tosho Corp.
Column temperature: 40°C
Carrier: 10 mM calcium phosphate aqueous solution, flown at 0.7 ml/min
Detection: UV 207 nanometers
Injection amount: 50 microliters

(5) Measurement of water-absorption capacity of resins under pressure

Absorbent resins E (g) (about 0.16 g) were placed uniformly in an acrylic resin cylindrical apparatus (outer diameter 35.0 mm, inner diameter 25.5 mm, height 30 mm, weight D (g)) having a 250-mesh nylon net on the bottom, no weight was placed thereon at 0.0 g/cm$^2$, and 278.3 grams of weight (outer diameter 24.5 mm) was placed at 57 g/cm$^2$. Into a SUS Petri dish (inner diameter 120 mm), 60 milliliters of saline was placed, and the cylindrical apparatus was let stand for 1 hour. After the predetermined period of time, water was drained with a paper towel, and weight F (g) of the whole apparatus was measured with a balance. Based on the obtained value, the water-absorption capacity under pressure was determined according to the following formula.

$$\text{Water-absorption capacity (g/g)} = (F\,(g) - D\,(g) - \text{weight of weight} \\ (g)) / E\,(g)$$

(6) Measurement of water-retaining capacity of resins

As an index indicating the water-retaining capability of the absorbent resins, the "water-retaining capacity" represented by the following formula was used. The tea bag that contains the resins containing water immediately after conducting the absorption capacity measurement tests by the above-described Tea-bag method was placed in a centrifuge, dewatered at 250 G for 3 minutes, and weighed. The same operation was performed without using the absorbent resins, and the weight was measured as a blank value. Based on these values, the water-retaining capacity was calculated according to the following formula. Measurement was conducted three times, and the average was determined as the water-retaining capacity.

$$\text{Water-retaining capacity of absorbent resins (g/g)} = \{(\text{weight of} \\ \text{tea bag after dewatering by post-water-absorption} \\ \text{centrifugation}) - (\text{weight of blank tea bag after absorption}) - (\text{weight of} \\ \text{absorbent resins})\} / (\text{weight of absorbent resins})$$

(7) Measurement of surface strength of resins

Equipment: Shimadzu Autograph AG-1
Sample: A 0.10-gram mass of absorbent resin particles were precisely weighed, and placed uniformly on the bottom of a cylindrical container with an inner diameter of 20.5 millimeters and a height of 50 millimeters on the bottom of which a nylon sheet with a pore size of 75 micrometers is pasted beforehand, A Petri dish with a diameter of 50 millimeters was prepared and filled with 0.90 gram of saline, and the cylindrical container containing the absorbent resin particles was let stand in the Petri dish to cause them to absorb the saline and swell for 1 hour.
Measurement: A 1 kN load cell was prepared, and a cylindrical shaft with a diameter of 19.7 millimeters was attached thereto. The measurement range was set at 0.2 kN, the position of the load cell was adjusted at the height where no load is applied thereon, and the load cell was set to descend at a fixed rate of 0.6 mm/min. The pressure loaded on the load cell was measured over time. The surface strength herein is indicated by the load (N) at the point when the particles reach their actual volume. The actual volume of the absorbent resin particles was calculated based on the relative density of saline of 1.010 $g/cm^3$ and the relative density of the absorbent resin particles.

(8) Measurement of absorption capacity of sheet and absorption capacity of absorbent resins in sheet

The absorption capacity of the sheet was measured by the T-Bag method in the same manner as that for the resins. The absorption capacity of the absorbent resins in the sheet was calculated according to the following formula, where the absorption capacity of a sheet that is equivalent to the absorbent sheet except containing no absorbent resins was measured and determined as a blank value.
Absorption capacity of absorbent resins in sheet (g/g)= {(weight of sheet after absorption)-(weight of blank sheet after absorption)-(weight of absorbent resins in sheet)}/(weight of absorbent resins in sheet)

(9) Measurement of absorption speed, diffusion length, and rewet amount

A sheet was cut into a rectangle of 160 millimeters long and 70 millimeters wide and let stand on a table, and 15 grams of saline was dripped thereon over 1.5 seconds. The surface of the absorbent body was carefully observed, and the time period from the start of dripping to the time when the saline becomes invisible on the surface of the sheet after absorption was determined as the absorbent speed (sec.). After 3 minutes from the start of the dripping, the length of diffusion of the liquid was measured and determined as the diffusion length (mm). After 5 minutes from the start of the dripping, a square of filter paper of 100 millimeters long and 100 millimeters wide was placed on the position of the dripping, a weight of 3.5 kilograms having the same bottom area as the filter paper was placed thereon, the weight was removed in 3 minutes after placing the filter paper, and then the filter paper was weighed. The increase of weight of the filter paper was determined as the rewet amount (g).

(10) Detachment test

After the sheet was made to absorb 200 g/m$^2$ of liquid completely, an end of the sheet was held by hand, the sheet was reciprocated ten times in a state of being held vertically at such a speed that it took one second for one stroke movement between a distance of 30 centimeters, and then whether deformation, movement, or detachment of resins substantially occurred was visually checked. After the sheet was made to absorb 500 g/m$^2$ of liquid completely, portion of absorption of the sheet was held by hand, the sheet was vertically reciprocated ten times at such a speed that it took one second for one stroke movement between a distance of 20 centimeters, and then whether the shape as a sheet changed was visually checked. Furthermore, the sheet was immersed completely in saline and, when the sheet saturated and fully swelled, the same tests as above were conducted, and whether deformation, movement, or detachment of resins or deformation of the sheet substantially occurred was visually checked.

(11) Absorption thickness

A sheet having a sufficient area was fixed on a horizontal table, and 100 milliliters of colored saline was flown thereon at a speed of 10 ml/s. After a lapse of one hour, the area of colored portion and the thickness of portion into which the saline was injected were measured. The thickness of absorption that was performed in a completely even manner was calculated by dividing the amount of the injected saline by the absorption area (area of the colored portion), and was compared with the thickness of the portion into which the saline was injected.

Evaluation of bending resistance

The bending resistance was evaluated by the bending resistance D method (heart loop method) specified in JIS L 1096.

(13) Evaluation as absorbent sheet for grill drip pan

A sheet was cut into a size same as that of a gas grill (Rinnai RGB-30B3), and was made to absorb 400 milliliters of water. An odor in the gas range when fish was grilled was measured by an odor sensor (Portable Odor level Indicator XP-329III manufactured by New Cosmos Electric Co., Ltd.). A saurel was used as a fish sample and both sides thereof were grilled for 5 minutes each. The odor was measured at the start and 3 minutes after turning off the grill. Once the sheet was removed, and the grill was washed and the sheet was reinstalled, and then the same tests were conducted. By ten monitors, the odor, appearance after use, and easiness of cleanup were evaluated. A sheet was cut in the size same as that of the gas grill (Rinnai RGB-30B3), and was made to absorb 400 milliliters of water. A fillet of yellowtail was placed in the grill and both sides thereof were grilled each for 10 minutes over a medium flame. Without water being added, the yellowtail was replaced, and grilled each for 10 minutes over a medium flame. These procedures were repeated and the number of times when the fillet got entirely burned into soot was counted.

(14) Evaluation as absorbent sheet for coffin lining pad

Ten milliliters of blue reagent was dripped in whole for 10 to 12 seconds, let stand for about 15 seconds, and then the state was observed.

(15) Evaluation as absorbent sheet for dew condensation preventing material

A sheet was pasted on a window, and was made to absorb 5 milliliters of water flown from above with a pipette. The absorption area at this time was measured. After standing for 1 hour, the sheet was removed and weighed, and the amount of water retained was measured.

(16) Evaluation as absorbent sheet for sweat-absorbent pad

A sheet was cut into a size fitting to an armpit, and placed thereon with a tape. Fit feeling at this time, and fit feeling and slipperiness after exercise and getting sweat were evaluated.

(17) Evaluation as absorbent sheet for antifouling pad for clothes

A sheet was pasted on a collar of a shirt with a tape. Feeling and grime after one-day use were evaluated.

(18) Evaluation as absorbent sheet for bedding cover

A sheet was pasted on a bedding cover with a tape. Feeling and soil on the bedding cover after one-day use were evaluated.

(19) Evaluation as absorbent sheet for thawing pad

A sheet was rolled around frozen fish and left in a state being rolled for half a day, and a state of water or dripping when the fish was thawed was evaluated.

(20) Evaluation as absorbent sheet for pad for removing water on cooking ingredient

Fish that was washed with water was placed on a sheet, and a state of water being removed was evaluated.

(21) Evaluation as absorbent sheet for mask

Five milliliters of water was sprayed on a sheet, and feeling and a state of the sheet becoming dry after one-hour use were evaluated.

(22) Evaluation as absorbent sheet for wet proof pad for electronic equipment

Ten milliliters of water was dropped on a sheet having a laminate on one side thereof in 2 seconds, and a state of spread and downward permeation were evaluated.

(23) Measurement of ammonium ion concentration

A sheet was weighed, and was immersed into an excessive amount of saline that had been weighed in advance. After standing for 24 hours, the amount of ammonium ions in the solution was measured by ion chromatography. From measurement values of the weight of the sheet and the amount of ammonium ions, the ammonium ion concentration (mass %) was calculated.

[Production 1] (Preparation of ammonium acrylate by neutralizing acrylic acid)

Acrylic acid of special grade reagent manufactured by Wako Pure Chemical Industries, Ltd. was used. The acrylic acid had been purified before use and polymerization inhibitors had been removed, and then it was used. Next, 100 kilograms of acrylic acid was dissolved into 91.02 kilograms of water. This aqueous solution was cooled in an ice bath and maintained at a liquid temperature of 30°C or lower, and therein 117.94 kilograms of 25 mass% ammonia aqueous solution was gradually added while being stirred, whereby 40 mass% ammonium acrylate aqueous solution was obtained.

[Production 2] (Preparation of sodium acrylate by neutralizing acrylic acid)

Acrylic acid of special grade reagent manufactured by Wako Pure Chemical Industries, Ltd. was used. The acrylic acid had been purified before use and polymerization inhibitors had been removed, and then it was used. Next, 100 kilograms of acrylic acid was dissolved into 43.2 kilograms of water. This aqueous solution was cooled in an ice bath and maintained at a liquid temperature of 30°C or lower, and therein 166.7 kilograms of 25 mass% sodium hydrate aqueous solution was gradually added while being stirred, whereby 40 mass% 75 mol%-neutralized sodium acrylate aqueous solution was obtained.

[Production 3]

Into 300 kilograms of 40 mass% ammonium acrylate aqueous solution produced in Production 1, 0.0623 kilogram of N,N'-methylenebisacrylamide was added. Next, 1.43 kilograms of 42 mass% glycerine aqueous solution was added. Subsequently, as photopolymerization initiators, 0.0067 kilogram of 2,2-dimethoxy-1,2-diphenylethane-1-on and 0.0033 kilogram of ammonium persulfate were added, and this monomer aqueous solution was cooled down to 10°C, and deaerated by bubbling with nitrogen gas to substitute nitrogen for the reaction system. The concentration of dissolved oxygen decreased to 1 ppm or less. This aqueous solution was placed in such a manner that the aqueous solution thickness became 20 millimeters, and irradiated with ultraviolet rays (light quantity 684 mJ/cm$^2$) for 2 minutes by using high-pressure mercury lamps (MUMK-20-25XE, 20W, emission light wavelength 253 nm, manufactured by SEN Engineering Co., Ltd.; three units of this were used). The internal temperature rose from the initial temperature of 13°C to the maximum reaching temperature of about 90°C. Subsequently, the gel was taken out and coarsely ground, and then preliminary dried for 1 hour with a hot-air dryer at 130°C. The resulting absorbent resins were referred to as absorbent resins (1).

[Production 4]

The absorbent resins (1) were further dried for 1 hour with the hot-air dryer at 130°C, and then were pulverized with a homogenizer. The resulting absorbent resins were referred to as absorbent resins (2).

[Production 5]

The absorbent resins (2) were heated for 25 minutes with the hot-air dryer at 175°C. The resulting absorbent resins were referred to as absorbent resins (3).

[Production 6]

Into 300 kilograms of 40 mass% ammonium acrylate aqueous solution produced in Production 1, 0.024 kilogram of trimethylol propane triacrylate was added. Subsequently, as photopolymerization initiators, 0.0067 kilogram of 2,2-dimethoxy-1,2-diphenylethane-1-on and 0.0033 kilogram of ammonium persulfate were added, and this monomer aqueous solution was cooled down to 10°C, and deaerated by bubbling with nitrogen gas to substitute nitrogen for the reaction system. The concentration of dissolved oxygen decreased to 1 ppm or less. This aqueous solution was placed in such a manner that the aqueous solution thickness became 20 millimeters, and irradiated with ultraviolet rays (light quantity 684 mJ/cm$^2$) for 2 minutes by using the high-pressure mercury lamps (MUMK-20-25XE, 20W, emission light wavelength 253 nm, manufactured by SEN Engineering Co., Ltd.; three units of this were used). The internal temperature rose from the initial temperature of 13°C to the maximum reaching temperature of about 90°C. Subsequently, the gel was taken out and coarsely ground, and then preliminary dried for 2 hours with the hot-air dryer at 130°C. After the drying was completed, pulverization was performed with the homogenizer. The resulting absorbent resins were referred to as absorbent resins (4).

[Production 7]

Into 50 kilograms of absorbent resins (4), 0.125 kilogram of ethylene glycol diglycidyl ether as a crosslinking agent, 3 kilograms of water, and 0.3 kilogram of silica were added and mixed. This mixture was vacuum dried at 25°C. The resulting resins were referred to as absorbent resins (5).

[Production 8]

The absorbent resins (5) were heated for 10 minutes with the hot-air dryer at 180°C. The resulting absorbent resins were referred to as absorbent resins (6).

[Production 9]

Into 300 kilograms of 40 mass% 75 mol%-neutralized sodium acrylate aqueous solution produced in Production 2, 0.036 kilogram of trimethylol propane triacrylate was added. Subsequently, as photopolymerization initiators, 0.0067 kilogram of 2,2-dimethoxy-1,2-diphenylethane-1-on and 0.0033 kilogram of sodium persulfate were added, and this monomer aqueous solution was cooled down to 10°C and deaerated by bubbling with nitrogen gas to substitute nitrogen for the reaction system. The concentration of dissolved oxygen decreased to 1 ppm or less. This aqueous solution was placed in such a manner that the aqueous solution thickness became 20 millimeters, and irradiated with ultraviolet rays (light quantity 684 mJ/cm$^2$) for 2 minutes by using the high-pressure mercury lamps (MUMK-20-25XE, 20W, emission light wavelength 253 nm, manufactured by SEN Engineering Co., Ltd.; three units of this were used). The internal temperature rose from the initial temperature of 13°C to the maximum reaching temperature of about 90°C. Subsequently, the gel was taken out and coarsely ground, and then dried for 2 hours with the hot-air dryer at 130°C. After the drying was completed, pulverization was performed with the homogenizer. The resulting absorbent resins were referred to as absorbent resins (7).

[Production 10]

Into a beaker, 50 kilograms of the absorbent resins (7) were placed, and 0.125 kilogram of ethylene glycol diglycidyl ether as a crosslinking agent, 3 kilograms of water, and 0.3 kilogram of silica were added and vacuum dried at 25°C. The resulting resins were referred to as absorbent resins (8).

[Production 11]

The absorbent resins (8) were heated for 10 minutes with the hot-air dryer at 180°C. The resulting absorbent resins were referred to as absorbent resins (9).

From the respective absorbent resins, ones each having a particle size of 106 to 300 micrometers were acquired by sieve classifying. Their performances are illustrated in the following table 1. The performances of the absorbent resins change by heating. With respect to the absorbent resins (3), (6), and (9), their heating conditions are set to be conditions that are preferred when they are used for a general absorbent body. In the present invention, the absorbent resins are subjected to heating during the sheet production, but when they are dehydrated and dried in a state of being in contact with the hydrophilic fibers, the performances change more significantly because the hydrophilic fibers and the absorbent resins are bonded. However, this state cannot be considered to be caused by the absorbent resins alone. When producing the sheet, because the resins are not in contact with each other and are uniformly present and the hydrophilic fibers effectively function, it is possible to achieve higher performances by heating at low temperature for a short period of time than those achieved by resins alone. In addition, at this time, the neutralization rate at the outer surfaces efficiently decreases. More specifically, when using the absorbent resins (2), the neutralization rate equivalent to that of the absorbent resins (3) can be achieved and, when using the absorbent resins (4) or (5), the neutralization rate equivalent to that of the absorbent resins (6) can be achieved. However, because the volatilization rate of ammonia becomes slow when the neutralization rate becomes lower than a certain level, the salt concentration hardly decreases when using the absorbent resins (3) or (6).

[Table 1]

| Absorbent resins | | (2) | (3) | (4) | (6) | (7) | (9) |
|---|---|---|---|---|---|---|---|
| Water-absorption capacity pressure (g/g) | | 61 | 56 | 63 | 57 | 53 | 49 |
| Water-retaining capacity (g/g) | | 53 | 44 | 55 | 45 | 33 | 29 |
| Absorption speed (sec.) | | 12 | 7 | 9 | 5 | 17 | 12 |
| Water-absorption capacity under pressure (g/g) | 0 (g/cm$^2$) | 61 | 53 | 62 | 50 | 51 | 48 |
| | 57 (g/cm$^2$) | 20 | 27 | 12 | 15 | 11 | 12 |
| Residual monomer concentration (ppm) | | <10 | <10 | <10 | <10 | 500 | 420 |
| Carboxyl group neutralization rate (%) | Central part | 95 | 78 | 94 | 70 | 75 | 75 |
| | Outer surface | 89 | 35 | 91 | 36 | 75 | 75 |
| Surface strength (N) | | 0.6 | 4.1 | 0.5 | 3.9 | 0.7 | 5.6 |

[Example 1]

An undersheet (16 g/m$^2$) constructed of hydrophobic fibers (PET/PE core-in-sheath fibers manufactured by Toyobo Co., Ltd., 1.7 deniers, fiber length 40 millimeters) was conveyed at 3.5 m/min. Hydrophilic fibers (a treatment-processed article of roll pulp manufactured by Rayonier, Inc. was hammer crushed) and hydrophobic fibers (PET/PE core-in-sheath fibers manufactured by Toyobo Co., Ltd., 11 deniers, fiber length 50 millimeters) were mixed at a weight ratio of 6:4 and this mixture was placed uniformly on the undersheet such that the area density became 50 g/m$^2$, and water was sprayed thereon at an area density of 150 g/m$^2$. On this mixture, particles having a particle size of 106 to 300 micrometers (average particle size of about 200 micrometers) out of the absorbent resins (5) were uniformly dispersed such that the area density became 100 g/m$^2$, and further water was sprayed such that the area density became 150 g/m$^2$. These resins and fibers were passed between rolls having projections and depressions on their surfaces while being vibrated and were mixed with each other. Hydrophilic fibers (a treatment-processed article of roll pulp manufactured by Rayonier, Inc. was hammer crushed) and hydrophobic fibers (PET/PE core-in-sheath fibers manufactured by Toyobo Co., Ltd., 1.7 deniers, fiber length 40 millimeters) were mixed at a weight ratio of 6:4 and this mixture was uniformly arranged such that the area density became 30 g/m$^2$. A surfactant (2.5% SANMORIN OT-70) was sprayed with a spray such that the area density became 50 g/m$^2$. At this time, the water content was 350 mass% with respect to the absorbent resins. Hot-air drying (drying temperature 145°C, drying time 1 minute, air speed 5 m/s) was performed in a drying furnace, pressing was performed with a flat press (temperature 110°C, air pressure 0.1 N/cm$^2$), winding up was performed, and thus a sheet was acquired. This sheet was referred to as an absorbent sheet (1).

[Example 2]

Hydrophilic fibers (a treatment-processed article of roll pulp manufactured by Rayonier, Inc. was hammer crushed) and particles having a particle size of 106 to 300 micrometers (average particle size of about 200 micrometers) out of the absorbent resins (5) were mixed at a weight ratio of 1:4. At this time, the water content was 4 mass% with respect to the absorbent resins. Water of the same weight as the absorbent resins was sprayed thereon with the spray, hot-air drying (drying temperature 140°C, drying time 2 minutes, air speed 5 m/s) was performed in the drying furnace, and composite compositions thus produced were stored in a tank. The water content after dehydration and drying was 3 mass% with respect to the absorbent resins. An undersheet (16 g/m$^2$) constructed of hydrophobic fibers (PET/PE core-in-sheath fibers manufactured by Toyobo Co., Ltd., 1.7 deniers, fiber length 40 millimeters) was conveyed at 30 m/min. On this undersheet, a mixture of the composite compositions and hydrophobic fibers (PET/PE core-in-sheath fibers manufactured by Toyobo Co., Ltd., 11 deniers, fiber length 50 millimeters) in a weight ratio of 5:1 was arranged such that the area density became 150 g/m$^2$. Hydrophilic fibers (a treatment-processed article of roll pulp manufactured by Rayonier, Inc. was hammer crushed) and hydrophobic fibers (PET/PE core-in-sheath fibers manufactured by Toyobo Co., Ltd., 1.7 deniers, fiber length 40 millimeters) were mixed at a weight ratio of 6:4 and were placed uniformly on the mixture such that the area density became 30 g/m$^2$, and a surfactant (5% SANMORIN OT-70) was sprayed with the spray such that the area density became 20 g/m$^2$. At this time, the water content was 20 mass% with respect to the absorbent resins. Hot-air drying (drying temperature 145°C, drying time 6 seconds, air speed 5 m/s) was performed in the drying furnace, pressing was performed with the flat press (temperature 110°C, air pressure 0.1 N/cm$^2$), winding up was performed, and thus a sheet was acquired. This sheet was referred to as an absorbent sheet (2).

[Example 3]

Other than using the absorbent resins (2), the same method as that of Example 2 was used to acquire an absorbent sheet. This sheet was referred to as an absorbent sheet (3).

[Example 4]

Other than using the absorbent resins (8), the same method as that of Example 2 was used to acquire an absorbent sheet. Because the surface salt concentration was low, bonding force between the hydrophilic fibers and the absorbent resins was small. This sheet was referred to as an absorbent sheet (4).

[Example 5]

An undersheet constructed of hydrophobic fibers (PET/PE core-in-sheath fibers manufactured by Toyobo Co., Ltd., 1.7 deniers, fiber length 40 millimeters) was conveyed at 2 m/min. On this undersheet, 5 g/m$^2$ of hydrophobic fibers (PET/PE core-in-sheath fibers manufactured by Toyobo Co., Ltd., 11 deniers, fiber length 50 millimeters), 5 g/m$^2$ of hydrophilic fibers (a treatment-processed article of roll pulp manufactured by Rayonier, Inc. was hammer crushed), 5 g/m$^2$ of hydrophobic fibers, 5 g/m$^2$ of hydrophilic fibers, 5 g/m$^2$ of hydrophobic fibers, 5 g/m$^2$ of hydrophilic fibers, 5 g/m$^2$ of hydrophobic fibers, and 15 g/m$^2$ of hydrophobic fibers were arranged in this order. Water was sprayed at an area density of 150 g/m$^2$ with the spray. Particles having a particle size of 100 to 300 micrometers out of the absorbent resins (5) were uniformly dispersed thereon such that the area density became 100 g/m$^2$, and further water was sprayed with the spray such that the area density became 150 g/m$^2$. On these resins, 6 g/m$^2$ of hydrophilic fibers (a treatment-processed article of roll pulp manufactured by Rayonier, Inc. was hammer crushed), 4 g/m$^2$ of hydrophobic fibers (PET/PE core-in-sheath fibers manufactured by Toyobo Co., Ltd., 1.7 deniers, fiber length 40 millimeters), 6 g/m$^2$ of hydrophilic fibers, 4 g/m$^2$ of hydrophobic fibers, 6 g/m$^2$ of hydrophilic fibers, and 4 g/m$^2$ of hydrophobic fibers were arranged. A surfactant (2.5% SANMORIN OT-70) was sprayed with the spray such that the area density became 50 g/m$^2$. At this time, the water content was 350 mass% with respect to the absorbent resins. Hot-air drying (drying temperature 145°C, drying time 90 seconds, air speed 5 m/s) was performed in the drying furnace, pressing was performed with the flat press (temperature 110°C, air pressure 0.1 N/cm$^2$), winding up was performed, and thus a sheet was acquired. This sheet was referred to as an absorbent sheet (5). Because there is no mixing step included herein, the effect of such combination was rather small.

[Example 6]

Hydrophilic fibers to be used were completely dried at 120°C in a vacuum dryer. Absorbent resins to be used were completely dried at 50°C in the vacuum dryer. Other than these, the same method as that of Example 2 was used to acquire an absorbent sheet. The water content when mixing the absorbent resins and the hydrophilic fibers was 0.09 mass% with respect to the absorbent resins Because interaction between the absorbent resins and the hydrophilic fibers became small and static electricity was also generated, it took time to mix them. This sheet was referred to as an absorbent sheet (6).

[Example 7]

Hydrophilic fibers to be used were caused to absorb water four times as much as the weight of hydrophilic fibers, and then were mixed with the absorbent resins (5). The water content was 102 mass% with respect to the absorbent resins. Other than these, the same method as that of Example 2 was used to acquire an absorbent sheet. The miscibility between the hydrophilic fibers and the absorbent resins decreased. This sheet was referred to as an absorbent sheet (7).

[Example 8]

After hydrophilic fibers and absorbent resins were mixed, water was dispersed with the spray such that the water content in the system became 19 mass% with respect to the absorbent resins, and an absorbent sheet was acquired by the same method as that of Example 2 except this dispersion. Because the water content was small before dehydration and drying, bonding force between the hydrophilic fibers and the absorbent resins decreased. This sheet was referred to as an absorbent sheet (8).

[Example 9]

After hydrophilic fibers and absorbent resins were mixed, water was dispersed with the spray such that the water content in the system became 1100 mass% with respect to the absorbent resins and the drying time was set to 10 minutes. Other than these, the same method as that of Example 2 was used to acquire an absorbent sheet. This sheet was referred to as an absorbent sheet (8).

[Example 10]

Composite compositions were produced by the same method as that of Example 2. An undersheet constructed of hydrophobic fibers (PET/PE core-in-sheath fibers manufactured by Toyobo Co., Ltd., 1.7 deniers, fiber length 40 millimeters) was conveyed at a speed of 30 m/min. The composite compositions and hydrophobic fibers (PET/PE core-in-sheath fibers manufactured by Toyobo Co., Ltd., 11 deniers, fiber length 50 millimeters) were mixed at a weight ratio of 5:1 and were arranged on the undersheet such that the area density became 150 g/m$^2$. A mixture of hydrophilic fibers (a treatment-processed article of roll pulp manufactured by Rayonier, Inc. was hammer crushed) that had been completely dried at 120°C in advance with the vacuum dryer and hydrophobic fibers (PET/PE core-in-sheath fibers manufactured by Toyobo Co., Ltd., 1.7 deniers, fiber length 40 millimeters) in a weight ratio of 6:4 was placed thereon uniformly such that the area density became 30 g/m$^2$. At this time, the water content was 0.9 mass% with respect to the absorbent resins. Hot-air drying (drying temperature 145°C, drying time 6 seconds (same as Example 2), air speed 5 m/s) was performed in the drying furnace, pressing was performed with the flat press (temperature 110°C, air pressure 0.1 N/cm$^2$), winding up was performed, and thus a sheet was acquired. This sheet was referred to as an absorbent sheet (10).

[Example 11]

A surfactant (0.5% aqueous solution of SANMORIN OT-70) was dispersed such that the area density became 250 g/m$^2$ (the water content at this time was 250 mass% with respect to the absorbent resins), the drying time was set to 1 minute, and the line speed was set to 3 m/min. Other than these, the same method as that of Example 2 was used to acquire an absorbent sheet. This sheet was referred to as an absorbent sheet (11).

[Example 12]

Fifty kilograms of the absorbent resins (5), 12.5 kilograms of hydrophilic fibers (a treatment-processed article of roll pulp manufactured by Rayonier, Inc. was hammer crushed), 0.125 kilogram of ethylene glycol diglycidyl ether, 3 kilograms of water, and 0.3 kilogram of silica were added and mixed. Furthermore, 30 kilograms of water was sprayed, and this mixture was hot-air dried in the drying furnace (drying temperature 140°C, drying time 2 minutes, air speed 5 m/s). Composite compositions thus produced were stored in a tank. Other than using these composite compositions, the same method as that of Example 2 was used to acquire an absorbent sheet. This sheet was referred to as an absorbent sheet (12).

[Example 13]

Fifty four kilograms of the absorbent resins (1) (containing 4 kilograms of water) and 12.5 kilograms of hydrophilic fibers (a treatment-processed article of roll pulp manufactured by Rayonier, Inc.) were mixed, and were pulverized while being mixed. Thirty kilograms of water was sprayed, and these particles were hot-air dried in the drying furnace (drying temperature 140°C, drying time 2 minutes, air speed 5 m/s). Composite compositions thus produced were stored in a tank. Other than using these composite compositions, the same method as that of Example 2 was used to acquire an absorbent sheet. This sheet was referred to as an absorbent sheet (13).

[Example 14]

Other than using no undersheet, the same method as that of Example 2 was used to acquire an absorbent sheet (a wire net was conveyed in place of an undersheet to form the absorbent sheet). This sheet was referred to as an absorbent sheet (14).

[Example 15]

Other than using no undersheet or using 20 g/m$^2$ of hydrophobic fibers (PET/PE core-in-sheath fibers manufactured by Toyobo Co., Ltd., 1.7 deniers, fiber length 40 millimeters), the same method as that of Example 2 was used to acquire an absorbent sheet (a wire net was conveyed in place of an undersheet to form the absorbent sheet). This sheet was referred to as an absorbent sheet (15).

[Example 16]

Composite compositions were produced by the same method as that of Example 2. An undersheet constructed of hydrophobic fibers (PET/PE core-in-sheath fibers manufactured by Toyobo Co., Ltd., 1.7 deniers, fiber length 40 millimeters) was conveyed at a speed of 30 m/min. On the undersheet, a mixture of the composite compositions and hydrophobic fibers (PET/PE core-in-sheath fibers manufactured by Toyobo Co., Ltd., 11 deniers, fiber length 50 millimeters) in a weight ratio of 75:1 were arranged such that the area density became 150 g/m$^2$. An absorbent sheet was acquired by the same method as that of Example 2. This sheet was referred to as an absorbent sheet (16).

[Example 17]

Composite compositions were produced by the same method as that of Example 2. An undersheet constructed of hydrophobic fibers (PET/PE core-in-sheath fibers manufactured by Toyobo Co., Ltd., 1.7 deniers, fiber length 40 millimeters) was conveyed at a speed of 30 m/min. On the undersheet, a mixture of the composite compositions and hydrophobic fibers (PET/PE core-in-sheath fibers manufactured by Toyobo Co., Ltd., 11 deniers, fiber length 50 millimeters) in a weight ratio of 5:4 was arranged such that the area density became 200 g/m$^2$. An absorbent sheet was acquired by the same method as that of Example 2. This sheet was referred to as an absorbent sheet (17).

[Example 18]

Hydrophilic fibers (a treatment-processed article of roll pulp manufactured by Rayonier, Inc. was hammer crushed) and particles having a particle size of 106 to 300 micrometers (average particle size of about 200 micrometers) out of the absorbent resins (5) were mixed at a weight ratio of 1:11. At this time, the water content was 4 mass% with respect to the absorbent resins. Water of the same weight as the absorbent resins was sprayed thereon with the spray, hot-air drying (drying temperature 140°C, drying time 2 minutes, air speed 5 m/s) was performed in the drying furnace. Composite compositions thus produced were stored in a tank. The water content after dehydration and drying was 4 mass% with respect to the absorbent resins. An absorbent sheet was acquired by the same method as that of Example 2. This sheet was referred to as an absorbent sheet (18).

[Example 19]

Hydrophilic fibers (a treatment-processed article of roll pulp manufactured by Rayonier, Inc. was hammer crushed) and particles having a particle size of 106 to 300 micrometers (average particle size of about 200 micrometers) out of the absorbent resins (5) were mixed at a weight ratio of 6:1. At this time, the water content was 4 mass% with respect to the absorbent resins. Water of the same weight as the absorbent resins was sprayed thereon with the spray, hot-air drying (drying temperature 140°C, drying time 2 minutes, air speed 5 m/s) was performed in the drying furnace. Composite compositions thus produced were stored in a tank. The water content after dehydration and drying was 2 mass% with respect to the absorbent resins. An absorbent sheet was acquired by the same method as that of Example 2. This sheet was referred to as an absorbent sheet (19).

[Example 20]

Other than using absorbent resins (having an average particle size of 170 micrometers) 51 % of which were particles having a particle size of 75 to 90 micrometers out of the absorbent resins (5) and 49% of which were particles having a particle size of 100 to 425 micrometers out of the absorbent resins (5), the same method as that of Example 2 was used to acquire an absorbent sheet. Detachment of fine powder components partially occurred. This sheet was referred to as an absorbent sheet (20).

[Example 21]

Other than using absorbent resins (having an average particle size of 310 micrometers) 49% of which were particles having a particle size of 75 to 300 micrometers out of the absorbent resins (5) and 51% of which were particles having a particle size of 425 to 500 micrometers out of the absorbent resins (5), the same method as that of Example 2 was used to acquire an absorbent sheet. This sheet was referred to as an absorbent sheet (21).

[Example 22]

An absorbent sheet was acquired by the same method as that of Example 2 except using the absorbent resins (9) as absorbent resins to be used. Because the surface strength was high, bonding force between the absorbent resins and the hydrophilic fibers was small. In addition, absorption capability of the absorbent resins themselves was low. This sheet was referred to as an absorbent sheet (22).

[Example 23]

An absorbent sheet was acquired by the same method as that of Example 2 except using the absorbent resins (4) as absorbent resins to be used. Because no condensation crosslinking agent was present, bonding force between the absorbent resins and the hydrophilic fibers was rather small. This sheet was referred to as an absorbent sheet (23).

[Example 24]

Subsequently to the same pressing machine of the device for producing an absorbent sheet as that of Example 2, a device for feeding a top sheet and a back sheet was installed, a hot-melt adhesive was dispersed, and thus an absorbent product having a top sheet on the upper side and a back sheet on the lower side of the absorbent sheet (2) was produced.

[Comparative Example 1]

An absorbent sheet was produced by the same method as that of Example 1 except using no hydrophobic fibers. Because the sheet had many detaching components due to weak bonding force, evaluation as a sheet was difficult.

[Comparative Example 2]

An absorbent sheet was produced by the same method as that of Example 1 except using no hydrophilic fibers nor water. Because of no hydrophilic fibers contained, absorption capability was low. This sheet was referred to as a comparative absorbent sheet (2).

[Comparative Example 3]

Hydrophilic fibers to be used (a treatment-processed article of roll pulp manufactured by Rayonier, Inc. was hammer crushed) were completely dried at 120°C in the vacuum dryer. Absorbent resins to be used were completely dried at 50°C in the vacuum dryer. An undersheet (16 g/m$^2$) constructed of hydrophobic fibers (PET/PE core-in-sheath fibers manufactured by Toyobo Co., Ltd., 1.7 deniers, fiber length 40 millimeters) was conveyed at 30 m/min. The hydrophilic fibers and hydrophobic fibers (PET/PE core-in-sheath fibers manufactured by Toyobo Co., Ltd., 11 deniers, fiber length 50 millimeters) were mixed at a weight ratio of 6:4, and this mixture was placed uniformly on the undersheet such that the area density became 50 g/m$^2$. Particles having a particle size of 106 to 300 micrometers (average particle size of about 200 micrometers) out of the absorbent resins (5) were uniformly dispersed thereon such that the area density became 100 g/m$^2$. These resins and fibers were passed between the rolls having projections and depressions on their surfaces and were mixed with each other. Hydrophilic fibers (a treatment-processed article of roll pulp manufactured by Rayonier, Inc. was hammer crushed) and hydrophobic fibers (PET/PE core-in-sheath fibers manufactured by Toyobo Co., Ltd., 1.7 deniers, fiber length 40 millimeters) were mixed at a weight ratio of 6:4 and this mixture was uniformly arranged such that the area density became 30 g/m$^2$. Hot-air drying (drying temperature 145°C, drying time 6 seconds, air speed 5 m/s) was performed in the drying furnace, pressing was performed with the flat press (temperature 110°C, air pressure 0.1 N/cm$^2$), winding up was performed, and thus a sheet was acquired. Because water was not present in the system, the dehydration and drying step was not included. This sheet was referred to as a comparative absorbent sheet (3).

[Comparative Example 4]

Other than setting the line speed to 0.1 m/min, setting the drying furnace to perform air drying (drying temperature 25°C, drying time 30 minutes, air speed 5 m/s), and performing pressing with the flat press (temperature 30°C, air pressure 0.1 N/cm$^2$), the same method as that of Example 2 was used to acquire an absorbent sheet. Because no heating was performed, fusion of hydrophobic fibers did not occur and bonding force was weak, and thus evaluation as a sheet was difficult.

[Comparative Example 5]

An incontinence pad "Lifree Sawayaka Pad for 15 cc" manufactured by Unicharm Corporation was prepared, and only absorbent body part was recollected by peeling the hot-melt adhesive with a dryer. This sheet was referred to as a comparative absorbent body (5).
For each of the absorbent sheets of Examples 1 to 23, the comparative absorbent sheets of Comparative Examples 2 and 3, and the comparative absorbent body of Comparative Example 5, measured results on absorption capacity of the sheet, absorption capacity of the absorbent resins in the sheet, absorption speed of the sheet, diffusion length (in the lengthwise direction), diffusion length (in the crosswise direction), and rewet amount are given in Table 2.

[Table 2]

| | Resin | Absorption capacity of sheet (g/g) | Absorption capacity of absorbent resins in sheet (g/g) | Absorption speed (sec.) | Diffusion length (lengthwise) (mm) | Diffusion length (crosswise) (mm) | Rewet amount (g) |
|---|---|---|---|---|---|---|---|
| Example 1 | 5 | 30 | 55 | 9 | 110 | 70 | 0.1 |
| Example 2 | 5 | 35 | 60 | 8.5 | 100 | 70 | 0.1 |
| Example 3 | 2 | 32 | 55 | 9.5 | 110 | 70 | 0.1 |
| Example 4 | 8 | 30 | 50 | 13.5 | 140 | 70 | 0.3 |
| Example 5 | 5 | 30 | 50 | 12 | 120 | 70 | 0.15 |
| Example 6 | 5 | 32 | 60 | 9.5 | 115 | 70 | 0.1 |
| Example 7 | 5 | 32 | 60 | 9.5 | 115 | 70 | 0.1 |

(continued)

| | Resin | Absorption capacity of sheet (g/g) | Absorption capacity of absorbent resins in sheet (g/g) | Absorption speed (sec.) | Diffusion length (lengthwise) (mm) | Diffusion length (crosswise) (mm) | Rewet amount (g) |
|---|---|---|---|---|---|---|---|
| Example 8 | 5 | 32 | 60 | 13 | 115 | 70 | 0.2 |
| Example 9 | 5 | 35 | 60 | 8.5 | 100 | 70 | 0.1 |
| Example 10 | 5 | 35 | 60 | 13 | 130 | 70 | 0.1 |
| Example 11 | 5 | 35 | 60 | 8.5 | 100 | 70 | 0.1 |
| Example 12 | 5 | 35 | 60 | 8.5 | 100 | 70 | 0.1 |
| Example 13 | 1 | 35 | 60 | 8.5 | 100 | 70 | 0.1 |
| Example 14 | 5 | 35 | 60 | 8.5 | 100 | 70 | 0.1 |
| Example 15 | 5 | 35 | 60 | 8.5 | 100 | 70 | 0.1 |
| Example 16 | 5 | 35 | 60 | 8.5 | 100 | 70 | 0.2 |
| Example 17 | 5 | 30 | 50 | 12 | 130 | 70 | 0.2 |
| Example 18 | 5 | 34 | 60 | 11 | 115 | 70 | 0.1 |
| Example 19 | 5 | 32 | 60 | 9.5 | 90 | 70 | 0.3 |
| Example 20 | 5 | 32 | 60 | 10 | 110 | 70 | 0.1 |
| Example 21 | 5 | 32 | 60 | 12 | 110 | 70 | 0.1 |
| Example 22 | 9 | 28 | 48 | 14 | 140 | 70 | 0.32 |
| Example 23 | 4 | 34 | 60 | 10 | 105 | 70 | 0.1 |
| Comparative Example 2 | 5 | 15 | 30 | A liquid was gathered on the surface and unlikely to be absorbed. | | | |
| Comparative Example 3 | 5 | 30 | 50 | 15 | 120 | 70 | 0.35 |
| Comparative Example 5 | - | - | - | 20 | 90 | 80 | 1.27 |

[Example 25]

An undersheet (16 g/m$^2$) constructed of hydrophobic fibers (PET/PE core-in-sheath fibers manufactured by Toyobo Co., Ltd., 1.7 deniers, fiber length 40 millimeters) was conveyed at 3.5 m/min. Hydrophilic fibers (a treatment-processed article of roll pulp manufactured by Rayonier, Inc. was hammer crushed) and hydrophobic fibers (PET/PE core-in-sheath fibers manufactured by Toyobo Co., Ltd., 11 deniers, fiber length 50 millimeters) were mixed at a weight ratio of 6:4 and this mixture was placed uniformly on the undersheet such that the area density became 50 g/m$^2$, and water was sprayed thereon at an area density of 150 g/m$^2$. On this mixture, particles having a particle size of 106 to 300 micrometers (average particle size of about 200 micrometers) out of the absorbent resins (5) were uniformly dispersed such that the area density became 100 g/m$^2$, and further water was sprayed such that the area density became 150 g/m$^2$. These resins and fibers were passed between the rolls having projections and depressions on their surfaces and were mixed. Hydrophilic fibers (a treatment-processed article of roll pulp manufactured by Rayonier, Inc. was hammer crushed) and hydrophobic fibers (PET/PE core-in-sheath fibers manufactured by Toyobo Co., Ltd., 1.7 deniers, fiber length 40 millimeters) were mixed at a weight ratio of 6:4 and this mixture was uniformly arranged such that the area density became 30 g/m$^2$. A surfactant (2.5% SANMORIN OT-70) was sprayed with the spray such that the area density became 50 g/m$^2$. Hot-air drying (drying temperature 145°C, drying time 50 seconds, air speed 5 m/s) was performed in the drying furnace, pressing was performed with the flat press (temperature 110°C, air pressure 0.1 N/cm$^2$), winding up was performed, and thus a sheet was acquired. This sheet was referred to as an absorbent sheet (25).

[Example 26]

An absorbent sheet was obtained by the same method as that of Example 25 except using no water or diluting the SANMORIN with isopropanol. This sheet was referred to as an absorbent sheet (26). Because no water was present, the fiber density of the absorbent layer was not so low.

[Example 27]

An undersheet (16 g/m$^2$) constructed of hydrophobic fibers (PET/PE core-in-sheath fibers manufactured by Toyobo Co., Ltd., 11 deniers, fiber length 50 millimeters) was conveyed at 3.5 m/min. Hydrophilic fibers (a treatment-processed article of roll pulp manufactured by Rayonier, Inc. was hammer crushed) and hydrophobic fibers (PET/PE core-in-sheath fibers manufactured by Toyobo Co., Ltd., 1.7 deniers, fiber length 40 millimeters) were mixed at a weight ratio of 6:4 and this mixture was placed uniformly on the undersheet such that the area density became 50 g/m$^2$. Particles having a particle size of 106 to 300 micrometers (average particle size of about 200 micrometers) out of the absorbent resins (5) were uniformly dispersed thereon such that the area density became 100 g/m$^2$. These resins and fibers were passed between the rolls having projections and depressions on their surfaces and were mixed. Hydrophilic fibers (a treatment-processed article of roll pulp manufactured by Rayonier, Inc. was hammer crushed) and hydrophobic fibers (PET/PE core-in-sheath fibers manufactured by Toyobo Co., Ltd., 1.7 deniers, fiber length 40 millimeters) were mixed at a weight ratio of 6:4 and this mixture was uniformly arranged such that the area density became 30 g/m$^2$. A surfactant (2.5% SANMORIN OT-70 diluted with isopropyl alcohol) was sprayed with the spray such that the area density became 50 g/m$^2$. Hot-air drying (drying temperature 145°C, drying time 50 seconds, air speed 5 m/s) was performed in the drying furnace, pressing was performed with the flat press (temperature 110°C, air pressure 0.1 N/cm$^2$), winding up was performed, and thus a sheet was acquired. This sheet was referred to as an absorbent sheet (27). The absorbent sheet (27) is different from the absorbent sheets (25) and (26) in that combination of hydrophobic fiber diameters is reversed. Because no water was present, the fiber density of the absorbent layer was not low.

[Example 28]

An absorbent sheet was acquired by the same method as that of Example 25 except using hydrophobic fibers that were not core-in-sheath fibers and were made of PET alone or setting the drying temperature at 180°C. This sheet was referred to as an absorbent sheet (28). Bonding force between the hydrophobic fibers and the hydrophilic fibers was small.

[Example 29]

An absorbent sheet was acquired by the same method as that of Example 25 except adjusting the ratio of the hydrophilic fibers to the hydrophobic fibers to be 9.1:1 (weight ratio in the whole absorbent sheet). This sheet was referred to as an absorbent sheet (29).

[Example 30]

An absorbent sheet was acquired by the same method as that of Example 25 except adjusting the ratio of the hydrophilic fibers to the hydrophobic fibers to be 2:8.1 (weight ratio in the whole absorbent sheet). This sheet was referred to as an absorbent sheet (30).

[Example 31]

An absorbent sheet was acquired by the same method as that of Example 25 except adjusting the quantity of hydrophilic fibers such that the ratio of the water-absorbent resins to the hydrophilic fibers became 10.1:1 (weight ratio in the whole absorbent sheet). This sheet was referred to as an absorbent sheet (31).

[Example 32]

An absorbent sheet was acquired by the same method as that of Example 25 except adjusting the quantity of hydrophilic fibers such that the ratio of the water-absorbent resins to the hydrophilic fibers became 1:5.1 (weight ratio in the whole absorbent sheet). This sheet was referred to as an absorbent sheet (32).

[Example 33]

An undersheet (16 g/m$^2$) constructed of hydrophobic fibers (PET/PE core-in-sheath fibers manufactured by Toyobo Co., Ltd., 1.7 deniers, fiber length 40 millimeters) was conveyed at 3.5 m/min. Hydrophilic fibers (a treatment-processed article of roll pulp manufactured by Rayonier, Inc. was hammer crushed) and hydrophobic fibers (PET/PE core-in-sheath fibers manufactured by Toyobo Co., Ltd., 11 deniers, fiber length 50 millimeters) were mixed at a weight ratio of 6:4 and this mixture was placed uniformly on the undersheet such that the area density became 50 g/m$^2$, and 28% ammonia water was sprayed thereon with the spray at an area density of 150 g/m$^2$. On this mixture, particles having a particle size of 106 to 300 micrometers (average particle size of about 200 micrometers) out of the absorbent resins (8) were uniformly dispersed such that the area density became 100 g/m$^2$, and further water was sprayed with the spray such that the area density became 150 g/m$^2$. These resins and fibers were passed between the rolls having projections and depressions and were mixed. Hydrophilic fibers (a treatment-processed article of roll pulp manufactured by Rayonier, Inc. was hammer crushed) and hydrophobic fibers (PET/PE core-in-sheath fibers manufactured by Toyobo Co., Ltd., 1.7 deniers, fiber length 40 millimeters) were mixed at a weight ratio of 6:4 and this mixture was uniformly arranged such that the area density became 30 g/m$^2$. A surfactant (2.5% SANMORIN OT-70) was sprayed with the spray such that the area density became 50 g/m$^2$. Hot-air drying (drying temperature 145°C, drying time 1 minute, air speed 5 m/s) was performed in the drying furnace, pressing was performed with the flat press (temperature 110°C, air pressure 0.1 N/cm$^2$), winding up was performed, and thus a sheet was acquired. This sheet was referred to as an absorbent sheet (33).

[Example 34]

An absorbent product was produced by adhering a top sheet and a back sheet on both sides of the absorbent sheet (25) with a hot-melt adhesive. This product was referred to as an absorbent product (34).

[Comparative Example 6]

An absorbent sheet was produced by the same method as that of Example 25 except using no hydrophobic fibers. Because of weak bonding force, the sheet had many detaching components. This sheet was referred to as a comparative absorbent sheet (6).

[Comparative Example 7]

An absorbent sheet was produced by the same method as that of Example 25 except using no hydrophilic fibers. Because no hydrophilic fibers were present, the absorption capability was low. This sheet was referred to as a comparative absorbent sheet (7).

[Comparative Example 8]

An absorbent sheet was produced by the same method as that of Example 25 except using the absorbent resins (8). This sheet was referred to as a comparative absorbent sheet (8).

[Comparative Example 9]

An incontinence pad "Lifree Sawayaka Pad for 15 cc" manufactured by Unicharm Corporation was prepared. This pad was referred to as a comparative absorbent product (9).

For each of the absorbent sheets (25) to (33), the absorbent product (34), the comparative absorbent sheets (6) to (8), and the comparative absorbent product (9), measured results on absorption capacity of the sheet, absorption capacity of the absorbent resins in the sheet, absorption speed of the sheet, diffusion length (in the lengthwise direction), diffusion length (in the crosswise direction), rewet amount, carboxy group neutralization rate at the resin outer surface; carboxy group neutralization rate at the resin central part, and ammonium ion concentration are given in Table 3. Note that the carboxy group neutralization rate at the resin outer surface can be considered to be the salt concentration at the resin outer surface and the carboxy group neutralization rate at the resin central part can be considered to be the salt concentration at the resin central part.

[Table 3]

| | Absorption capacity of sheet (g/g) | Absorption capacity of absorbent resins in sheet (g/g) | Absorption speed (sec.) | Diffusion length (lengthwise) (mm) | Diffusion length (crosswise) (mm) | Rewet amount (g) | Carboxyl group neutralization rate (%) | | Ammonium ion concentration (mass%) |
|---|---|---|---|---|---|---|---|---|---|
| | | | | | | | Resin outer surface | Resin central part | |
| Example 25 | 30 | 55 | 9 | 110 | 70 | 0.1 | 37 | 79 | 7.2 |
| Example 26 | 30 | 55 | 10 | 120 | 70 | 0.1 | 35 | 77 | 7.0 |
| Example 27 | 30 | 50 | 12 | 130 | 70 | 0.15 | 34 | 76 | 7.0 |
| Example 28 | 30 | 50 | 12 | 130 | 70 | 0.15 | 33 | 73 | 6.8 |
| Example 29 | 32 | 55 | 9 | 110 | 70 | 0.1 | 36 | 76 | 7.1 |
| Example 30 | 28 | 50 | 12.5 | 135 | 70 | 0.25 | 35 | 78 | 7.3 |
| Example 31 | 32 | 55 | 12 | 120 | 70 | 0.15 | 37 | 77 | 7.5 |
| Example 32 | 30 | 55 | 10 | 100 | 70 | 0.3 | 36 | 76 | 6.5 |
| Example 33 | 28 | 45 | 15 | 140 | 70 | 0.35 | 75 | 78 | 0.6 |
| Example 34 | 30 | 55 | 9 | 100 | 70 | 0.1 | - | - | - |
| Comparative Example 6 | There were many detaching components because no hydrophobic fibers were present | | | | | | | | |
| Comparative Example 7 | 15 | 30 | Liquid was gathered on surface and water was unlikely to be absorbed | | | | 37 | 76 | - |
| Comparative Example 8 | 28 | 45 | 17 | 150 | 70 | 0.4 | 75 | 75 | 0 |
| Comparative Example 9 | Not in sheet shape | | 20 | 90 | 80 | 1.27 | - | - | - |

[Example 36]

An undersheet (16 g/m$^2$) constructed of hydrophobic fibers (PET/PE core-in-sheath fibers manufactured by Toyobo Co., Ltd., 1.7 deniers, fiber length 40 millimeters) was conveyed at 30 m/min. Hydrophilic fibers (a treatment-processed article of roll pulp manufactured by Rayonier, Inc. was hammer crushed) and hydrophobic fibers (PET/PE core-in-sheath fibers manufactured by Toyobo Co., Ltd., 1.7 deniers, fiber length 40 millimeters) were mixed at a weight ratio of 5:5, and this mixture was placed uniformly on the undersheet such that the area density became 15 g/m$^2$. On this mixture, particles having a particle size of 150 to 710 micrometers (average particle size of about 400 micrometers) out of the absorbent resins (1) were uniformly dispersed such that the area density became 30 g/m$^2$. These resins and fibers were passed between rolls having needles on their surfaces, and were mixed with each other. Hydrophilic fibers (a treatment-processed article of roll pulp manufactured by Rayonier, Inc. was hammer crushed) and hydrophobic fibers (PET/PE core-in-sheath fibers manufactured by Toyobo Co., Ltd., 1.7 deniers, fiber length 40 millimeters) were mixed at a weight ratio of 5:5, and this mixture was uniformly arranged such that the area density became 30 g/m$^2$. Hot-air drying (drying temperature 145°C, drying time 6 seconds, air speed 5 m/s) was performed in the drying furnace, pressing was performed with the flat press (temperature 110°C, air pressure 0.1 N/cm$^2$), winding up was performed, and thus a sheet was acquired. As a result of evaluation of the sheet thus obtained on the above items, the absorption capacity of the sheet was 10 g/g, the absorption capacity of the resins in the sheet was 20 g/g, the water-absorption speed was 180 seconds, the diffusion lengths were 160 millimeters long and 70 millimeters wide, the rewet amount was 8 grams, the detachment test indicated no detachment, the water-absorption test indicated 3 millimeters in uniform thickness, the bending resistance was 70 millimeters, and the sheet thickness was 1 millimeter.

[Example 37]

A wire net was conveyed at 30 m/s without using an undersheet, and hydrophilic fibers (a treatment-processed article of roll pulp manufactured by Rayonier, Inc. was hammer crushed) and hydrophobic fibers (PET/PE core-in-sheath fibers manufactured by Toyobo Co., Ltd., 1.7 deniers, fiber length 40 millimeters) were mixed at a weight ratio of 5:5, and this mixture was placed uniformly on the wire net such that the area density became 30 g/m$^2$. On this mixture, particles having a particle size of 150 to 710 micrometers (average particle size of about 400 micrometers) out of the absorbent resins (1) were uniformly dispersed such that the area density became 30 g/m$^2$. These resins and fibers were passed between the rolls having needles on their surfaces, and were mixed with each other. Hydrophilic fibers (a treatment-processed article of roll pulp manufactured by Rayonier, Inc. was hammer crushed) and hydrophobic fibers (PET/PE core-in-sheath fibers manufactured by Toyobo Co., Ltd., 1.7 deniers, fiber length 40 millimeters) were mixed at a weight ratio of 5:5, and this mixture was uniformly arranged such that the area density became 30 g/m$^2$. Hot-air drying (drying temperature 145°C, drying time 6 seconds, air speed 5 m/s) was performed in the drying furnace, pressing was performed with the flat press (temperature 110°C, air pressure 0.1 N/cm$^2$), winding up was performed, and thus a sheet was acquired. As a result of evaluation of the sheet thus obtained on the above items, the absorption capacity of the sheet was 12 g/g, the absorption capacity of the resins in the sheet was 20 g/g, the water-absorption speed was 170 seconds, the diffusion lengths were 160 millimeters long and 70 millimeters wide, the rewet amount was 7.5 grams, the detachment test indicated no detachment, the water-absorption test indicated 3.5 millimeters in uniform thickness, the bending resistance was 75 millimeters, and the sheet thickness was 1.2 millimeters.

[Example 38]

An undersheet (16 g/m$^2$) constructed of hydrophobic fibers (PET/PE core-in-sheath fibers manufactured by Toyobo Co., Ltd., 1.7 deniers, fiber length 40 millimeters) was conveyed at 30 m/min. Hydrophilic fibers (a treatment-processed article of roll pulp manufactured by Rayonier, Inc. was hammer crushed) and hydrophobic fibers (PET/PE core-in-sheath fibers manufactured by Toyobo Co., Ltd., 1.7 deniers, fiber length 40 millimeters) were mixed at a weight ratio of 5:5, and this mixture was placed uniformly on the undersheet such that the area density became 15 g/m$^2$. On this mixture, particles having a particle size of 150 to 710 micrometers (average particle size of about 400 micrometers) out of the absorbent resins (1) were uniformly dispersed such that the area density became 30 g/m$^2$. These resins and fibers were passed between the rolls having needles on their surfaces, and were mixed with each other. Hydrophilic fibers (a treatment-processed article of roll pulp manufactured by Rayonier, Inc. was hammer crushed) and hydrophobic fibers (PET/PE core-in-sheath fibers manufactured by Toyobo Co., Ltd., 1.7 deniers, fiber length 40 millimeters) were mixed at a weight ratio of 5:5, this mixture was uniformly arranged such that the area density became 15 g/m$^2$ and was covered with an oversheet (16 g/m$^2$) constructed of hydrophobic fibers (PET/PE core-in-sheath fibers manufactured by Toyobo Co., Ltd., 1.7 deniers, fiber length 40 millimeters), and hot-air drying (drying temperature 145°C, drying time 6 seconds, air speed 5 m/s) was performed in the drying furnace, pressing was performed with the flat press (temperature 110°C, air pressure 0.1 N/cm$^2$), winding up was performed, and thus a sheet was acquired. As a result of evaluation of the sheet thus obtained on the above items, the absorption capacity of the sheet was 8 g/g, the absorption capacity of the resins in the sheet was 20 g/g, the water-absorption speed was 200 seconds, the diffusion lengths were 160 millimeters long and 70 millimeters wide, the rewet amount was 8.5 grams, the detachment test indicated no detachment, the water-absorption test indicated 2.8 millimeters in uniform thickness, the bending resistance was 65 millimeters, and the sheet thickness was 0.7 millimeter.

[Comparative Example 10]

An absorbent sheet was produced by the same method as that of Example 36 except using no hydrophilic fibers. As a result of evaluation of the sheet thus obtained on the above items, the absorption capacity of the sheet was 5 g/g, the absorption capacity of the resins in the sheet was 10 g/g, the water-absorption speed was not available because water could not be completely absorbed, the diffusion lengths were 160 millimeters long and 70 millimeters wide, the rewet amount was 11 grams, the detachment test indicated no detachment, the water-absorption test indicated 1.0 millimeter in uniform thickness, the bending resistance was 50 millimeters, and the sheet thickness was 0.6 millimeter.

[Comparative Example 11]

An absorbent sheet was produced by the same method as that of Example 36 except using no hydrophobic fibers. Detachment occurred before absorption.

[Comparative Example 12]

An absorbent sheet was produced by the same method as that of Example 36 except setting the mixing ratio of the hydrophilic fibers to the hydrophobic fibers to 9:91. Because the ratio of the hydrophilic fibers was too small, the absorption capability was low. The absorption capacity of the sheet was 7 g/g, the absorption capacity of the resins in the sheet was 12 g/g, the water-absorption speed was not available because water could not be completely absorbed, the diffusion lengths were 160 millimeters long and 70 millimeters wide, the rewet amount was 11 grams, the detachment test indicated no detachment, the water-absorption test indicated 1.0 millimeter in uniform thickness, the bending resistance was 50 millimeters, and the sheet thickness was 0.6 millimeter.

[Comparative Example 13]

An absorbent sheet was produced by the same method as that of Example 36 except setting the mixing ratio of the hydrophilic fibers to the hydrophobic fibers to 71:29. Because the ratio of the hydrophobic fibers was too small, detachment occurred after absorption. The absorption capacity of the sheet was 11 g/g, the absorption capacity of the resins in the sheet was 22 g/g, the water-absorption speed was 170 seconds, the diffusion lengths were 160 millimeters long and 70 millimeters wide, the rewet amount was 8 grams, the detachment test indicated detachment occurring, the water-absorption test indicated 3.5 millimeters in uniform thickness, the bending resistance was 70 millimeters, and the sheet thickness was 1.4 millimeters.

An absorbent sheet was produced by the same method as that of Example 36 except setting the area density of the absorbent resins to 9 g/m$^2$. Because the quantity of the resins was small, the absorbed amount was too small. The absorption capacity of the sheet was 9 g/g, the absorption capacity of the resins in the sheet was 18 g/g, the water-absorption speed was not available because water could not be completely absorbed, the diffusion lengths were 160 millimeters long and 70 millimeters wide, the rewet amount was 10 grams, the detachment test indicated no detachment, the water-absorption test indicated 2.0 millimeters in uniform thickness, the bending resistance was 70 millimeters, and the sheet thickness was 1 millimeter.

[Comparative Example 15]

An absorbent sheet was produced by the same method as that of Example 36 except setting the area density of the absorbent resins to 51 g/m$^2$. Because of an excessive quantity of resins, detachment occurred after absorption. The absorption capacity of the sheet was 12 g/g, the absorption capacity of the resins in the sheet was 20 g/g, the water-absorption speed was 150 seconds, the diffusion lengths were 160 millimeters long and 70 millimeters wide, the rewet amount was 7 grams, the detachment test indicated detachment occurring, the water-absorption test indicated 4.0 millimeters in uniform thickness, the bending resistance was 70 millimeters, and the sheet thickness was 1.2 millimeters.

EP 2 583 647 B1

[Comparative Example 16]

An undersheet (16 g/m$^2$) constructed of hydrophobic fibers (PET/PE core-in-sheath fibers manufactured by Toyobo Co., Ltd., 1.7 deniers, fiber length 40 millimeters) was conveyed at 30 m/min. Hydrophilic fibers (a treatment-processed article of roll pulp manufactured by Rayonier, Inc. was hammer crushed) and hydrophobic fibers (PET/PE core-in-sheath fibers manufactured by Toyobo Co., Ltd., 1.7 deniers, fiber length 40 millimeters) were mixed at a weight ratio of 5:5, and this mixture was placed uniformly on the undersheet such that the area density became 15 g/m$^2$. On this mixture, particles having a particle size of 150 to 710 micrometers (average particle size of about 400 micrometers) out of the absorbent resins (1) were uniformly dispersed such that the area density became 30 g/m$^2$. These resins and fibers were passed between the rolls having needles on their surfaces, and were mixed with each other. Hydrophilic fibers (a treatment-processed article of roll pulp manufactured by Rayonier, Inc. was hammer crushed) and hydrophobic fibers (PET/PE core-in-sheath fibers manufactured by Toyobo Co., Ltd., 1.7 deniers, fiber length 40 millimeters) were mixed at a weight ratio of 5:5, this mixture was uniformly arranged such that the area density became 30 g/m$^2$, and hot-air drying (drying temperature 130°C, drying time 6 seconds, air speed 5 m/s) was performed in the drying furnace, winding up was performed without pressing by the flat press, and thus a sheet was acquired. As a result of evaluation of the sheet thus obtained on the above items, the absorption capacity of the sheet was 8 g/g, the absorption capacity of the resins in the sheet was 30 g/g, the water-absorption speed was 150 seconds, the diffusion lengths were 150 millimeters long and 70 millimeters wide, the rewet amount was 10 grams, the detachment test indicated detachment occurring, the water-absorption test was invalid because water was not absorbed uniformly, the bending resistance was 60 millimeters, and the sheet thickness was 2.5 millimeters.

[Comparative Example 17]

A sheet that had 50 g/m$^2$ of super water-absorbent polymers (SAP) sandwiched between upper and lower paper tissues (18 g/m$^2$) having a conventional absorbent structure was prepared.
The above-described tests in (13) to (22) were conducted on the sheet of Example 38 and the sheet of Comparative Example 17, and the results are given in Table 4 below.

[Table 4]

|  | Sheet of Example 38 | Sheet of Comparative Example 17 |
|---|---|---|
| (13) odor | first time: 0193 at start, 0066 after turning off grill<br>second time: 0211 at start, 0057 after turning off grill | first time: 0222 at start, 0088 after turning off grill<br>second time: 0233 at start, 0123 after turning off grill |
| (13) usability | good in odor, appearance after use, and easiness of cleanup | bad in appearance after use and easiness of cleanup |
| (13) endurance test | burned into soot at fifth time | burned into soot at second time |
| (14) | excellent in liquid diffusion indicating absorption length of 11 centimeters with resins being fixed even after absorption and no peeling of (upper or lower) sheet | bad in diffusion indicating absorption length of 5 centimeters with swelling (6 to 7 m/m) that was easily torn and broken when being poked by finger |
| (15) | 4.5 milliliters of water largely diffused 3 centimeters and transpired, this portion of sheet was not broken even when touched | 4.8 milliliters of water diffused only 1.5 centimeters and did not transpire, this portion of sheet was easily broken when touched |
| (16) | good in fit feeling because of uniform absorption and no breakage | uncomfortable because breakage occurred gradually as absorbed amount increased and resins stuck to skin |
| (17) | no grime on clothes, good in appearance only with slight swelling on whole sheet | could not be used until end of test because breakage occurred |
| (18) | no soil, good in appearance only with slight swelling on whole sheet | bad in feeling because resin movement occurred, part of sheet peeled off during test |

(continued)

| | | Sheet of Example 38 | Sheet of Comparative Example 17 |
|---|---|---|---|
| | (19) | water was completely absorbed and sheet was flat, so that whether liquid was absorbed could not determined at first glance | part of sheet swelled, water that could not be absorbed remain, fish was excessively deprived of water by resins sticking thereon |
| | (20) | excessive water was completely absorbed | only part around fish swelled and liquid could not be absorbed completely |
| | (21) | proper humidity was maintained | it was difficult to cause mask to uniformly absorb water in first place |
| | (22) | water was absorbed with wide area, no permeation | there was possibility of leakage inside equipment because part of sheet swelled and absorbed water, there was possibility of leakage of resins because strength was weak |

[Example 39]

hydrophilic fibers having an average fiber length of 200 micrometers into which hydrophilic fibers (a treatment-processed article of roll pulp manufactured by Rayonier, Inc. was hammer crushed) were mechanically pulverized and particles having a particle size of 106 to 300 micrometers (average particle size of about 200 micrometers) out of the absorbent resins (5) were mixed at a weight ratio of 1:4. At this time, the water content was 4 mass% with respect to the absorbent resins. Water of the same weight as the absorbent resins was sprayed thereon with the spray, and hot-air drying (drying temperature 140°C, drying time 2 minutes, air speed 5 m/s) was performed in the drying furnace. Composite compositions thus produced were stored in a tank. The water content after dehydration and drying was 3 mass% with respect to the absorbent resins. An undersheet (16 g/m$^2$) constructed of hydrophobic fibers (PET/PE core-in-sheath fibers manufactured by Toyobo Co., Ltd., 1.7 deniers, fiber length 40 millimeters) was conveyed at 30 m/min. On this undersheet, a mixture of the composite compositions and hydrophobic fibers (PET/PE core-in-sheath fibers manufactured by Toyobo Co., Ltd., 11 deniers, fiber length 50 millimeters) in a weight ratio of 5:1 was arranged such that the area density became 150 g/m$^2$. A mixture of hydrophilic fibers (a treatment-processed article of roll pulp manufactured by Rayonier, Inc. was hammer crushed) and hydrophobic fibers (PET/PE core-in-sheath fibers manufactured by Toyobo Co., Ltd., 1.7 deniers, fiber length 40 millimeters) in a weight ratio of 6:4 was placed thereon uniformly such that the area density became 30 g/m$^2$, and a surfactant (5% SANMORIN OT-70) was sprayed with the spray such that the area density became 20 g/m$^2$. At this time, the water content was 20 mass% with respect to the absorbent resins. Hot-air drying (drying temperature 145°C, drying time 6 seconds, air speed 5 m/s) was performed in the drying furnace, pressing was performed with the flat press (temperature 110°C, air pressure 0.1 N/cm$^2$), winding up was performed, and thus a sheet was acquired. This sheet was referred to as an absorbent sheet (39).

[Example 40]

Other than using hydrophilic fibers having an average fiber length of 1000 micrometers when producing composite compositions, the same method as that of Example 39 was used to acquire an absorbent sheet. This sheet was referred to as an absorbent sheet (40).

[Example 41]

Other than using hydrophilic fibers having an average fiber length of 400 micrometers when producing composite compositions, the same method as that of Example 39 was used to acquire an absorbent sheet. This sheet was referred to as an absorbent sheet (41).

[Example 42]

Other than using hydrophilic fibers having an average fiber length of 20 micrometers when producing composite compositions, the same method as that of Example 39 was used to acquire an absorbent sheet. This sheet was referred to as an absorbent sheet (41).

Evaluation results on the absorbent sheet of Examples 39 to 42 are given in Table 3 below.

[Table 5]

|  | Resin | Absorption capacity of sheet (g/g) | Absorption capacity of absorbent resins in sheet (g/g) | Absorption speed (sec.) | Diffusion length (lengthwise) (mm) | Diffusion length (crosswise) (mm) | Rewet amount (g) |
|---|---|---|---|---|---|---|---|
| Example 39 | 5 | 35 | 60 | 6 | 100 | 70 | 0.1 |
| Example 40 | 5 | 35 | 60 | 8.5 | 100 | 70 | 0.1 |
| Example 41 | 5 | 35 | 60 | 7 | 100 | 70 | 0.1 |
| Example 42 | 5 | 35 | 60 | 8 | 100 | 70 | 0.1 |

**Industrial Applicability**

The absorbent sheet of the present invention is industrially applicable to absorbent members of disposable sanitary materials such as disposable diapers, incontinence pads, and sanitary napkins; absorbent members of excretion treatment materials such as sheets for animals and pets; absorbent sheets that prevent marine products from getting wet with thawing water when frozen marine products are transported; absorbent sheets that cover potted plants to prevent water evaporation, absorbent sheets that are laid under potted plants; absorbent sheets that are arranged around a water tank; absorbent sheets that are used for sheets for dew condensation preventing material, for example; waterdrop absorbing mats that are arranged in a part such as a water receiver of an umbrella stand and absorb waterdrops dripping from an umbrella, for example; mats for a headrest cover for a vehicle; mats for preventing one's head from becoming sweaty in a helmet or a hat; toilet paper sheets that are used after defecation at an electric toilet seat with a warm-water spray feature (e.g., Washlet manufactured by TOTO, Ltd.), for example; absorbent mats that prevent a floor of an open event site from getting wet when it rains; absorbent mats that prevent a floor of a vehicle such as a car, a train, or a plane from getting wet on a rainy day; absorbent mats that prevent a floor of facilities such as a hospital, a rest area, a department store, a hotel, a store, an office building, or a recreational facility from getting wet on a rainy day; absorbent mats that prevent the inside of a refrigerator from getting wet; absorbent mats that prevent a floor of a kitchen from getting wet, and absorbent sheets that absorb drips from raw garbage in a kitchen or a cooking place; absorbent mats that prevent a floor equipped with sanitary fixtures such as a water supply system, a hot-water supply system, a toilet bowl, or a washstand from getting wet; absorbent mats that prevent a floor around a refrigerator from getting wet; a mat for leisure or a mat for massotherapy, and an auxiliary mat for a bed; packaging materials that have a water retention or humidity control function for vegetables, fruits, or flowers and ornamental plants; packaging materials that have a water retention or humidity control function for fresh fish, raw meat, a daily dish, or a packed lunch, for example; packaging materials for seeds, bacterial strains, seedlings, or bulbs; waste rags or clothes for cleaning machinery or windows, or for wiping dew condensation water and other water off ceilings, walls, floors, or windows; sheets that prevent water evaporation when growing garden plants; or other articles.

**Reference Signs List**

1... absorbent resins, 2... hydrophilic fibers, 3... hydrophobic fibers, 4... absorbent layer, 5... hydrophobic fiber layer, 10... absorbent sheet, 11... back sheet, 12... top sheet, 20, 21, 22... absorbent product

**Claims**

**1.**  A composite compositions containing absorbent resins and hydrophilic fibers,
the composite compositions being obtained by dehydrating absorbent resins and hydrophilic fibers from a state of being in contact with each other and containing water,
wherein the absorbent resins and the hydrophilic fibers have bonding therebetween, wherein the absorbent resins are surface crosslinked by condensation crosslinking agents,
wherein the absorbent resins have acid groups in the side chains and wherein the residual monomer concentration in the absorbent resins with respect to the whole quantity of the absorbent resins is 200 ppm by mass or less, and wherein the surface salt concentration of the absorbent resin particles is reduced by 10 mol% or more with respect to the salt concentration at the resin central part.

2.  An absorbent sheet, comprising the composite compositions according to claim 1 and hydrophobic fibers, wherein the composite compositions are dispersed among the hydrophobic fibers.

3.  The absorbent sheet according to claim 2, wherein the hydrophobic fibers are substantially fused with each other.

4.  The absorbent sheet according to claim 2 or claim 3, further comprising ammonium ions at 0.5 to 18 mass%.

5.  A method for producing an absorbent sheet containing absorbent resins, hydrophilic fibers, and hydrophobic fibers, comprising:

    a sheet-forming step of forming a sheet of the composite compositions according to claim 1 and the hydrophobic fibers by heating while bringing the composite compositions and the hydrophobic fibers into contact with each other.

6.  The method for producing an absorbent sheet according to claim 5, wherein at the sheet-forming step, on a support that is continuously fed, the composite compositions and the hydrophobic fibers are heated while being brought into contact with each other.

7.  An absorbent product, comprising:

    the absorbent sheet according to any one of claims 2 to 4, and
    a top sheet provided on one side of the absorbent sheet and/or a back sheet provided on the other side of the absorbent sheet.

8.  A method for producing an absorbent product, comprising a step of adhering the absorbent sheet according to any one of claims 2 to 4 to a top sheet that is continuously fed and/or a back sheet that is continuously fed.

**Patentansprüche**

1.  Verbundzusammensetzungen, enthaltend saugfähige Harze und hydrophile Fasern,
    hergestellt durch Entwässerung der saugfähigen Harze und hydrophilen Fasern aus einem Zustand, in dem sie sich in Kontakt miteinander befinden und Wasser enthalten,
    wobei die saugfähigen Harze und die hydrophilen Fasern über eine sich dazwischen befindende Bindung verfügen,
    wobei die saugfähigen Harze durch kondensationsvernetzende Mittel oberflächlich vernetzt sind,
    wobei die saugfähigen Harze über seitenständige Säuregruppen verfügen und wobei die saugfähigen Harze über eine auf die Gesamtmenge der saugfähigen Harze bezogene Monomerrestkonzentration von 200 Massen-ppm und weniger verfügen und
    wobei die saugfähigen Harze als Teilchen über eine Salzkonzentration verfügen, die an der Oberfläche gegenüber dem Zentralbereich des Harzes um 10 mol-% und mehr vermindert ist.

2.  Saugfähige Folie, umfassend die Verbundzusammensetzungen gemäß Anspruch 1 und hydrophobe Fasern, wobei die Verbundzusammensetzungen unter den hydrophoben Fasern verteilt sind.

3.  Saugfähige Folie nach Anspruch 2, bei der die hydrophoben Fasern im Wesentlichen miteinander verschmolzen sind.

4.  Saugfähige Folie nach Anspruch 2 oder 3, ferner umfassend Ammoniumionen mit 0,5 bis 18 Gew.-%.

5.  Verfahren zur Herstellung einer saugfähigen Folie, enthaltend saugfähige Harze, hydrophile Fasern und hydrophobe Fasern, umfassend:

    einen Schritt der Folienbildung, bei dem man aus den Verbundzusammensetzungen gemäß Anspruch 1 und den hydrophoben Fasern eine Folie bildet, indem man die Verbundzusammensetzungen und die hydrophoben Fasern unter Erwärmung miteinander in Berührung bringt.

**6.** Verfahren zur Herstellung einer saugfähigen Folie nach Anspruch 5, bei dem man bei der Folienbildung die Verbundzusammensetzungen und die hydrophoben Fasern auf einer kontinuierlich zugeführten Unterlage unter Erwärmung miteinander in Berührung bringt.

**7.** Saugfähiges Produkt, umfassend:

die saugfähige Folie gemäß einem der Ansprüche 2 bis 4 und
eine auf der einen Seite der saugfähigen Folie vorgesehene Oberfolie und/oder eine auf der anderen Seite der saugfähigen Folie vorgesehene Unterfolie.

**8.** Verfahren zur Herstellung eines saugfähigen Produkts, umfassend einen Schritt, bei dem man die saugfähige Folie gemäß einem der Ansprüche 2 bis 4 mit einer kontinuierlich zugeführten Oberfolie und/oder einer kontinuierlich zugeführten Unterfolie verklebt.

**Revendications**

**1.** Compositions composites contenant des résines absorbantes et des fibres hydrophiles,
les compositions composites étant obtenues par déshydratation de résines absorbantes et de fibres hydrophiles à partir d'un état où elles sont en contact les unes avec les autres et contiennent de l'eau,
dans lesquelles il existe une liaison entre les résines absorbantes et les fibres hydrophiles, et dans lesquelles les résines absorbantes sont réticulées en surface par des agents de réticulation par condensation,
dans lesquelles les résines absorbantes comportent des groupes acides dans les chaînes latérales et dans lesquelles la concentration de monomère résiduel dans les résines absorbantes relativement à la quantité totale des résines absorbantes est de 200 ppm en masse ou moins, et
dans lesquelles la concentration en sel à la surface des particules de résine absorbante est réduite de 10 %mol ou plus relativement à la concentration en sel dans la partie centrale de la résine.

**2.** Feuille absorbante, comprenant les compositions composites selon la revendication 1 et des fibres hydrophobes, dans laquelle les compositions composites sont dispersées parmi les fibres hydrophobes.

**3.** Feuille absorbante selon la revendication 2, dans laquelle les fibres hydrophobes sont sensiblement fixées par fusion les unes aux autres.

**4.** Feuille absorbante selon la revendication 2 ou la revendication 3, comprenant en outre des ions ammonium dans une proportion de 0,5 %mass à 18 %mass.

**5.** Procédé de fabrication d'une feuille absorbante contenant des résines absorbantes, des fibres hydrophiles, et des fibres hydrophobes, comprenant :

une étape de formation de feuille, qui consiste à former une feuille des compositions composites selon la revendication 1 et des fibres hydrophobes en chauffant tout en amenant les compositions composites et les fibres hydrophobes en contact les unes avec les autres.

**6.** Procédé de fabrication d'une feuille absorbante selon la revendication 5, dans lequel, durant l'étape de formation de feuille, sur un support qui est amené en continu, les compositions composites et les fibres hydrophobes sont chauffées tout en étant amenées en contact les unes avec les autres.

**7.** Produit absorbant, comprenant :

la feuille absorbante selon l'une quelconque des revendications 2 à 4, et
une feuille supérieure présente sur un côté de la feuille absorbante et/ou une feuille postérieure présente sur l'autre côté de la feuille absorbante.

**8.** Procédé de fabrication d'un produit absorbant, comprenant une étape qui consiste à faire adhérer la feuille absorbante selon l'une quelconque des revendications 2 à 4 à une feuille supérieure qui est amenée en continu et/ou à une feuille postérieure qui est amenée en continu.

*Fig.1*

ABSORBENT
LAYER

# Fig.2

**Fig.3**

**Fig.4**

*Fig.5*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3196933 B
- JP 53004789 A
- JP 56060556 A
- JP 2003508647 W
- JP 2003011118 A
- WO 2006121148 A
- JP S5584304 B
- JP S4943395 B
- JP S51125468 B
- JP S5214689 B
- JP S5315959 B
- JP 2003192794 A
- JP 2005124833 A